(19)

Europäisches
Patentamt
European
Patent Office
Office européen
des brevets

(11)     EP 4 372 006 A1

(12)     EUROPEAN PATENT APPLICATION
published in accordance with Art. 153(4) EPC

(43) Date of publication:
    22.05.2024  Bulletin 2024/21

(21) Application number: 22842195.4

(22) Date of filing: 15.07.2022

(51) International Patent Classification (IPC):
    C07K 16/28 (2006.01)          A61K 39/395 (2006.01)
    A61K 47/55 (2017.01)          A61K 47/68 (2017.01)
    A61P 35/00 (2006.01)          A61P 37/04 (2006.01)
    C07K 16/46 (2006.01)          C12M 1/34 (2006.01)
    C12N 15/13 (2006.01)          C12Q 1/02 (2006.01)
    G01N 33/50 (2006.01)

(52) Cooperative Patent Classification (CPC):
    A61K 39/395; A61K 47/55; A61K 47/68;
    A61P 35/00; A61P 37/04; C07K 16/00;
    C07K 16/28; C07K 16/46; C12M 1/34; C12Q 1/02;
    G01N 33/50; Y02A 50/30

(86) International application number:
    PCT/JP2022/027809

(87) International publication number:
    WO 2023/286854 (19.01.2023 Gazette 2023/03)

(84) Designated Contracting States:
    AL AT BE BG CH CY CZ DE DK EE ES FI FR GB
    GR HR HU IE IS IT LI LT LU LV MC MK MT NL NO
    PL PT RO RS SE SI SK SM TR
    Designated Extension States:
    BA ME
    Designated Validation States:
    KH MA MD TN

(30) Priority:  16.07.2021  JP 2021118316

(71) Applicant: Brightpath Biotherapeutics Co., Ltd.
    Kawasaki-shi, Kanagawa 210-0821 (JP)

(72) Inventors:
    • NAKAJIMA Kanto
      Kawasaki-shi, Kanagawa 210-0821 (JP)

    • SHIRAISHI Mamoru
      Kawasaki-shi, Kanagawa 210-0821 (JP)
    • ARAMAKI Takahiko
      Kawasaki-shi, Kanagawa 210-0821 (JP)
    • MATSUMURA Haruka
      Kawasaki-shi, Kanagawa 210-0821 (JP)
    • MATSUMOTO Noriko
      Kawasaki-shi, Kanagawa 210-0821 (JP)

(74) Representative: Plasseraud IP
    66, rue de la Chaussée d'Antin
    75440 Paris Cedex 09 (FR)

Remarks:
    The complete document including Reference
    Table(s) and the Sequence Listing(s) can be
    downloaded from the EPO website

(54)    **ANTI-TIM-3 ANTIGEN ANTIBODY OR ANTIBODY DERIVATIVE, AND USE THEREOF**

(57)    The present invention is directed to develop an antibody or an antibody derivative with enhanced functionality targeting the Tim-3 antigen, more specifically an antibody etc. with higher activity to remove immunosuppression signal, and to develop cancer treatment composition comprising such an antibody etc.

The present invention has identified an antibody etc. that binds to the Tim-3 antigen, having complementary determining regions in the heavy and light chains with specific amino acid sequences, and has an ability to activate T cells with cytotoxic effects against cancer cells, thereby demonstrating that the present invention can solve the above problem.

EP 4 372 006 A1

**Description**

Technical Field

The present invention relates to an antibody or an antibody derivative that has a binding specificity for the Tim-3 antigen expressed on cancer cells and immune cells and has the effect of removing inhibitory signals from the immune system directed at cancer cells, and also relates to a composition and a method for the treatment of cancer and for the diagnosis of cancer that include the antibody or antibody derivative.

Background Art

Cancer cells actively utilize the immunosuppressive function of immune checkpoint molecules, in addition to regulatory T cells (Treg) and myeloid-derived suppressor cells (MDSC), to avoid attacks from the immune system. Various immune checkpoint molecules and their ligands have been identified.

Immune checkpoint inhibitors block the binding between immune checkpoint molecules and their ligands, to inhibit the transmission of immunosuppressive signals, thereby releasing the suppression of T cell activation by immune checkpoint molecules on cancer cells, and can reactivate the immune function and generate an immune response against the cancer cells. By utilizing this mechanism, immune checkpoint inhibitors offer a different approach to treat cancer from conventional cancer drugs and molecular target drugs.

So far, several immune checkpoint inhibitors have been approved as pharmaceuticals including nivolumab and pembrolizumab (anti-PD-1 antibodies), atezolizumab, and durvalumab (anti-PD-L1 antibodies) as an inhibitor of PD-1/PD-1L, as well as ipilimumab (anti-CTLA-4 antibody) as an inhibitor of CTLA-4/B7 (CD80/CD86). Drugs comprising antibodies as a primary component demonstrate high efficacy in some cancer types when used as monotherapies or in combination with other anticancer agents.

However, the response rate of conventional immune checkpoint inhibitors is reported to be approximately 5-30%. It has been revealed that the individual differences in the immune status of the patient's cancer relates to the effectiveness of immune checkpoint inhibitors. The individual differences in cancer immune status are considered to be influenced by the genetic abnormalities of cancer cells as a main cause, in combination with factors such as the patient's immune constitution (genetic background, such as HLA type) and various environmental factors (such as intestinal bacteria, smoking, ultraviolet radiation, diet, stress), and the conventional immune checkpoint inhibitors are unable to address certain patients.

Tim-3 (also known as HAVCR2) is recognized as one of the immune checkpoint molecules. Tim-3 belongs to the T cell Ig and mucin domain-containing molecule superfamily. Tim-3 is an essential regulatory molecule in T cell tolerance and plays a crucial role in autoimmune responses and T cell exhaustion during chronic viral infections (Non Patent Literature No. 1) .

Tim-3 is expressed on Th1 cells (type 1 T helper cells), dendritic cells, CD8[+] T cells and other lymphocyte subsets. Time-3 interacts with a lot of ligands such as Phosphatidylserine (PS), CEACAM1 (Carcinoembryonic antigen-related cell adhesion molecule 1), Galectin-9, HMGB1 (High mobility group box 1) and so on. Among them, since the Tim-3-Galectin-9 pathway negatively regulates Th1 cells (type 1 T helper cells) immunity (Non Patent Literature No. 2), inhibition of Tim-3 is expected to enhance innate and adaptive immunity.

It is reported that Tim-3 expressed in some cancer cells plays a crucial role in the progression process of cancer. For instance, in myeloid tumors, it has been suggested that Tim-3 expressed on cancer stem cells binds to Galectin-9 secreted by the tumor cells themselves to transmit essential signals for self-replication to Tim-3-expressing cancer stem cells, thereby expanding the clone size (Non-Patent Literature No. 3).

[Citation List]

[Non Patent Literature]

[Non Patent Literature No. 1] Isabel Sada-Ovalle, et al., J. Immunol. 2012, 189(12):5896-902
[Non Patent Literature No. 2] Chen Zhu, et al., Nat. Immunol. 2005, 6(12) :1245-52
[Non Patent Literature No. 3] Yoshikane Kikushige, et al., Cell Stem Cell. 2015,17:341-352

Summary of Invention

Technical Problem

The present invention is directed to develop an antibody or an antibody derivative (hereinafter, "an antibody or an antibody derivative" is collectively referred to as as "an antibody etc.") with enhanced function targeting Tim-3 antigen, more specifically an antibody etc. with higher activity to remove immunosuppressive signal, and to develop a cancer treatment composition comprising such an antibody etc.

Solution to Problem

The present invention has identified an antibody etc. that binds to the Tim-3 antigen and has an ability to remove the immunosuppressive signals on cancer cells, thereby demonstrating that the present invention can solve the above problem.

More specifically, this application provides the following embodiments to solve the problem aforementioned:

[1] A heterodimeric antibody or antibody derivative that is a combination of two different half-molecules each of which binds to the Tim-3 antigen and enhances immune activity against cancer cells.

[2] The antibody or antibody derivative according to Claim 1, wherein the one half-molecule of the antibody or antibody derivative binds to the first region of the Tim-3 antigen, and the other half-molecule of the antibody or antibody derivative binds to the second region of the Tim-3 antigen.

[3] The antibody or antibody derivative according to [1] or [2], wherein the half-molecule of the antibody or antibody derivative is a combination of two different types of half-molecule of the antibody or antibody derivative comprising either set of the complementarity-determining regions of the heavy and light chains selected from the group consisting of:

(1) Complementarity-determining regions of the heavy chain, CDR1 (SYYMS, SEQ ID No: 1), CDR2 (TISNSGGSIYYLDSVKD, SEQ ID No: 2), and CDR3 (DPYYSNYVPMDY, SEQ ID No.:3), and complementarity-determining regions of the light chain, CDR1 (KASQYVDTYVA, SEQ ID No: 4), CDR2 (SASTRHT, SEQ ID No.:5), and CDR3 (AQYSSSPLT, SEQ ID No: 6);

(2) Complementarity-determining regions of heavy chains, CDR1 (DYYMD, SEQ ID No: 9), CDR2 (YIYPNNGGTSYNQKFKG, SEQ ID No: 10), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(3) Complementarity-determining regions of the heavy chains, CDR1 (DYYMD, SEQ ID No: 17), CDR2 (YIYPNNGGTSYNQKFKG, SEQ ID No: 18), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(4) Complementarity-determining regions of the heavy chains, CDR1 (DYYMD, SEQ ID No: 25), CDR2 (YIYPNNGGTSYNQKFKG, SEQ ID No: 26), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(5) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);

(6) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASENVGTYVS, SEQ ID No: 52), CDR2 (GASNRYT, SEQ ID No: 53), and CDR3 (GQSYSYPLT, SEQ ID No: 54);

(7) Complementarity-determining regions of heavy chains, CDR1 (DYYMD, SEQ ID No: 9), CDR2 (YIYPNNAGTS YNQKFKG, SEQ ID No: 74), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(8) Complementarity-determining regions of the heavy chain, CDR1 (DYYMD, SEQ ID No: 9), CDR2 (YIYPNKGGTS YNQKFKG, SEQ ID No: 76), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(9) Complementarity-determining regions of the heavy chain, CDR1 (DYYMD, SEQ ID No: 17), CDR2 (YIYPNNAGTS YNQKFKG, SEQ ID No: 78), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(10) Complementarity-determining regions of heavy chains, CDR1 (DYYMD, SEQ ID No: 17), CDR2 (YIYPNKGGTS YNQKFKG, SEQ ID No: 80), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(11) Complementarity-determining regions of heavy chains, CDR1 (DYYMD, SEQ ID No: 25), CDR2 (YIYPNNAGTS YNQKFKG, SEQ ID No: 82), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(12) Complementarity-determining regions of heavy chains, CDR1 (DYYMD, SEQ ID No: 25), CDR2 (YIYPNKGGTS YNQKFKG, SEQ ID No: 84), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30).

[4] The antibody or antibody derivative according to any one of [1] to [3], wherein the one half-molecule of the antibody or antibody derivative is a half-molecule of (1), (5) or (6), and the other half-molecule of the antibody or antibody derivative is a half-molecule of (2) to (4) or (7) to (12) .

[5] The antibody or antibody derivative according to any one of [1] to [4], wherein the antibody derivative is selected from a modified antibody selected from a humanized antibody, a chimeric antibody, a single chain Fv antibody (a scFv antibody), Fab, Fab', F(ab')2, Fc effector function modified antibody, IgG1LALA, IgG1_N297A, IgG4_S228P, or a functional fragment thereof.

[6] The antibody or antibody derivative according to any one of [1] to [5], wherein the amino acid sequence of the heavy chain variable region VH domain of the half-molecule of the antibody or antibody derivative is selected from the group consisting of:

(VH-1a) the amino acid sequence of SEQ ID No: 7 or an amino acid sequence comprising one or several amino acid substitutions (e.g., conservative substitutions), insertions, or deletions in regions of the amino acid sequence of SEQ ID No: 7 other than CDR1 (SEQ ID No: 1), CDR2 (SEQ ID No: 2), and CDR3 (SEQ ID No: 3);

(VH-1b) the amino acid sequence of SEQ ID No: 49 or an amino acid sequence comprising one or several amino acid substitutions (e.g., conservative substitutions), insertions, or deletions in regions of the amino acid sequence of SEQ ID No: 49 other than CDR1 (SEQ ID No: 1), CDR2 (SEQ ID No: 2), and CDR3 (SEQ ID No: 3);

(VH-1c) the amino acid sequence of SEQ ID No: 57 or an amino acid sequence comprising one or several amino acid substitutions (e.g., conservative substitutions), insertions, or deletions in regions of the amino acid sequence of SEQ ID No: 57 other than CDR1 (SEQ ID No: 1), CDR2 (SEQ ID No: 2), and CDR3 (SEQ ID No: 3);

(VH-2a) the amino acid sequence of SEQ ID No: 15 or an amino acid sequence comprising one or several amino acid substitutions (e.g., conservative substitutions), insertions, or deletions in regions of the amino acid sequence of SEQ ID No: 15 other than CDR1 (SEQ ID No: 9), CDR2 (SEQ ID No: 10), and CDR3 (SEQ ID No: 11);

(VH-2b) the amino acid sequence of SEQ ID No: 62 or an amino acid sequence comprising one or several amino acid substitutions (e.g., conservative substitutions), insertions, or deletions in regions of the amino acid sequence of SEQ ID No: 62 other than CDR1 (SEQ ID No: 9), CDR2 (SEQ ID No: 10), and CDR3 (SEQ ID No: 11);

(VH-3a) the amino acid sequence of SEQ ID No: 23 or an amino acid sequence comprising one or several amino acid substitutions (e.g., conservative substitutions), insertions, or deletions in regions of the amino acid sequence of SEQ ID No: 23 other than CDR1 (SEQ ID No: 17), CDR2 (SEQ ID No: 18), and CDR3 (SEQ ID No: 19);

(VH-3b) the amino acid sequence of SEQ ID No: 66 or an amino acid sequence comprising one or several amino acid substitutions (e.g., conservative substitutions), insertions, or deletions in regions of the amino acid sequence of SEQ ID No: 66 other than CDR1 (SEQ ID No: 17), CDR2 (SEQ ID No: 18), and CDR3 (SEQ ID No: 19);

(VH-4a) the amino acid sequence of SEQ ID No: 31 or an amino acid sequence comprising one or several amino acid substitutions (e.g., conservative substitutions), insertions, or deletions in regions of the amino acid sequence of SEQ ID No: 31 other than CDR1 (SEQ ID No: 25), CDR2 (SEQ ID No: 26), and CDR3 (SEQ ID No: 27);

(VH-4b) the amino acid sequence of SEQ ID No: 70 or an amino acid sequence comprising one or several amino acid substitutions (e.g., conservative substitutions), insertions, or deletions in regions of the amino acid sequence of SEQ ID No: 70 other than CDR1 (SEQ ID No: 25), CDR2 (SEQ ID No: 26), and CDR3 (SEQ ID No: 27);

(VH-5a) the amino acid sequence of SEQ ID No: 36 or an amino acid sequence comprising one or several amino acid substitutions (e.g., conservative substitutions), insertions, or deletions in regions of the amino acid sequence of SEQ ID No: 36 other than CDR1 (SEQ ID No: 33), CDR2 (SEQ ID No: 34), and CDR3 (SEQ ID No: 35);

(VH-5b) the amino acid sequence of SEQ ID No: 51 or an amino acid sequence comprising one or several amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35);

(VH-5c) the amino acid sequence of SEQ ID No: 59 or an amino acid sequence comprising one or several amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35);

(VH-6a) the amino acid sequence of SEQ ID No: 55 or an amino acid sequence comprising one or several amino acid substitutions (e.g., conservative substitutions), insertions, or deletions in regions of the amino acid sequence of SEQ ID No: 55 other than CDR1 (SEQ ID No: 33), CDR2 (SEQ ID No: 34), and CDR3 (SEQ ID No: 35);

(VH-6b) the amino acid sequence of SEQ ID No: 51 or an amino acid sequence comprising one or several amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35);

(VH-6c) the amino acid sequence of SEQ ID No: 59 or an amino acid sequence comprising one or several amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35);

(VH-7) the amino acid sequence of SEQ ID No: 75 or an amino acid sequence comprising one or several amino acid substitutions (e.g., conservative substitutions), insertions, or deletions in regions of the amino acid sequence of SEQ ID No: 75 other than CDR1 (SEQ ID No: 9), CDR2 (SEQ ID No: 74), and CDR3 (SEQ ID No: 11);

(VH-8) the amino acid sequence of SEQ ID No: 77 or an amino acid sequence comprising one or several amino acid substitutions (e.g., conservative substitutions), insertions, or deletions in regions of the amino acid sequence of SEQ ID No: 77 other than CDR1 (SEQ ID No: 9), CDR2 (SEQ ID No: 76), and CDR3 (SEQ ID No: 11);

(VH-9) the amino acid sequence of SEQ ID No: 79 or an amino acid sequence comprising one or several amino acid substitutions (e.g., conservative substitutions), insertions, or deletions in regions of the amino acid sequence of SEQ ID No: 79 other than CDR1 (SEQ ID No: 17), CDR2 (SEQ ID No: 78), and CDR3 (SEQ ID No: 19);

(VH-10) the amino acid sequence of SEQ ID No: 81 or an amino acid sequence comprising one or several amino acid substitutions (e.g., conservative substitutions), insertions, or deletions in regions of the amino acid sequence of SEQ ID No: 81 other than CDR1 (SEQ ID No: 17), CDR2 (SEQ ID No: 80), and CDR3 (SEQ ID No: 19);

(VH-11) the amino acid sequence of SEQ ID No: 83 or an amino acid sequence comprising one or several amino acid substitutions (e.g., conservative substitutions), insertions, or deletions in regions of the amino acid sequence of SEQ ID No: 83 other than CDR1 (SEQ ID No: 25), CDR2 (SEQ ID No: 82), and CDR3 (SEQ ID No: 27);

(VH-12) the amino acid sequence of SEQ ID No: 85 or an amino acid sequence comprising one or several amino acid substitutions (e.g., conservative substitutions), insertions, or deletions in regions of the amino acid sequence of SEQ ID No: 85 other than CDR1 (SEQ ID No: 25), CDR2 (SEQ ID No: 84), and CDR3 (SEQ ID No: 27).

[7] The antibody or antibody derivative according to any one of [1] to [6], wherein the amino acid sequence of the light chain variable region VL domain of the half-molecule of the antibody or antibody derivative is selected from the group consisting of:

(VL-1a) the amino acid sequence of SEQ ID No: 8 or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6);

(VL-1b) the amino acid sequence of SEQ ID No: 58 or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6);

(VL-1c) the amino acid sequence of SEQ ID No: 50 or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6);
(VL-2a) the amino acid sequence of SEQ ID No: 16 or an amino acid sequence comprising one or several amino acid substitutions (e.g., conservative substitutions), insertions, or deletions in regions of the amino acid sequence of SEQ ID No: 16 other than CDR1 (SEQ ID No: 12), CDR2 (SEQ ID No: 13), and CDR3 (SEQ ID No: 14);
(VL-2b) the amino acid sequence of SEQ ID No: 63 or an amino acid sequence comprising one or several amino acid substitutions (e.g., conservative substitutions), insertions, or deletions in regions of the amino acid sequence of SEQ ID No: 63 other than CDR1 (SEQ ID No: 12), CDR2 (SEQ ID No: 13), and CDR3 (SEQ ID No: 14);
(VL-2c) the amino acid sequence of SEQ ID No: 64 or an amino acid sequence comprising one or several amino acid substitutions (e.g., conservative substitutions), insertions, or deletions in regions of the amino acid sequence of SEQ ID No: 64 other than CDR1 (SEQ ID No: 12), CDR2 (SEQ ID No: 13), and CDR3 (SEQ ID No: 14);
(VL-2d) the amino acid sequence of SEQ ID No: 65 or an amino acid sequence comprising one or several amino acid substitutions (e.g., conservative substitutions), insertions, or deletions in regions of the amino acid sequence of SEQ ID No: 65 other than CDR1 (SEQ ID No: 12), CDR2 (SEQ ID No: 13), and CDR3 (SEQ ID No: 14);
(VL-3a) the amino acid sequence of SEQ ID No: 24 or an amino acid sequence comprising one or several amino acid substitutions (e.g., conservative substitutions), insertions, or deletions in regions of the amino acid sequence of SEQ ID No: 24 other than CDR1 (SEQ ID No: 20), CDR2 (SEQ ID No: 21), and CDR3 (SEQ ID No: 22);
(VL-3b) the amino acid sequence of SEQ ID No: 67 or an amino acid sequence comprising one or several amino acid substitutions (e.g., conservative substitutions), insertions, or deletions in regions of the amino acid sequence of SEQ ID No: 67 other than CDR1 (SEQ ID No: 20), CDR2 (SEQ ID No: 21), and CDR3 (SEQ ID No: 22);
(VL-3c) the amino acid sequence of SEQ ID No: 68 or an amino acid sequence comprising one or several amino acid substitutions (e.g., conservative substitutions), insertions, or deletions in regions of the amino acid sequence of SEQ ID No: 68 other than CDR1 (SEQ ID No: 20), CDR2 (SEQ ID No: 21), and CDR3 (SEQ ID No: 22);
(VL-3d) the amino acid sequence of SEQ ID No: 69 or an amino acid sequence comprising one or several amino acid substitutions (e.g., conservative substitutions), insertions, or deletions in regions of the amino acid sequence of SEQ ID No: 69 other than CDR1 (SEQ ID No: 20), CDR2 (SEQ ID No: 21), and CDR3 (SEQ ID No: 22);
(VL-4a) the amino acid sequence of SEQ ID No: 32 or an amino acid sequence comprising one or several amino acid substitutions (e.g., conservative substitutions), insertions, or deletions in regions of the amino acid sequence of SEQ ID No: 32 other than CDR1 (SEQ ID No: 28), CDR2 (SEQ ID No: 29), and CDR3 (SEQ ID No: 30);
(VL-4b) the amino acid sequence of SEQ ID No: 71 or an amino acid sequence comprising one or several amino acid substitutions (e.g., conservative substitutions), insertions, or deletions in regions of the amino acid sequence of SEQ ID No: 71 other than CDR1 (SEQ ID No: 28), CDR2 (SEQ ID No: 29), and CDR3 (SEQ ID No: 30);
(VL-4c) the amino acid sequence of SEQ ID No: 72 or an amino acid sequence comprising one or several amino acid substitutions (e.g., conservative substitutions), insertions, or deletions in regions of the amino acid sequence of SEQ ID No: 72 other than CDR1 (SEQ ID No: 28), CDR2 (SEQ ID No: 29), and CDR3 (SEQ ID No: 30);
(VL-4d) the amino acid sequence of SEQ ID No: 73 or an amino acid sequence comprising one or several amino acid substitutions (e.g., conservative substitutions), insertions, or deletions in regions of the amino acid sequence of SEQ ID No: 73 other than CDR1 (SEQ ID No: 28), CDR2 (SEQ ID No: 29), and CDR3 (SEQ ID No: 30);
(VL-5a) the amino acid sequence of SEQ ID No: 8 or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6);
(VL-5b) the amino acid sequence of SEQ ID No: 58 or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6);
(VL-5c) the amino acid sequence of SEQ ID No: 50 or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6);
(VL-6a) the amino acid sequence of SEQ ID No: 56 or an amino acid sequence comprising one or several amino acid substitutions (e.g., conservative substitutions), insertions, or deletions in regions of the amino acid sequence of SEQ ID No: 56 other than CDR1 (SEQ ID No: 52), CDR2 (SEQ ID No: 53), and CDR3 (SEQ ID No: 54);
(VL-6b) the amino acid sequence of SEQ ID No: 60 or an amino acid sequence comprising one or several amino acid substitutions (e.g., conservative substitutions), insertions, or deletions in regions of the amino acid sequence of SEQ ID No: 60 other than CDR1 (SEQ ID No: 52), CDR2 (SEQ ID No: 53), and CDR3 (SEQ ID No: 54);
(VL-6c) the amino acid sequence of SEQ ID No: 61 or an amino acid sequence comprising one or several amino acid substitutions (e.g., conservative substitutions), insertions, or deletions in regions of the amino acid sequence of SEQ ID No: 61 other than CDR1 (SEQ ID No: 52), CDR2 (SEQ ID No: 53), and CDR3 (SEQ ID No: 54).

[8] The antibody or antibody derivative according to any one of [1] to [4], wherein the antibody derivative is selected from the following variable regions of the half-molecules composed of the single chain Fv antibodies (scFv):

(1) an amino acid sequence in which the heavy chain variable region of (VH-1a), (VH-1b) or (VH-1c) and the light chain variable region of (VL-1a), (VL-1b) or (VL-1c) are connected via a linker,
(2) an amino acid sequence in which the heavy chain variable region of (VH-2a) or (VH-2b) and the light chain variable region of (VL-2a), (VL-2b), (VL-2c) or (VL-2d) are connected via a linker,
(3) an amino acid sequence in which the heavy chain variable region of (VH-3a) or (VH-3b) and the light chain variable region of (VL-3a), (VL-3b), (VL-3c) or (VL-3d) are connected via a linker,
(4) an amino acid sequence in which the heavy chain variable region of (VH-4a) or (VH-4b) and the light chain variable region of (VL-4a), (VL-4b), (VL-4c) or (VL-4d) connected via a linker, and
(5) an amino acid sequence in which the heavy chain variable region of (VH-5a), (VH-5b) or (VH-5c) and the light chain variable region of (VL-5a), (VL-5b) or (VL-5c) are connected via a linker,
(6) an amino acid sequence in which the heavy chain variable region of (VH-6a), (VH-6b) or (VH-6c) and the light chain variable region of (VL-6a), (VL-6b) or (VL-6c) are connected via a linker,
(7) an amino acid sequence in which the heavy chain variable region of (VH-7) and the light chain variable region of (VL-2a), (VL-2b), (VL-2c) or (VL-2d) are connected via a linker,
(8) an amino acid sequence in which the heavy chain variable region of (VH-8) and the light chain variable region of (VL-2a), (VL-2b), (VL-2c) or (VL-2d) are connected via a linker,
(9) an amino acid sequence in which the heavy chain variable region of (VH-9) and the light chain variable region of (VL-3a), (VL-3b), (VL-3c) or (VL-3d) are connected via a linker,
(10) an amino acid sequence in which the heavy chain variable region of (VH-10) and the light chain variable region of (VL-3a), (VL-3b), (VL-3c) or (VL-3d) are connected via a linker,
(11) an amino acid sequence in which the heavy chain variable region of (VH-11) and the light chain variable region of (VL-4a), (VL-4b), (VL-4c) or (VL-4d) are connected via a linker,
(12) an amino acid sequence in which the heavy chain variable region of (VH-12) and the light chain variable region of (VL-4a), (VL-4b), (VL-4c) or (VL-4d) are connected via a linker.

[9] The antibody or antibody derivative according to any one of [1] to [8], wherein the enhancement of immune activity is activation of T cells selected from the group consisting of removal of immunosuppressive signals, relieving T cell exhaustion, proliferation of T cells, increase in cytotoxicity of T cells against cancer cells, and promotion of T cell cytokine secretion.
[10] The antibody or antibody derivative according to any one of [1] to [9] wherein the antibody or antibody derivative induces cytotoxicity against cancer cells but not against normal cells.
[11] The antibody or antibody derivative according to any one of [1] to [10], wherein the cancer cell is selected from the group consisting of melanoma, breast cancer, lung cancer, colon cancer, gastric cancer, pancreatic cancer, liver cancer, and blood cancer.
[12] A homodimeric antibody or antibody derivative which binds to the Tim-3 antigen and enhances immune activity against cancer cells, comprising a set of the complementarity-determining regions of the heavy and light chains selected from the group consisting of;

(1) Complementarity-determining regions of the heavy chain, CDR1 (SYYMS, SEQ ID No: 1), CDR2 (TISNSGGSIYYLDSVKD, SEQ ID No: 2), and CDR3 (DPYYSNYVPMDY, SEQ ID No: 3), and Complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);
(2) Complementarity-determining regions of heavy chains, CDR1 (DYYMD, SEQ ID No: 9), CDR2 (YIYPNNGGTSYNQKFKG, SEQ ID No: 10), and CDR3 (SGYGNYYTMDY, SEQ ID No: 11), and Complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);
(3) Complementarity-determining regions of the heavy chains, CDR1 (DYYMD, SEQ ID No: 17), CDR2 (YIYPNNGGTSYNQKFKG, SEQ ID No: 18), and CDR3 (GGYYSYYSYDY, SEQ ID No: 19), and Complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);
(4) Complementarity-determining regions of the heavy chains, CDR1 (DYYMD, SEQ ID No: 25), CDR2 (YIYPNNGGTSYNQKFKG, SEQ ID No: 26), and CDR3 (SGYKAYYAMDY, SEQ ID No: 27), and Complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);
(5) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and

Complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);

(6) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and Complementarity-determining regions of the light chain, CDR1 (KASENVGTYVS, SEQ ID No: 52), CDR2 (GASNRYT, SEQ ID No: 53), and CDR3 (GQSYSYPLT, SEQ ID No: 54);

(7) Complementarity-determining regions of heavy chains, CDR1 (DYYMD, SEQ ID No: 9), CDR2 (YIYPNNAGTS YNQKFKG, SEQ ID No: 74), and CDR3 (SGYGNYYTMDY, SEQ ID No: 11), and Complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(8) Complementarity-determining regions of the heavy chain, CDR1 (DYYMD, SEQ ID No: 9), CDR2 (YIYPNKGGTS YNQKFKG, SEQ ID No: 76), and CDR3 (SGYGNYYTMDY, SEQ ID No: 11), and Complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(9) Complementarity-determining regions of the heavy chain, CDR1 (DYYMD, SEQ ID No: 17), CDR2 (YIYPNNAGTS YNQKFKG, SEQ ID No: 78), and CDR3 (GGYYSYYSYDY, SEQ ID No: 19), and Complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(10) Complementarity-determining regions of heavy chains, CDR1 (DYYMD, SEQ ID No: 17), CDR2 (YIYPNKGGTS YNQKFKG, SEQ ID No: 80), and CDR3 (GGYYSYYSYDY, SEQ ID No: 19), and Complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(11) Complementarity-determining regions of heavy chains, CDR1 (DYYMD, SEQ ID No: 25), CDR2 (YIYPNNAGTS YNQKFKG, SEQ ID No: 82), and CDR3 (SGYKAYYAMDY, SEQ ID No: 27), and Complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(12) Complementarity-determining regions of heavy chains, CDR1 (DYYMD, SEQ ID No: 25), CDR2 (YIYPNKGGTS YNQKFKG, SEQ ID No: 84), and CDR3 (SGYKAYYAMDY, SEQ ID No: 27), and Complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30).

[13] The antibody or antibody derivative according to [12], wherein the antibody derivative is selected from a modified antibody selected from a humanized antibody, a chimeric antibody, a single chain Fv antibody (a scFv antibody), Fab, Fab', F(ab')2, Fc effector function modified antibody, IgG1LALA, IgG1_N297A, IgG4_S228P, or a functional fragment thereof.

[14] The antibody or antibody derivative according to [12] or [13], wherein the amino acid sequence of the heavy chain variable region VH domain of the half-molecule of the antibody or antibody derivative is selected from the group consisting of:

(VH-1a) the amino acid sequence of SEQ ID No: 7 or an amino acid sequence comprising one or several amino acid substitutions (e.g., conservative substitutions), insertions, or deletions in regions of the amino acid sequence of SEQ ID No: 7 other than CDR1 (SEQ ID No: 1), CDR2 (SEQ ID No: 2), and CDR3 (SEQ ID No: 3),

(VH-1b) the amino acid sequence of SEQ ID No: 49 or an amino acid sequence comprising one or several amino acid substitutions (e.g., conservative substitutions), insertions, or deletions in regions of the amino acid sequence of SEQ ID No: 49 other than CDR1 (SEQ ID No: 1), CDR2 (SEQ ID No: 2), and CDR3 (SEQ ID No: 3),

(VH-1c) the amino acid sequence of SEQ ID No: 57 or an amino acid sequence comprising one or several amino acid substitutions (e.g., conservative substitutions), insertions, or deletions in regions of the amino acid sequence of SEQ ID No: 57 other than CDR1 (SEQ ID No: 1), CDR2 (SEQ ID No: 2), and CDR3 (SEQ ID No: 3),

(VH-2a) the amino acid sequence of SEQ ID No: 15 or an amino acid sequence comprising one or several amino acid substitutions (e.g., conservative substitutions), insertions, or deletions in regions of the amino acid sequence of SEQ ID No: 15 other than CDR1 (SEQ ID No: 9), CDR2 (SEQ ID No: 10), and CDR3 (SEQ ID No: 11),

(VH-2b) the amino acid sequence of SEQ ID No: 62 or an amino acid sequence comprising one or several amino acid substitutions (e.g., conservative substitutions), insertions, or deletions in regions of the amino acid sequence of SEQ ID No: 62 other than CDR1 (SEQ ID No: 9), CDR2 (SEQ ID No: 10), and CDR3 (SEQ ID No: 11),

(VH-3a) the amino acid sequence of SEQ ID No: 23 or an amino acid sequence comprising one or several amino acid substitutions (e.g., conservative substitutions), insertions, or deletions in regions of the amino acid sequence of SEQ ID No: 23 other than CDR1 (SEQ ID No: 17), CDR2 (SEQ ID No: 18), and CDR3 (SEQ ID No: 19),

(VH-3b) the amino acid sequence of SEQ ID No: 66 or an amino acid sequence comprising one or several amino acid substitutions (e.g., conservative substitutions), insertions, or deletions in regions of the amino acid sequence of SEQ ID No: 66 other than CDR1 (SEQ ID No: 17), CDR2 (SEQ ID No: 18), and CDR3 (SEQ ID No: 19),

(VH-4a) the amino acid sequence of SEQ ID No: 31 or an amino acid sequence comprising one or several amino acid substitutions (e.g., conservative substitutions), insertions, or deletions in regions of the amino acid sequence of SEQ ID No: 31 other than CDR1 (SEQ ID No: 25), CDR2 (SEQ ID No: 26), and CDR3 (SEQ ID No: 27),

(VH-4b) the amino acid sequence of SEQ ID No: 70 or an amino acid sequence comprising one or several amino acid substitutions (e.g., conservative substitutions), insertions, or deletions in regions of the amino acid sequence of SEQ ID No: 70 other than CDR1 (SEQ ID No: 25), CDR2 (SEQ ID No: 26), and CDR3 (SEQ ID No: 27),

(VH-5a) the amino acid sequence of SEQ ID No: 36 or an amino acid sequence comprising one or several amino acid substitutions (e.g., conservative substitutions), insertions, or deletions in regions of the amino acid sequence of SEQ ID No: 36 other than CDR1 (SEQ ID No: 33), CDR2 (SEQ ID No: 34), and CDR3 (SEQ ID No: 35),

(VH-5b) the amino acid sequence of SEQ ID No: 51 or an amino acid sequence comprising one or several amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35),

(VH-5c) the amino acid sequence of SEQ ID No: 59 or an amino acid sequence comprising one or several amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35),

(VH-6a) the amino acid sequence of SEQ ID No: 55 or an amino acid sequence comprising one or several amino acid substitutions (e.g., conservative substitutions), insertions, or deletions in regions of the amino acid sequence of SEQ ID No: 55 other than CDR1 (SEQ ID No: 33), CDR2 (SEQ ID No: 34), and CDR3 (SEQ ID No: 35),

(VH-6b) the amino acid sequence of SEQ ID No: 51 or an amino acid sequence comprising one or several amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35),

(VH-6c) the amino acid sequence of SEQ ID No: 59 or an amino acid sequence comprising one or several amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35),

(VH-7) the amino acid sequence of SEQ ID No: 75 or an amino acid sequence comprising one or several amino acid substitutions (e.g., conservative substitutions), insertions, or deletions in regions of the amino acid sequence of SEQ ID No: 75 other than CDR1 (SEQ ID No: 9), CDR2 (SEQ ID No: 74), and CDR3 (SEQ ID No: 11),

(VH-8) the amino acid sequence of SEQ ID No: 77 or an amino acid sequence comprising one or several amino acid substitutions (e.g., conservative substitutions), insertions, or deletions in regions of the amino acid sequence of SEQ ID No: 77 other than CDR1 (SEQ ID No: 9), CDR2 (SEQ ID No: 76), and CDR3 (SEQ ID No: 11),

(VH-9) the amino acid sequence of SEQ ID No: 79 or an amino acid sequence comprising one or several amino acid substitutions (e.g., conservative substitutions), insertions, or deletions in regions of the amino acid sequence of SEQ ID No: 79 other than CDR1 (SEQ ID No: 17), CDR2 (SEQ ID No: 78), and CDR3 (SEQ ID No: 19),

(VH-10) the amino acid sequence of SEQ ID No: 81 or an amino acid sequence comprising one or several amino acid substitutions (e.g., conservative substitutions), insertions, or deletions in regions of the amino acid sequence of SEQ ID No: 81 other than CDR1 (SEQ ID No: 17), CDR2 (SEQ ID No: 80), and CDR3 (SEQ ID No: 19),

(VH-11) the amino acid sequence of SEQ ID No: 83 or an amino acid sequence comprising one or several amino acid substitutions (e.g., conservative substitutions), insertions, or deletions in regions of the amino acid sequence of SEQ ID No: 83 other than CDR1 (SEQ ID No: 25), CDR2 (SEQ ID No: 82), and CDR3 (SEQ ID No: 27),

(VH-12) the amino acid sequence of SEQ ID No: 85 or an amino acid sequence comprising one or several amino acid substitutions (e.g., conservative substitutions), insertions, or deletions in regions of the amino acid sequence of SEQ ID No: 85 other than CDR1 (SEQ ID No: 25), CDR2 (SEQ ID No: 84), and CDR3 (SEQ ID No: 27).

[15] The antibody or antibody derivative according to any one of [12]-[14], wherein the amino acid sequence of the light chain variable region VL domain of the half-molecule of the antibody or the antibody derivative is selected from the group consisting of:

(VL-1a) the amino acid sequence of SEQ ID No: 8 or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6),
(VL-1b) the amino acid sequence of SEQ ID No: 58 or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6),
(VL-1c) the amino acid sequence of SEQ ID No: 50 or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6),
(VL-2a) the amino acid sequence of SEQ ID No: 16 or an amino acid sequence comprising one or several amino acid substitutions (e.g., conservative substitutions), insertions, or deletions in regions of the amino acid sequence of SEQ ID No: 16 other than CDR1 (SEQ ID No: 12), CDR2 (SEQ ID No: 13), and CDR3 (SEQ ID No: 14),
(VL-2b) the amino acid sequence of SEQ ID No: 63 or an amino acid sequence comprising one or several amino acid substitutions (e.g., conservative substitutions), insertions, or deletions in regions of the amino acid sequence of SEQ ID No: 63 other than CDR1 (SEQ ID No: 12), CDR2 (SEQ ID No: 13), and CDR3 (SEQ ID No: 14),
(VL-2c) the amino acid sequence of SEQ ID No: 64 or an amino acid sequence comprising one or several amino acid substitutions (e.g., conservative substitutions), insertions, or deletions in regions of the amino acid sequence of SEQ ID No: 64 other than CDR1 (SEQ ID No: 12), CDR2 (SEQ ID No: 13), and CDR3 (SEQ ID No: 14),
(VL-2d) the amino acid sequence of SEQ ID No: 65 or an amino acid sequence comprising one or several amino acid substitutions (e.g., conservative substitutions), insertions, or deletions in regions of the amino acid sequence of SEQ ID No: 65 other than CDR1 (SEQ ID No: 12), CDR2 (SEQ ID No: 13), and CDR3 (SEQ ID No: 14),
(VL-3a) the amino acid sequence of SEQ ID No: 24 or an amino acid sequence comprising one or several amino acid substitutions (e.g., conservative substitutions), insertions, or deletions in regions of the amino acid sequence of SEQ ID No: 24 other than CDR1 (SEQ ID No: 20), CDR2 (SEQ ID No: 21), and CDR3 (SEQ ID No: 22),
(VL-3b) the amino acid sequence of SEQ ID No: 67 or an amino acid sequence comprising one or several amino acid substitutions (e.g., conservative substitutions), insertions, or deletions in regions of the amino acid sequence of SEQ ID No: 67 other than CDR1 (SEQ ID No: 20), CDR2 (SEQ ID No: 21), and CDR3 (SEQ ID No: 22),
(VL-3c) the amino acid sequence of SEQ ID No: 68 or an amino acid sequence comprising one or several amino acid substitutions (e.g., conservative substitutions), insertions, or deletions in regions of the amino acid sequence of SEQ ID No: 68 other than CDR1 (SEQ ID No: 20), CDR2 (SEQ ID No: 21), and CDR3 (SEQ ID No: 22),
(VL-3d) the amino acid sequence of SEQ ID No: 69 or an amino acid sequence comprising one or several amino acid substitutions (e.g., conservative substitutions), insertions, or deletions in regions of the amino acid sequence of SEQ ID No: 69 other than CDR1 (SEQ ID No: 20), CDR2 (SEQ ID No: 21), and CDR3 (SEQ ID No: 22),
(VL-4a) the amino acid sequence of SEQ ID No: 32 or an amino acid sequence comprising one or several amino acid substitutions (e.g., conservative substitutions), insertions, or deletions in regions of the amino acid sequence of SEQ ID No: 32 other than CDR1 (SEQ ID No: 28), CDR2 (SEQ ID No: 29), and CDR3 (SEQ ID No: 30),
(VL-4b) the amino acid sequence of SEQ ID No: 71 or an amino acid sequence comprising one or several amino acid substitutions (e.g., conservative substitutions), insertions, or deletions in regions of the amino acid sequence of SEQ ID No: 71 other than CDR1 (SEQ ID No: 28), CDR2 (SEQ ID No: 29), and CDR3 (SEQ ID No: 30),
(VL-4c) the amino acid sequence of SEQ ID No: 72 or an amino acid sequence comprising one or several amino acid substitutions (e.g., conservative substitutions), insertions, or deletions in regions of the amino acid sequence of SEQ ID No: 72 other than CDR1 (SEQ ID No: 28), CDR2 (SEQ ID No: 29), and CDR3 (SEQ ID No: 30),
(VL-4d) the amino acid sequence of SEQ ID No: 73 or an amino acid sequence comprising one or several amino acid substitutions (e.g., conservative substitutions), insertions, or deletions in regions of the amino acid sequence of SEQ ID No: 73 other than CDR1 (SEQ ID No: 28), CDR2 (SEQ ID No: 29), and CDR3 (SEQ ID No: 30),
(VL-5a) the amino acid sequence of SEQ ID No: 8 or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6),
(VL-5b) the amino acid sequence of SEQ ID No: 58 or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6),
(VL-5c) the amino acid sequence of SEQ ID No: 50 or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6),

(VL-6a) the amino acid sequence of SEQ ID No: 56 or an amino acid sequence comprising one or several amino acid substitutions (e.g., conservative substitutions), insertions, or deletions in regions of the amino acid sequence of SEQ ID No: 56 other than CDR1 (SEQ ID No: 52), CDR2 (SEQ ID No: 53), and CDR3 (SEQ ID No: 54),
(VL-6b) the amino acid sequence of SEQ ID No: 60 or an amino acid sequence comprising one or several amino acid substitutions (e.g., conservative substitutions), insertions, or deletions in regions of the amino acid sequence of SEQ ID No: 60 other than CDR1 (SEQ ID No: 52), CDR2 (SEQ ID No: 53), and CDR3 (SEQ ID No: 54),
(VL-6c) the amino acid sequence of SEQ ID No: 61 or an amino acid sequence comprising one or several amino acid substitutions (e.g., conservative substitutions), insertions, or deletions in regions of the amino acid sequence of SEQ ID No: 61 other than CDR1 (SEQ ID No: 52), CDR2 (SEQ ID No: 53), and CDR3 (SEQ ID No: 54).

[16] The antibody or antibody derivative according to [12] or [13], wherein the variable region of the half-molecule composed of the single chain Fv antibody (scFv) is selected from:

(1) an amino acid sequence in which the heavy chain variable region of (VH-1a), (VH-1b) or (VH-1c) and the light chain variable region of (VL-1a), (VL-1b) or (VL-1c) are connected via a linker,
(2) an amino acid sequence in which the heavy chain variable region of (VH-2a) or (VH-2b) and the light chain variable region of (VL-2a), (VL-2b), (VL-2c) or (VL-2d) are connected via a linker,
(3) an amino acid sequence in which the heavy chain variable region of (VH-3a) or (VH-3b) and the light chain variable region of (VL-3a), (VL-3b), (VL-3c) or (VL-3d) are connected via a linker,
(4) an amino acid sequence in which the heavy chain variable region of (VH-4a) or (VH-4b) and the light chain variable region of (VL-4a), (VL-4b), (VL-4c) or (VL-4d) connected via a linker, and
(5) an amino acid sequence in which the heavy chain variable region of (VH-5a), (VH-5b) or (VH-5c) and the light chain variable region of (VL-5a), (VL-5b) or (VL-5c) are connected via a linker,
(6) an amino acid sequence in which the heavy chain variable region of (VH-6a), (VH-6b) or (VH-6c) and the light chain variable region of (VL-6a), (VL-6b) or (VL-6c) are connected via a linker,
(7) an amino acid sequence in which the heavy chain variable region of (VH-7) and the light chain variable region of (VL-2a), (VL-2b), (VL-2c) or (VL-2d) are connected via a linker,
(8) an amino acid sequence in which the heavy chain variable region of (VH-8) and the light chain variable region of (VL-2a), (VL-2b), (VL-2c) or (VL-2d) are connected via a linker,
(9) an amino acid sequence in which the heavy chain variable region of (VH-9) and the light chain variable region of (VL-3a), (VL-3b), (VL-3c) or (VL-3d) are connected via a linker,
(10) an amino acid sequence in which the heavy chain variable region of (VH-10) and the light chain variable region of (VL-3a), (VL-3b), (VL-3c) or (VL-3d) are connected via a linker,
(11) an amino acid sequence in which the heavy chain variable region of (VH-11) and the light chain variable region of (VL-4a), (VL-4b), (VL-4c) or (VL-4d) are connected via a linker,
(12) an amino acid sequence in which the heavy chain variable region of (VH-12) and the light chain variable region of (VL-4a), (VL-4b), (VL-4c) or (VL-4d) are connected via a linker.

[17] The antibody or antibody derivative according to any one of [12] to [16], wherein the enhancement of immune activity is activation of T cells selected from the group consisting of removal of immunosuppressive signals, relieving T cell exhaustion, proliferation of T cells, increase in cytotoxicity of T cells against cancer cells, and promotion of T cell cytokine secretion;
[18] The antibody or antibody derivative according to any one of [12] to [17] wherein the antibody or antibody derivative induces cytotoxicity against cancer cells but not against normal cells;
[19] The antibody or antibody derivative according to any one of [12] to [18], wherein the cancer cell is selected from the group consisting of melanoma, breast cancer, lung cancer, colon cancer, gastric cancer, pancreatic cancer, liver cancer, and blood cancer];
[20] A pharmaceutical composition for the treatment of cancer comprising the antibody or antibody derivative according to any one of [1] to [19];
[21] The pharmaceutical composition according to [20], wherein the cancer is selected from the group consisting of melanoma, breast cancer, lung cancer, colon cancer, stomach cancer, pancreatic cancer, liver cancer, and blood cancer;
[22] A method for predicting increased cytotoxicity against cancer cells, comprising

a step of bringing cancer cells collected from a subject into contact in vitro with the antibody or antibody derivative according to any one of [1] to [19], and
a step of measuring whether the cell viability of the cancer cells is decreased or a step of measuring whether the secretion of an immune-activating substance is enhanced under culture conditions;

[23] The method according to [22], wherein whether the cell viability of cancer cells is decreased or whether immune cells derived from peripheral blood lymphocytes are activated, is measured in the presence of peripheral blood lymphocytes of the same subject;

[24] The method according to [22] or [23], wherein the enhancement of cytotoxicity against cancer cells in vitro when the antibody or antibody derivative according to any one of [1] to [19] is administered to that subject is predicted based on the in vitro cytotoxicity against cancer cells collected from the subject;

[25] The method according to [22] or [23], wherein the enhancement of cytotoxicity against cancer cells when the antibody or antibody derivative according to any one of [1] to [19] is administered to the subject is predicted based on the enhancement of secretion of an immune activating substance in vitro;

[26] A measurement kit comprising the antibody or antibody derivative according to any one of [1] to [19], for measuring in vitro the cytotoxicity against cancer cells from a subject, secretion of immune activating substances against the cancer cells, or activation of immune cells against cancer cells of the antibody or antibody derivative.

Advantageous Effects of the Invention

An antibody or antibody derivative (an antibody etc.) obtained by the present invention inhibits the function of Tim-3 antigen expressed on cancer cells or immune cells and activate immune cells through various mechanisms, for example by relieving the exhaustion of immune cells (especially T cells), thereby demonstrating an effect of tumor growth suppression and an effect of cancer treatment.

The present invention can be widely used for a therapeutic and diagnostic agent for cancer. The antibody etc. according to the present invention is expected to be particularly effective in combination therapy with other immunotherapies, since it exhibits the effect of releasing immune cell exhaustion.

Brief Description of the Drawings

[Figure 1] Figure 1 shows the results of epitope binning of antibodies to confirm competition and non-competition between antibodies obtained in Example 1.

[Figure 2] Figure 2 shows the results of binding analysis of anti-human Tim-3 antibodies selected in Example 2 using Jurkat cells stably expressing human Tim3 or Expi293 cells transiently expressing human Tim-3.

[Figure 3] Figure 3 shows the results of flow cytometry analysis of the binding of the anti-human Tim-3 antibodies selected in Example 2 to human Tim-3 on activated T cell membranes.

[Figure 4] Figure 4 shows the results of ELISA analysis of the inhibitory ability of the anti-human Tim-3 antibodies selected in Example 2 to the binding between human Tim-3 and human Galectin-9.

[Figure 5] Figure 5 shows the results of flow cytometry analysis of the inhibitory ability of the anti-human Tim-3 antibodies selected in Example 2 on the binding between human Tim-3 and phosphatidylserine.

[Figure 6] Figure 6 shows the results of binding analysis of the anti-human Tim-3 biparatopic antibodies produced in Example 5 to Jurkat cells stably expressing human Tim-3 or Expi293 cells transiently expressing human Tim-3.

[Figure 7] Figure 7 shows the results of flow cytometry analysis of the binding of the anti-human Tim-3 biparatopic antibodies produced in Example 5 to human Tim-3 on activated T cell membranes.

[Figure 8] Figure 8 shows the results of ELISA analysis of the inhibitory ability of the anti-human Tim-3 biparatopic antibodies produced in Example 5 to the binding between human Tim-3 and human Galectin-9.

[Figure 9] Figure 9 shows the results of flow cytometry analysis of the inhibitory ability of the anti-human Tim-3 biparatopic antibodies produced in Example 5 to the binding between human Tim-3 and phosphatidylserine.

[Figure 10-1] Figure 10-1 shows the changes in the number of IFN-g positive T cells when the effect of the anti-human Tim-3 monoclonal antibodies or the anti-human Tim-3 biparatopic antibodies on human peripheral blood T cell activation was evaluated by the activation of peripheral blood mononuclear cells (PBMCs) with Staphylococcus aureus enterotoxin B (SEB).

[Figure 10-2] Figure 10-2 shows the expression level of Tim-3 on IFN-g positive T cells (MFI value) when the effect of the anti-human Tim-3 monoclonal antibodies or the anti-human Tim-3 biparatopic antibodies of the present invention on human peripheral blood T cell activation was evaluated by peripheral blood mononuclear cell (PBMC) activation with Staphylococcus Enterotoxin B (SEB).

[Figure 11-1] Figure 11-1 shows the results of the evaluation of whether the anti-human Tim-3 monoclonal antibodies or the anti-human Tim-3 biparatopic antibodies inhibit the Galectin-9 induced apoptosis of human peripheral blood T cells.

[Figure 11-2] Figure 11-2 shows the results of the evaluation of whether the anti-human Tim-3 monoclonal antibodies or the anti-human Tim-3 biparatopic antibodies inhibit the Galectin-9 induced apoptosis of human peripheral blood T cells.

[Figure 12] Figure 12 shows the results of binding analysis using the humanized anti-human Tim-3 antibodies of the invention produced in Example 11 with Jurkat cells stably expressing human Tim-3 or Expi293 cells transiently expressing human Tim-3.

[Figure 13] Figure 13 shows the results of flow cytometry analysis of the binding of the humanized anti-human Tim-3 antibodies of the invention produced in Example 11 to human Tim-3 on activated T cell membranes.

[Figure 14] Figure 14 shows the results of ELISA analysis of the inhibitory ability of the humanized anti-human Tim-3 antibodies of the invention produced in Example 11 to the binding between human Tim-3 and human Galectin-9.

[Figure 15] Figure 15 shows the results of flow cytometry analysis of the inhibitory ability of the humanized anti-human Tim-3 antibodies of the invention produced in Example 11 to the binding between human Tim-3 and phosphatidylserine.

[Figure 16-1] Figure 16-1 shows the changes in the number of the IFN-g positive T cells when the effect of the humanized anti-human Tim-3 antibodies of the invention produced in Example 11 on human peripheral blood T cell activation was evaluated by the activation of peripheral blood mononuclear cells (PBMCs) by Staphylococcus aureus enterotoxin B (SEB).

[Figure 16-2] Figure 16-2 shows the expression level of the Tim-3 on the IFN-g positive T cells (MFI values) when the effect of the humanized anti-human Tim-3 antibodies of the invention produced in Example 11 on human peripheral blood T cell activation was evaluated by peripheral blood mononuclear cells (PBMCs) activation by Staphylococcus aureus enterotoxin B (SEB) .

[Figure 17] Figure 17 shows the results of binding analysis using the humanized anti-human Tim-3 biparatopic antibodies produced in Example 15 with Jurkat cells stably expressing human Tim-3 or Expi293 cells transiently expressing human Tim-3.

[Figure 18] Figure 18 shows the results of flow cytometry analysis of the binding of the humanized anti-human Tim-3 biparatopic antibodies produced in Example 15 to human Tim-3 on activated T cell membranes.

[Figure 19] Figure 19 shows the results of ELISA analysis of the inhibitory ability of the humanized anti-human Tim-3 biparatopic antibodies produced in Example 15 to the binding between human Tim-3 and human Galectin-9.

[Figure 20] Figure 20 shows the results of flow cytometry analysis of the inhibitory ability of the humanized anti-human Tim-3 biparatopic antibodies produced in Example 15 to the binding between human Tim-3 and phosphatidylserine.

[Figure 21-1] Figure 21-1 shows the changes in the number of IFN-g-positive T cells when the effect of the humanized anti-human Tim-3 monoclonal antibodies or the humanized anti-human Tim-3 biparatopic antibodies on human peripheral blood T cell activation was evaluated by activation of peripheral blood mononuclear cells (PBMCs) with Staphylococcus aureus enterotoxin B (SEB).

[Figure 21-2] Figure 21-2 shows the expression levels of Tim-3 on IFN-g-positive T cells (MFI values) when the effect of the humanized anti-human Tim-3 monoclonal antibodies or the humanized anti-human Tim-3 biparatopic antibodies of the present invention on human peripheral blood T cell activation was evaluated by activation of peripheral blood mononuclear cells (PBMCs) with Staphylococcus aureus enterotoxin B (SEB).

[Figure 22] Figure 22 shows the results of the evaluation of whether the humanized antihuman Tim-3 monoclonal antibodies or the humanized anti-human Tim-3 biparatopic antibodies of the present invention inhibit the Galectin-9-induced apoptosis of human peripheral blood T cells.

[Figure 23] Figure 23 shows the rate of CMV-tetramer positive cells and IFN$\gamma$ and TNF$\alpha$ concentrations in the culture supernatant when the effect of the humanized anti-human Tim-3 monoclonal antibodies or the humanized anti-human Tim-3 biparatopic antibodies of the present invention on the activation of human peripheral blood T cell was evaluated by activation of PBMCs with CMV antigen peptides.

[Figure 24] Figure 24 shows the effects of the anti-human Tim-3 biparatopic antibodies of the present invention on the activation of peripheral blood T cells and the antitumor effects in a coculture system of PBMCs and tumor cells, which were examined by the level of luminescence from ATP derived from live tumor cells, and by IFN$\gamma$ and TNF$\alpha$ concentrations in the culture supernatants.

[Figure 25] Figure 25 shows the changes in the average tumor volume and the changes in the tumor volume in the individual mice in each treatment group as the anti-tumor effect of the humanized antihuman Tim-3 biparatopic antibodies in a mouse model.

[Figure 26] Figure 26 shows the analysis results of the lymphocytes infiltrating tumor tissue collected from mice as an anti-tumor effect of the humanized anti-human Tim-3 biparatopic antibodies of the present invention in a mouse model.

[Figure 27] Figure 27 shows the structure of the human Tim-3 IgV domain and the epitope site of the Hu003_13_F20_scFv antibody obtained from the complex of the human Tim-3 IgV domain and the Hu003_13_F20_scFv antibody.

[Figure 28] Figure 28 shows the structure of the human Tim-3 IgV domain and the epitope site of the Hu149_83_scFv antibody obtained from the complex of the human Tim-3 IgV domain and the Hu149_83_scFv antibody.
[Figure 29] Figure 29 shows the results of modeling the binding of the humanized biparatopic antibody Hu149003 antibody to human Tim-3.

Description of Embodiments

In one embodiment, the present invention can provide a binding substance, especially an antibody or a humanized antibody derivative (hereinafter simply referred to as "antibody etc.") against the Tim-3 antigen, which has binding properties against the Tim-3 antigen on cancer cells or immune cells and has an ability to remove the activity of immunosuppressive signal against cancer cells. The antibody etc. may be an antibody composed of homodimeric half-molecules or an antibody composed of heterodimeric half-molecules. The binding substance, especially the antibody, of the present invention can be used to eliminate immunosuppressive signals against cancer cells, to increase immune activity against cancer cells, and consequently for treatment against cancer cells, for inhibition of cancer cell growth, for shrinking or elimination of tumors, and also be used to activate T cells that when T cells having the ability to kill cancer cells have become exhausted.

Structure of a homodimeric antibody and antibody derivative (antibody etc.)

In a first embodiment, the present invention provides a homodimeric antibody etc. having binding ability to the Tim-3 antigen. This homodimeric antibody etc. is characterized in that it binds to the Tim-3 antigen and activates T cells that have cytotoxic activity to cancer cells.
The homodimeric antibody etc. in the first embodiment of the invention may bind to the Tim-3 antigen to suppress the function of the Tim-3 antigen, to remove immunosuppressive signals against cancer cells, to suppress the function of the Tim-3 antigen expressed on immune cells (especially T cells), to activate immune cells, and to exert growth suppression effect on the tumors and a therapeutic effect on cancers.
When simply referring to "antibody" in this invention, the antibody may be derived from any species of mammals, and the species from which the antibody is derived are not limited to humans, but may be derived from mouse, rat, guinea pig, hamster, rabbit, VHH antibody (variable domain of heavy chain of heavy chain of heavy chain antibodies) derived from camelid animal (such as camel, and alpaca), ostrich antibodies, and so on. For example, in the case of the human antibody, this includes various isotypes such as IgG, IgM, IgA, IgD, IgE, etc.
In the first embodiment of the present invention, it may also be an "antibody derivative" of the above antibody, as long as it has the functional characteristics of binding to the Tim-3 antigen and having the activity of removing immunosuppressive signal against cancer cells. When referring to the antibody derivative in the present invention, for example, the present invention may provide an antibody derivative which is a combination of the amino acid sequence of the six CDRs (complementarity determining regions CDR1-CDR3 of the heavy chain and CDR1-CDR3 of the light chain) for any of IgG, IgM, IgA, IgD, IgE, etc. of the above antibodies, the amino acid sequence of the human antibody-derived constant regions, and other amino acid sequence comprising a combination of the amino acid sequence from the original antibody and that from the human antibody. Examples of the antibody derivative includes, but not limited to, a humanized antibody in which the amino acid sequences other than the complementarity determining regions (CDRs) of the above described "antibody" are replaced with those derived from a human antibody, a chimeric antibody such as those in which the amino acid sequences of the variable region of the above antibody are linked to the constant regions of a human antibody, or a single chain antibody (e.g., scFv) in which the heavy chain and light chain are linked by a linker.
The homodimeric antibody etc. of the invention is characterized, by way of example, by having a total of six amino acid sequences of CDRs 1-3 of the heavy chain and CDRs 1-3 of the light chain which are identical to those of the antibody having binding activity to the Tim-3 antigen. Examples of the amino sequences of six specific CDRs of a half-molecule of the antibody having binding activity to the Tim-3 antigen may include those containing complementarity-determining regions of the heavy and the light chains selected from the group consisting of (1) to (12) below:

(1) Complementarity-determining regions of the heavy chain, CDR1 (SYYMS, SEQ ID No: 1), CDR2 (TISNSGGSIYYLDSVKD, SEQ ID No: 2), and CDR3 (DPYYSNYVPMDY, SEQ ID No: 3), and
complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);
(2) Complementarity-determining regions of heavy chains, CDR1 (DYYMD, SEQ ID No: 9), CDR2 (YIYPNNGGTSYNQKFKG, SEQ ID No: 10), and CDR3 (SGYGNYYTMDY, SEQ ID No: 11), and
complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);
(3) Complementarity-determining regions of the heavy chains, CDR1 (DYYMD, SEQ ID No: 17), CDR2 (YIYPNNGGTSYNQKFKG, SEQ ID No: 18), and CDR3 (GGYYSYYSYDY, SEQ ID No: 19), and

complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(4) Complementarity-determining regions of the heavy chains, CDR1 (DYYMD, SEQ ID No: 25), CDR2 (YIYPNNGGTSYNQKFKG, SEQ ID No: 26), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(5) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);

(6) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASENVGTYVS, SEQ ID No: 52), CDR2 (GASNRYT, SEQ ID No: 53), and CDR3 (GQSYSYPLT, SEQ ID No: 54);

(7) Complementarity-determining regions of heavy chains, CDR1 (DYYMD, SEQ ID No: 9), CDR2 (YIYPNNAGTS YNQKFKG, SEQ ID No: 74), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(8) Complementarity-determining regions of heavy chains, CDR1 (DYYMD, SEQ ID No: 9), CDR2 (YIYPNKGGTS YNQKFKG, SEQ ID No: 76), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(9) Complementarity-determining regions of the heavy chain, CDR1 (DYYMD, SEQ ID No: 17), CDR2 (YIYPNNAGTS YNQKFKG, SEQ ID No: 78), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(10) Complementarity-determining regions of heavy chains, CDR1 (DYYMD, SEQ ID No: 17), CDR2 (YIYPNKGGTS YNQKFKG, SEQ ID No: 80), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(11) Complementarity-determining regions of heavy chains, CDR1 (DYYMD, SEQ ID No: 25), CDR2 (YIYPNNAGTS YNQKFKG, SEQ ID No: 82), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(12) Complementarity-determining regions of heavy chains, CDR1 (DYYMD, SEQ ID No: 25), CDR2 (YIYPNKGGTS YNQKFKG, SEQ ID No: 84), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

but may also include other antibody etc. identified with CDR combinations other than those listed above, as long as the antibody etc. is characterized as having binding activity to the Tim-3 antigen.

Specific antibody etc. of the first embodiment

In this embodiment of the present invention, the antibody etc. can be specified, for example, as having an amino acid sequence selected from the group consisting of the following (VH-1) to (VH-12) (and the branch numbers of each number) of the heavy chain variable region VH domains:

(VH-1a) the amino acid sequence of SEQ ID No: 7 or an amino acid sequence comprising one or several amino acid substitutions (e.g., conservative substitutions), insertions, or deletions, in regions of the amino acid sequence of SEQ ID No: 7 other than CDR1 (SEQ ID No: 1), CDR2 (SEQ ID No: 2), and CDR3 (SEQ ID No: 3);

(VH-1b) the amino acid sequence of SEQ ID No: 49 or an amino acid sequence comprising one or several amino acid substitutions (e.g., conservative substitutions), insertions, or deletions, in regions of the amino acid sequence of SEQ ID No: 49 other than CDR1 (SEQ ID No: 1), CDR2 (SEQ ID No: 2), and CDR3 (SEQ ID No: 3);

(VH-1c) the amino acid sequence of SEQ ID No: 57 or an amino acid sequence comprising one or several amino acid substitutions (e.g., conservative substitutions), insertions, or deletions, in regions of the amino acid sequence of SEQ ID No: 57 other than CDR1 (SEQ ID No: 1), CDR2 (SEQ ID No: 2), and CDR3 (SEQ ID No: 3);

(VH-2a) the amino acid sequence of SEQ ID No: 15 or an amino acid sequence comprising one or several amino acid substitutions (e.g., conservative substitutions), insertions, or deletions, in regions of the amino acid sequence of SEQ ID No: 15 other than CDR1 (SEQ ID No: 9), CDR2 (SEQ ID No: 10), and CDR3 (SEQ ID No: 11);

(VH-2b) the amino acid sequence of SEQ ID No: 62 or an amino acid sequence comprising one or several amino acid substitutions (e.g., conservative substitutions), insertions, or deletions, in regions of the amino acid sequence of SEQ ID No: 62 other than CDR1 (SEQ ID No: 9), CDR2 (SEQ ID No: 10), and CDR3 (SEQ ID No: 11);

(VH-3a) the amino acid sequence of SEQ ID No: 23 or an amino acid sequence comprising one or several amino acid substitutions (e.g., conservative substitutions), insertions, or deletions, in regions of the amino acid sequence of SEQ ID No: 23 other than CDR1 (SEQ ID No: 17), CDR2 (SEQ ID No: 18), and CDR3 (SEQ ID No: 19);

(VH-3b) the amino acid sequence of SEQ ID No: 66 or an amino acid sequence comprising one or several amino acid substitutions (e.g., conservative substitutions), insertions, or deletions, in regions of the amino acid sequence of SEQ ID No: 66 other than CDR1 (SEQ ID No: 17), CDR2 (SEQ ID No: 18), and CDR3 (SEQ ID No: 19);

(VH-4a) the amino acid sequence of SEQ ID No: 31 or an amino acid sequence comprising one or several amino acid substitutions (e.g., conservative substitutions), insertions, or deletions, in regions of the amino acid sequence of SEQ ID No: 31 other than CDR1 (SEQ ID No: 25), CDR2 (SEQ ID No: 26), and CDR3 (SEQ ID No: 27);

(VH-4b) the amino acid sequence of SEQ ID No: 70 or an amino acid sequence comprising one or several amino acid substitutions (e.g., conservative substitutions), insertions, or deletions, in regions of the amino acid sequence of SEQ ID No: 70 other than CDR1 (SEQ ID No: 25), CDR2 (SEQ ID No: 26), and CDR3 (SEQ ID No: 27);

(VH-5a) the amino acid sequence of SEQ ID No: 36 or an amino acid sequence comprising one or several amino acid substitutions (e.g., conservative substitutions), insertions, or deletions, in regions of the amino acid sequence of SEQ ID No: 36 other than CDR1 (SEQ ID No: 33), CDR2 (SEQ ID No: 34), and CDR3 (SEQ ID No: 35);

(VH-5b) the amino acid sequence of SEQ ID No: 51 or an amino acid sequence comprising one or several amino acid substitutions (e.g., conservative substitutions), insertions, or deletions, in regions of the amino acid sequence of SEQ ID No: 51 other than CDR1 (SEQ ID No: 33), CDR2 (SEQ ID No: 34), and CDR3 (SEQ ID No: 35);

(VH-5c) the amino acid sequence of SEQ ID No: 59 or an amino acid sequence comprising one or several amino acid substitutions (e.g., conservative substitutions), insertions, or deletions, in regions of the amino acid sequence of SEQ ID No: 59 other than CDR1 (SEQ ID No: 33), CDR2 (SEQ ID No: 34), and CDR3 (SEQ ID No: 35);

(VH-6a) the amino acid sequence of SEQ ID No: 55 or an amino acid sequence comprising one or several amino acid substitutions (e.g., conservative substitutions), insertions, or deletions, in regions of the amino acid sequence of SEQ ID No: 55 other than CDR1 (SEQ ID No: 33), CDR2 (SEQ ID No: 34), and CDR3 (SEQ ID No: 35);

(VH-6b) the amino acid sequence of SEQ ID No: 51 or an amino acid sequence comprising one or several amino acid substitutions (e.g., conservative substitutions), insertions, or deletions, in regions of the amino acid sequence of SEQ ID No: 51 other than CDR1 (SEQ ID No: 33), CDR2 (SEQ ID No: 34), and CDR3 (SEQ ID No: 35);

(VH-6c) the amino acid sequence of SEQ ID No: 59 or an amino acid sequence comprising one or several amino acid substitutions (e.g., conservative substitutions), insertions, or deletions, in regions of the amino acid sequence of SEQ ID No: 59 other than CDR1 (SEQ ID No: 33), CDR2 (SEQ ID No: 34), and CDR3 (SEQ ID No: 35);

(VH-7) the amino acid sequence of SEQ ID No: 75 or an amino acid sequence comprising one or several amino acid substitutions (e.g., conservative substitutions), insertions, or deletions, in regions of the amino acid sequence of SEQ ID No: 75 other than CDR1 (SEQ ID No: 9), CDR2 (SEQ ID No: 74), and CDR3 (SEQ ID No: 11);

(VH-8) the amino acid sequence of SEQ ID No: 77 or an amino acid sequence comprising one or several amino acid substitutions (e.g., conservative substitutions), insertions, or deletions, in regions of the amino acid sequence of SEQ ID No: 77 other than CDR1 (SEQ ID No: 9), CDR2 (SEQ ID No: 76), and CDR3 (SEQ ID No: 11);

(VH-9) the amino acid sequence of SEQ ID No: 79 or an amino acid sequence comprising one or several amino acid substitutions (e.g., conservative substitutions), insertions, or deletions, in regions of the amino acid sequence of SEQ ID No: 79 other than CDR1 (SEQ ID No: 17), CDR2 (SEQ ID No: 78), and CDR3 (SEQ ID No: 19);

(VH-10) the amino acid sequence of SEQ ID No: 81 or an amino acid sequence comprising one or several amino acid substitutions (e.g., conservative substitutions), insertions, or deletions, in regions of the amino acid sequence of SEQ ID No: 81 other than CDR1 (SEQ ID No: 17), CDR2 (SEQ ID No: 80), and CDR3 (SEQ ID No: 19);

(VH-11) the amino acid sequence of SEQ ID No: 83 or an amino acid sequence comprising one or several amino acid substitutions (e.g., conservative substitutions), insertions, or deletions, in regions of the amino acid sequence of SEQ ID No: 83 other than CDR1 (SEQ ID No: 25), CDR2 (SEQ ID No: 82), and CDR3 (SEQ ID No: 27);

(VH-12) the amino acid sequence of SEQ ID No: 85 or an amino acid sequence comprising one or several amino acid substitutions (e.g., conservative substitutions), insertions, or deletions, in regions of the amino acid sequence of SEQ ID No: 85 other than CDR1 (SEQ ID No: 25), CDR2 (SEQ ID No: 84), and CDR3 (SEQ ID No: 27);

wherein the numbers (VH-1) to (VH-12) correspond to the numbers (1) to (12) of the aforementioned combinations of the heavy chain CDR1 to CDR3 sequences. For example, the amino acid sequence of (VH-1) means the sequence including the amino acid sequences of heavy chain CDR1 to CDR3 identified in (1) SEQ ID No: 1 to SEQ ID No: 3 of SEQ ID No: 7. The branch numbers (e.g., (VH-1a) and (VH-1b)) for the respective heavy chain variable region VH domains mean that these sequences correspond to a variation of the heavy chain variable region having the CDR combination of the common sequence of (VH-1), and having a framework sequence consisting of various sequences.

In this case, the amino acid sequence of the framework of the heavy chain variable region can be that of existing antibodies, for example, derived from the amino acid sequence of the variable region of any human antibody or an amino acid sequence modified from such an amino acid sequence. Specifically, since, among the amino acid sequences of the heavy chain variable region, each of CDR1 to CDR3 is known to be essential for binding to the target antigen, while an amino acid sequence comprising one or several amino acid substitutions (e.g., conservative substitutions), insertions, or deletions in regions other than CDR1 to CDR3 can be used, an amino acid sequence comprising one or several amino acid substitutions (e.g., conservative substitutions, insertions, or deletions) in regions other than CDR1 to CDR3 can also be used in the present invention as long as they can constitute an antibody or an antibody derivative thereof that have binding activity against Tim-3 and enhance the immune activity against cancer cells.

In this embodiment of the present invention, the antibody etc. of the present invention can be identified as having an amino acid sequence selected from the group consisting of the following (VL-1)-(VL-6) (and the branch numbers of each number) of the light chain variable region VL domains:

(VL-1a) the amino acid sequence of SEQ ID No: 8 or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6),
(VL-1b) the amino acid sequence of SEQ ID No: 58 or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6),
(VL-1c) the amino acid sequence of SEQ ID No: 50 or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6),
(VL-2a) the amino acid sequence of SEQ ID No: 16 or an amino acid sequence comprising one or several amino acid substitutions (e.g., conservative substitutions), insertions, or deletions in regions of the amino acid sequence of SEQ ID No: 16 other than CDR1 (SEQ ID No: 12), CDR2 (SEQ ID No: 13), and CDR3 (SEQ ID No: 14),
(VL-2b) the amino acid sequence of SEQ ID No: 63 or an amino acid sequence comprising one or several amino acid substitutions (e.g., conservative substitutions), insertions, or deletions in regions of the amino acid sequence of SEQ ID No: 63 other than CDR1 (SEQ ID No: 12), CDR2 (SEQ ID No: 13), and CDR3 (SEQ ID No: 14),
(VL-2c) the amino acid sequence of SEQ ID No: 64 or an amino acid sequence comprising one or several amino acid substitutions (e.g., conservative substitutions), insertions, or deletions in regions of the amino acid sequence of SEQ ID No: 64 other than CDR1 (SEQ ID No: 12), CDR2 (SEQ ID No: 13), and CDR3 (SEQ ID No: 14),
(VL-2d) the amino acid sequence of SEQ ID No: 65 or an amino acid sequence comprising one or several amino acid substitutions (e.g., conservative substitutions), insertions, or deletions in regions of the amino acid sequence of SEQ ID No: 65 other than CDR1 (SEQ ID No: 12), CDR2 (SEQ ID No: 13), and CDR3 (SEQ ID No: 14),
(VL-3a) the amino acid sequence of SEQ ID No: 24 or an amino acid sequence comprising one or several amino acid substitutions (e.g., conservative substitutions), insertions, or deletions in regions of the amino acid sequence of SEQ ID No: 24 other than CDR1 (SEQ ID No: 20), CDR2 (SEQ ID No: 21), and CDR3 (SEQ ID No: 22),
(VL-3b) the amino acid sequence of SEQ ID No: 67 or an amino acid sequence comprising one or several amino acid substitutions (e.g., conservative substitutions), insertions, or deletions in regions of the amino acid sequence of SEQ ID No: 67 other than CDR1 (SEQ ID No: 20), CDR2 (SEQ ID No: 21), and CDR3 (SEQ ID No: 22),
(VL-3c) the amino acid sequence of SEQ ID No: 68 or an amino acid sequence comprising one or several amino acid substitutions (e.g., conservative substitutions), insertions, or deletions in regions of the amino acid sequence of SEQ ID No: 68 other than CDR1 (SEQ ID No: 20), CDR2 (SEQ ID No: 21), and CDR3 (SEQ ID No: 22),
(VL-3d) the amino acid sequence of SEQ ID No: 69 or an amino acid sequence comprising one or several amino acid substitutions (e.g., conservative substitutions), insertions, or deletions in regions of the amino acid sequence of SEQ ID No: 69 other than CDR1 (SEQ ID No: 20), CDR2 (SEQ ID No: 21), and CDR3 (SEQ ID No: 22),
(VL-4a) the amino acid sequence of SEQ ID No: 32 or an amino acid sequence comprising one or several amino acid substitutions (e.g., conservative substitutions), insertions, or deletions in regions of the amino acid sequence of SEQ ID No: 32 other than CDR1 (SEQ ID No: 28), CDR2 (SEQ ID No: 29), and CDR3 (SEQ ID No: 30),
(VL-4b) the amino acid sequence of SEQ ID No: 71 or an amino acid sequence comprising one or several amino acid substitutions (e.g., conservative substitutions), insertions, or deletions in regions of the amino acid sequence of SEQ ID No: 71 other than CDR1 (SEQ ID No: 28), CDR2 (SEQ ID No: 29), and CDR3 (SEQ ID No: 30),
(VL-4c) the amino acid sequence of SEQ ID No: 72 or an amino acid sequence comprising one or several amino acid substitutions (e.g., conservative substitutions), insertions, or deletions in regions of the amino acid sequence of SEQ ID No: 72 other than CDR1 (SEQ ID No: 28), CDR2 (SEQ ID No: 29), and CDR3 (SEQ ID No: 30),
(VL-4d) the amino acid sequence of SEQ ID No: 73 or an amino acid sequence comprising one or several amino acid substitutions (e.g., conservative substitutions), insertions, or deletions in regions of the amino acid sequence of SEQ ID No: 73 other than CDR1 (SEQ ID No: 28), CDR2 (SEQ ID No: 29), and CDR3 (SEQ ID No: 30),

(VL-5a) the amino acid sequence of SEQ ID No: 8 or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6),

(VL-5b) the amino acid sequence of SEQ ID No: 58 or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6),

(VL-5c) the amino acid sequence of SEQ ID No: 50 or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6),

(VL-6a) the amino acid sequence of SEQ ID No: 56 or an amino acid sequence comprising one or several amino acid substitutions (e.g., conservative substitutions), insertions, or deletions in regions of the amino acid sequence of SEQ ID No: 56 other than CDR1 (SEQ ID No: 52), CDR2 (SEQ ID No: 53), and CDR3 (SEQ ID No: 54),

(VL-6b) the amino acid sequence of SEQ ID No: 60 or an amino acid sequence comprising one or several amino acid substitutions (e.g., conservative substitutions), insertions, or deletions in regions of the amino acid sequence of SEQ ID No: 60 other than CDR1 (SEQ ID No: 52), CDR2 (SEQ ID No: 53), and CDR3 (SEQ ID No: 54),

(VL-6c) the amino acid sequence of SEQ ID No: 61 or an amino acid sequence comprising one or several amino acid substitutions (e.g., conservative substitutions), insertions, or deletions in regions of the amino acid sequence of SEQ ID No: 61 other than CDR1 (SEQ ID No: 52), CDR2 (SEQ ID No: 53), and CDR3 (SEQ ID No: 54);

wherein the numbers (VL-1) to (VL-6) correspond to the numbers (1) to (6) of the aforementioned combinations of the light chain CDR1 to CDR3 sequences. For example, the amino acid sequence of (VL-1) means the sequence including the amino acid sequences of light chain CDR1 to CDR3 identified in (1) SEQ ID No: 4 to SEQ ID No: 6 of SEQ ID No: 8. The branch numbers (e.g., (VL-1a) and (VL-1b)) for the respective light chain variable region VL domains mean that these sequences correspond to a variation of the light chain variable region having the CDR combination of the common sequence (VL-1), and having a framework sequence consisting of various sequences.

In addition,

- the light chain variable region defined by the combination of the sequences of the light chain CDR1 to CDR3 in (7) and (8) described above is identical to the light chain variable region defined by the combination of the sequences of the light chain CDR1 to CDR3 in (2) described above (i.e., the light chain variable region defined by (VL-2) or its branch number),
- the light chain variable region defined by the combination of the sequences of the light chain CDR1 to CDR3 in (9) and (10) described above is identical to the light chain variable region defined by the combination of the sequences of the light chain CDR1 to CDR3 in (3) described above (i.e., the light chain variable region defined by (VL-3) or its branch number),
- the light chain variable region defined by the combination of the sequences of the light chain CDR1 to CDR3 in (11) and (12) described above is identical to the light chain variable region defined by the combination of the sequences of the light chain CDR1 to CDR3 in (4) described above (i.e., the light chain variable region defined by (VL-4) or its branch number).

In this case, the amino acid sequence of the framework of the light chain variable region can be that of an existing antibody, for example, derived from the amino acid sequence of the variable region of any human antibody or an amino acid sequence modified from such an amino acid sequence. Specifically, since, among the amino acid sequences of the light chain variable region, each of CDR1 to CDR3 is known to be essential for binding to the target antigen, while an amino acid sequence comprising one or several amino acid substitutions (e.g., conservative substitutions), insertions, or deletions in regions other than CDR1 to CDR3 can be used, an amino acid sequence comprising one or several amino acid substitutions (e.g., conservative substitutions, insertions, or deletions) in regions other than CDR1 to CDR3 can also be used in the present invention, as long as they can constitute an antibody etc. that have binding activity against Tim-3 and enhance the immune activity against cancer cells.

In the present invention, the antibody derivatives of the present invention also include functional fragments of the above described antibody etc. and modified antibodies thereof. Functional fragments of the antibody etc. in the present invention include F(ab')2, Fab', Fab, single chain Fv (scFv), and so on. Modified antibodies include Fc effector function modified antibody, IgG1LALA, IgG1_N297A, IgG4_S228P, and do on. Any types of these fragments or modified antibodies can be used as functional fragments or modified antibodies in the present invention, as long as the functional fragments or modified antibodies are characterized by their ability to bind to the Tim-3 antigen and to inhibit binding between Tim-3-Galectin-9, resulting in acting as so-called immune checkpoint inhibitors which can remove immunosuppressive signals based on the Tim-3-Galectin-9 pathway, or in inducing cytotoxicity against cancer cells as a result. For example, single-chain Fv (scFv) can be used as such functional fragments.

For example, when a single-chain Fv (scFv) antibody is used as an antibody etc. of this invention, the half-molecule of the antibody etc. of this invention can be identified as a single chain by linking the amino acid sequence of the heavy chain variable region VH domain described below with the amino acid sequence of the light chain variable region VL domain described below:

(1) an amino acid sequence in which the heavy chain variable region of (VH-1a), (VH-1b) or (VH-1c) and the light chain variable region of (VL-1a), (VL-1b) or (VL-1c) are connected via a linker,
(2) an amino acid sequence in which the heavy chain variable region of (VH-2a) or (VH-2b) and the light chain variable region of (VL-2a), (VL-2b), (VL-2c) or (VL-2d) are connected via a linker,
(3) an amino acid sequence in which the heavy chain variable region of (VH-3a) or (VH-3b) and the light chain variable region of (VL-3a), (VL-3b), (VL-3c) or (VL-3d) are connected via a linker,
(4) an amino acid sequence in which the heavy chain variable region of (VH-4a) or (VH-4b) and the light chain variable region of (VL-4a), (VL-4b), (VL-4c) or (VL-4d) are connected via a linker, and
(5) an amino acid sequence in which the heavy chain variable region of (VH-5a), (VH-5b) or (VH-5c) and the light chain variable region of (VL-5a), (VL-5b) or (VL-5c) are connected via a linker,
(6) an amino acid sequence in which the heavy chain variable region of (VH-6a), (VH-6b) or (VH-6c) and the light chain variable region of (VL-6a), (VL-6b) or (VL-6c) are connected via a linker,
(7) an amino acid sequence in which the heavy chain variable region of (VH-7) and the light chain variable region of (VL-2a), (VL-2b), (VL-2c) or (VL-2d) are connected via a linker,
(8) an amino acid sequence in which the heavy chain variable region of (VH-8) and the light chain variable region of (VL-2a), (VL-2b), (VL-2c) or (VL-2d) are connected via a linker,
(9) an amino acid sequence in which the heavy chain variable region of (VH-9) and the light chain variable region of (VL-3a), (VL-3b), (VL-3c) or (VL-3d) are connected via a linker,
(10) an amino acid sequence in which the heavy chain variable region of (VH-10) and the light chain variable region of (VL-3a), (VL-3b), (VL-3c) or (VL-3d) are connected via a linker,
(11) an amino acid sequence in which the heavy chain variable region of (VH-11) and the light chain variable region of (VL-4a), (VL-4b), (VL-4c) or (VL-4d) are connected via a linker,
(12) an amino acid sequence in which the heavy chain variable region of (VH-12) and the light chain variable region of (VL-4a), (VL-4b), (VL-4c) or (VL-4d) are connected via a linker.

wherein, the numbers (1) to (12) correspond to the numbers of the aforementioned combinations of the heavy chain CDR1-CDR3 sequences and the numbers of the aforementioned combinations of the light chain CDR1-CDR3 sequences. For example, the amino acid sequence (1) of a single-chain Fv antibody means a sequence that comprises (1) the amino acid sequences of the heavy-chain CDR1 to CDR3 identified by SEQ ID No: 1 to SEQ ID No: 3 and the light-chain CDR1 to CDR3 identified by SEQ ID No: 4 to SEQ ID No: 6.

The heavy chain variable region and the light chain variable region in scFv may be linked so that either one is on the N-terminal side, and, that is to say, the order of these regions may be, from N-terminal side to C-terminal side, heavy chain variable region-linker-light chain variable region or light chain variable region-linker-heavy chain variable region. Furthermore, the linker connecting between the heavy chain variable region and the light chain variable region may be any linker known in the art for use in the field. For example, in the examples described later in this application, GGGGS×4 (i.e., a linker composed of four consecutive GGGGS amino acid sequences) can be used.

The scFv antibodies exemplified here can be used as a form of a single chain, but a dimeric structure may be prepared by using two scFv-Fcs each of which is produced by binding the Fc region of the antibody commonly used in the field of the art, to the scFv via a linker. In this case, for example, the Fc region can be attached to the C-terminus of the scFv via a linker (e.g., a linker consisting of GGGG).

Structure of heterodimeric antibody and antibody derivative (e.g., antibody etc.)

In another embodiment, the present invention provides a heterodimeric antibody or antibody derivative, which is a combination of two different half-molecules that have binding ability to the Tim-3 antigen. This heterodimeric antibody etc. is characterized in that it enhances immune activity against cancer cells.

The heterodimeric antibody etc. in the second embodiment of the invention may bind to the Tim-3 antigen to suppress the function of the Tim-3 antigen, to remove immunosuppressive signals against cancer cells, to suppress the function of the Tim-3 antigen expressed on immune cells (especially T cells), to activate immune cells, and to exert a growth suppression effect on the tumors and This may be done in a manner that exerts a therapeutic effect on cancers.

When simply referring to "antibody" in this invention, the antibody may be derived from any species of mammals, and the species from which the antibody is derived are not limited to humans, but may be derived from mouse, rat, guinea pig, hamster, rabbit, VHH antibody (variable domain of heavy chain of heavy chain of heavy chain antibodies) derived from camelid (such as camel, and alpaca), ostrich antibodies, and so on. For example, in the case of the human antibody, this includes various isotypes such as IgG, IgM, IgA, IgD, IgE, etc.

In the second embodiment of the present invention, it may also be an "antibody derivative" of the above antibody, as long as it has the functional characteristics of binding to the Tim-3 antigen and having the activity of removing immunosuppressive signal against cancer cells. When referring to the antibody derivative in the present invention, for example, the present invention may provide an antibody derivative which is a combination of the amino acid sequence of the six CDRs (complementarity determining regions CDR1-CDR3 of the heavy chain and CDR1-CDR3 of the light chain) for any of IgG, IgM, IgA, IgD, IgE, etc. of the above antibodies, the amino acid sequence of the human antibody-derived constant regions, and other amino acid sequence comprising a combination of the amino acid sequence from the original antibody and that from the human antibody. Examples of the antibody derivative includes, but not limited to, a humanized antibody in which the amino acid sequences other than the complementarity determining regions (CDRs) of the above described "antibody" are replaced with those derived from a human antibody, or a chimeric antibody such as those in which the amino acid sequences of the variable region of the above antibody are linked to the constant regions of a human antibody, or a single chain antibody (e.g., scFv) in which the heavy chain and light chain are linked by a linker.

When "half-molecule" of an antibody etc. of is used in the present invention, it means a single molecule or a fragment thereof when the bond between the heavy chains in the antibody etc. is dissociated. In one example, a half-molecule of an antibody etc., is a combination of the heavy chain (H chain) and light chain (L chain) of an antibody derived from various isotypes such as IgG, IgM, IgA, IgD, and IgE. For example, among these, in the case where an antibody etc. is an IgG antibody, a half-molecule is, for example, a complex consisting of one H chain of IgG and one L chain of IgG. The half-molecule of the antibody etc. may also be derived from an antibody derivative, such as a chimeric antibody or a humanized antibody, as described above.

Furthermore, the half-molecules of the antibody etc. may include any types of the half-molecules of an antibody etc. other than those mentioned above, such as a molecule consisting of a single H chain which is prepared by dissociating a bond between two H chains of so-called a heavy-chain (H-chain) antibody (an antibody consisting of two H chains found in camelids, etc., also called as a VHH (VH originating from heavy chain antibody) (these are collectively called a "single domain antibody"), a single chain Fv molecule (scFv molecule) that can bind to another scFv molecule, a molecule in which an additional Fc region is added to a single-chain Fv molecule (scFv-Fc molecule), a molecule with a Knob structure for applying Knob-into-hole technology to the Fc domain of the scFv-Fc molecule, a molecule with a Hole structure for applying Knob-into-hole technology to the Fc domain of the scFv-Fc molecule and the like.

In this embodiment, the heterodimeric antibody etc. is characterized by being composed of half-molecules from two different antibodies, and the first epitope which is bound by the half-molecule of the first antibody etc. and the second epitope which is bound by the half-molecule of the second antibody etc. may be the same or be different from each other. By nature, one or both half-molecules of the antibody etc. that exhibits binding properties to Tim-3 antigen can bind to Tim-3. In the case where the one half-molecule of the antibody etc. binds to Tim-3 and the other half-molecule binds to a substance other than Tim-3, the antibody etc. in this embodiment is a bispecific antibody type. In the case where both half-molecules of the antibody etc. bind to different epitopes on Tim-3, the antibody etc. in this embodiment is a biparatopic antibody type. In the second embodiment of the present invention, it is preferred that the antibody etc. are a biparatopic antibody, whose both half-molecules bind to different epitope regions on Tim-3.

Thereby, among the antibody etc. of the present invention, a heterodimeric antibody etc. may be an antibody etc. composed of a combination of a half-molecule of the first antibody etc. having a binding ability to the Tim-3 antigen and a half-molecule of the second antibody etc. having a binding ability to an antigen other than the Tim-3 antigen (i.e., a bispecific antibody). In this case, as the structure of the antibody etc., it is preferred that the half-molecule of the first antibody etc. is the half-molecule of the antibody etc. identified by a total of six positions having the specific sequence disclosed herein of the amino acid sequences which are CDR1-3 of the heavy chain and CDR1-3 of the light chain. It is possible to form an antibody etc. that can simultaneously achieve binding to the Tim-3 antigen by the half-molecule of the first antibody etc. and binding to an antigen other than the Tim-3 antigen by the half-molecule of the second antibody etc.. In this embodiment, as long as the half-molecule of the first antibody etc. binds to the Tim-3 antigen, a half-molecule of the second antibody etc. can bind to any antigen.

In the present invention, when the antibody etc. having binding properties to the Tim-3 antigen is a biparatopic antibody, in order to achieve stronger binding to the Tim-3 antigen, it is preferable that the half-molecule of the first antibody etc. and the half-molecule of the second antibody etc. are both half-molecules of the antibody etc. which bind to the regions of the Tim-3 antigen. The first region (epitope) to which the half-molecule of the first antibody etc. binds and the second region (epitope) to which the half-molecule of the second antibody etc. binds may be the same region or the different regions on the Tim-3 protein.

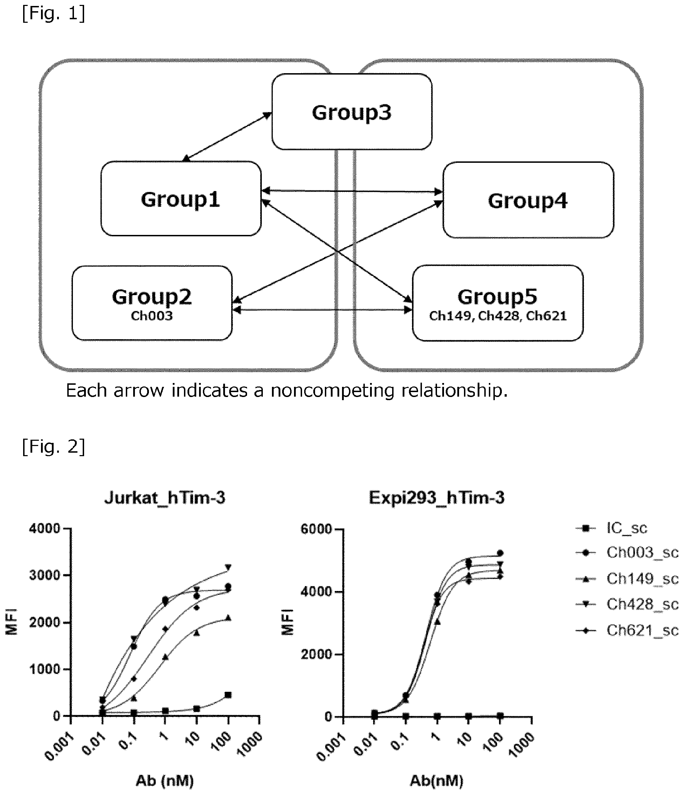

As a result of analyzing the competitive relationship between the antibody etc. and Tim-3 protein, the antibody etc. that binds to the epitope site of Tim-3 antigen can be divided into 5 groups, i.e., Group 1 to Group 5 (see, Figure 1). These antibodies are classified into two groups based on the functionality: those that inhibit PtdSer (the antibodies belonging to Group 1 and Group 2) and those that do not inhibit or partially inhibit PtdSer (the antibodies belonging to Group 3, Group 4, and Group 5). When these antibodies are classified in terms of the competitive relationship for binding to Tim-3, antibodies belonging to Group 1 and Group 2 do not compete with antibodies belonging to Group 4 and Group 5, while antibodies belonging to Group 1 compete with antibodies belonging to Group 2, and antibodies belonging to Group 3 do not compete with antibodies belonging to Group 1, but compete with antibodies belonging to Group 2, Group 4, and Group 5. Based on the results of this analysis, when one biparatopic antibody molecule in this embodiment of the present invention is to bind to two sites on one molecule of Tim-3 protein, it is preferable that the first region (epitope) to which the first half-molecule of the antibody etc. binds is different from the second region (epitope) to which the second half-molecule of the antibody etc. binds, and, for example, that the one half-molecule of the antibody etc. belongs to Group 1 or Group 2 and the other half-molecule of the antibody etc. belongs to Group 3, Group 4 and Group 5.

As for the structure of the antibody etc. in this embodiment of the invention, an antibody etc. where both half-molecule of the first antibody etc. and half-molecule of the second antibody etc. bind to the Tim-3 antigen (i.e., biparatopic antibody (BpAb)) may be produced by combining the half-molecule of the above first antibody etc. that binds to the Tim-3 protein having a total of six amino acid sequences of CDRs 1-3 of a specific heavy chain and CDRs 1-3 of a specific light chain, and a half-molecule of the above second antibody etc. that binds to the Tim-3 protein having a total of six amino acid sequences of CDRs 1-3 of a specific heavy chain and CDRs 1-3 of a specific light chain.

Examples of amino acid sequences of six specific CDRs of a half-molecule of such an antibody etc. with binding ability to the Tim-3 antigen may include those containing complementarity-determining regions of the heavy and light chains selected from the group consisting of (1) to (12) below:

(1) Complementarity-determining regions of the heavy chain, CDR1 (SYYMS, SEQ ID No: 1), CDR2 (TISNSGGSIYYLDSVKD, SEQ ID No: 2), and CDR3 (DPYYSNYVPMDY, SEQ ID No: 3), and complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);

(2) Complementarity-determining regions of heavy chains, CDR1 (DYYMD, SEQ ID No: 9), CDR2 (YIYPNNGGTSYNQKFKG, SEQ ID No: 10), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(3) Complementarity-determining regions of the heavy chains, CDR1 (DYYMD, SEQ ID No: 17), CDR2 (YIYPNNGGTSYNQKFKG, SEQ ID No: 18), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(4) Complementarity-determining regions of the heavy chains, CDR1 (DYYMD, SEQ ID No: 25), CDR2 (YIYPNNGGTSYNQKFKG, SEQ ID No: 26), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(5) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);

(6) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASENVGTYVS, SEQ ID No: 52), CDR2 (GASNRYT, SEQ ID No: 53), and CDR3 (GQSYSYPLT, SEQ ID No: 54);

(7) Complementarity-determining regions of heavy chains, CDR1 (DYYMD, SEQ ID No: 9), CDR2 (YIYPNNAGTS YNQKFKG, SEQ ID No: 74), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(8) Complementarity-determining regions of the heavy chain, CDR1 (DYYMD, SEQ ID No: 9), CDR2 (YIYPNKGGTS YNQKFKG, SEQ ID No: 76), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(9) Complementarity-determining regions of the heavy chain, CDR1 (DYYMD, SEQ ID No: 17), CDR2 (YIYPNNAGTS YNQKFKG, SEQ ID No: 78), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(10) Complementarity-determining regions of heavy chains, CDR1 (DYYMD, SEQ ID No: 17), CDR2 (YIYPNKGGTS YNQKFKG, SEQ ID No: 80), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(11) Complementarity-determining regions of heavy chains, CDR1 (DYYMD, SEQ ID No: 25), CDR2 (YIYPNNAGTS YNQKFKG, SEQ ID No: 82), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(12) Complementarity-determining regions of heavy chains, CDR1 (DYYMD, SEQ ID No: 25), CDR2 (YIYPNKGGTS YNQKFKG, SEQ ID No: 84), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

and an antibody etc. identified with CDR combinations other than those listed above may be also included, as long as it exhibits characterization of a binding ability to the Tim-3 antigen.

Specific antibody etc. of the second embodiment

In the second embodiment of the present invention, since, as mentioned above, the first epitope to which the first half-molecule of an antibody etc. binds and the second epitope to which the second half-molecule of an antibody etc. binds may be the same or different sites on the Tim-3 protein, the half-molecules of the antibody etc. exemplified above (1) through (5) may be used in any combination. In other words, the antibody etc. that is a biparatopic antibody in the present invention may be an antibody etc. composed of any combination of two different half-molecules of an antibody etc.

As mentioned above, among these half-molecules, the half-molecules of an antibody etc. such as (1), (5) and (6) are half-molecules derived from antibodies belonging to Group 2, and the half-molecules of an antibody etc. such as (2) through (4) and (7) through (12) are half-molecules derived from antibodies belonging to Group 5. Thus, for example, to make the biparatopic antibody in the second embodiment of the invention which binds to two different epitope sites on the Tim-3 protein, the present invention may provide an antibody etc. in which the one half-molecule of the antibody etc. is the half-molecule of (1), (5) or (6) exemplified above, and the other half-molecule of the antibody etc. is the half-molecule of (2) through (4) or (7) through (12).

In this embodiment of the present invention, the half-molecule of the antibody etc. can be specified, for example, as having an amino acid sequence selected from the group consisting of the following (VH-1) to (VH-12) (and the branch numbers of each number) of the heavy chain variable region VH domains:

(VH-1a) the amino acid sequence of SEQ ID No: 7, or an amino acid sequence comprising one or several amino acid substitutions (e.g., conservative substitutions), insertions, or deletions in regions of the amino acid sequence of SEQ ID No: 7 other than CDR1 (SEQ ID No: 1), CDR2 (SEQ ID No: 2), and CDR3 (SEQ ID No: 3),

(VH-1b) the amino acid sequence of SEQ ID No: 49, or an amino acid sequence comprising one or several amino acid substitutions (e.g., conservative substitutions), insertions, or deletions in regions of the amino acid sequence of SEQ ID No: 49 other than CDR1 (SEQ ID No: 1), CDR2 (SEQ ID No: 2), and CDR3 (SEQ ID No: 3),

(VH-1c) the amino acid sequence of SEQ ID No: 57, or an amino acid sequence comprising one or several amino acid substitutions (e.g., conservative substitutions), insertions, or deletions in regions of the amino acid sequence of SEQ ID No: 57 other than CDR1 (SEQ ID No: 1), CDR2 (SEQ ID No: 2), and CDR3 (SEQ ID No: 3),

(VH-2a) the amino acid sequence of SEQ ID No: 15, or an amino acid sequence comprising one or several amino acid substitutions (e.g., conservative substitutions), insertions, or deletions in regions of the amino acid sequence of SEQ ID No: 15 other than CDR1 (SEQ ID No: 9), CDR2 (SEQ ID No: 10), and CDR3 (SEQ ID No: 11),

(VH-2b) the amino acid sequence of SEQ ID No: 62, or an amino acid sequence comprising one or several amino acid substitutions (e.g., conservative substitutions), insertions, or deletions in regions of the amino acid sequence of SEQ ID No: 62 other than CDR1 (SEQ ID No: 9), CDR2 (SEQ ID No: 10), and CDR3 (SEQ ID No: 11),

(VH-3a) the amino acid sequence of SEQ ID No: 23, or an amino acid sequence comprising one or several amino acid substitutions (e.g., conservative substitutions), insertions, or deletions in regions of the amino acid sequence of SEQ ID No: 23 other than CDR1 (SEQ ID No: 17), CDR2 (SEQ ID No: 18), and CDR3 (SEQ ID No: 19),

(VH-3b) the amino acid sequence of SEQ ID No: 66, or an amino acid sequence comprising one or several amino acid substitutions (e.g., conservative substitutions), insertions, or deletions in regions of the amino acid sequence of SEQ ID No: 66 other than CDR1 (SEQ ID No: 17), CDR2 (SEQ ID No: 18), and CDR3 (SEQ ID No: 19),

(VH-4a) the amino acid sequence of SEQ ID No: 31, or an amino acid sequence comprising one or several amino acid substitutions (e.g., conservative substitutions), insertions, or deletions in regions of the amino acid sequence of SEQ ID No: 31 other than CDR1 (SEQ ID No: 25), CDR2 (SEQ ID No: 26), and CDR3 (SEQ ID No: 27),

(VH-4b) the amino acid sequence of SEQ ID No: 70, or an amino acid sequence comprising one or several amino acid substitutions (e.g., conservative substitutions), insertions, or deletions in regions of the amino acid sequence of SEQ ID No: 70 other than CDR1 (SEQ ID No: 25), CDR2 (SEQ ID No: 26), and CDR3 (SEQ ID No: 27),

(VH-5a) the amino acid sequence of SEQ ID No: 36, or an amino acid sequence comprising one or several amino acid substitutions (e.g., conservative substitutions), insertions, or deletions in regions of the amino acid sequence of SEQ ID No: 36 other than CDR1 (SEQ ID No: 33), CDR2 (SEQ ID No: 34), and CDR3 (SEQ ID No: 35),

(VH-5b) the amino acid sequence of SEQ ID No: 51, or an amino acid sequence comprising one or several amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35),

(VH-5c) the amino acid sequence of SEQ ID No: 59, or an amino acid sequence comprising one or several amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35),

(VH-6a) the amino acid sequence of SEQ ID No: 55, or an amino acid sequence comprising one or several amino acid substitutions (e.g., conservative substitutions), insertions, or deletions in regions of the amino acid sequence of SEQ ID No: 55 other than CDR1 (SEQ ID No: 33), CDR2 (SEQ ID No: 34), and CDR3 (SEQ ID No: 35,

(VH-6b) the amino acid sequence of SEQ ID No: 51, or an amino acid sequence comprising one or several amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35,

(VH-6c) the amino acid sequence of SEQ ID No: 59, or an amino acid sequence comprising one or several amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35,

(VH-7) the amino acid sequence of SEQ ID No: 75, or an amino acid sequence comprising one or several amino acid substitutions (e.g., conservative substitutions), insertions, or deletions in regions of the amino acid sequence of SEQ ID No: 75 other than CDR1 (SEQ ID No: 9), CDR2 (SEQ ID No: 74), and CDR3 (SEQ ID No: 11,

(VH-8) the amino acid sequence of SEQ ID No: 77, or an amino acid sequence comprising one or several amino acid substitutions (e.g., conservative substitutions), insertions, or deletions in regions of the amino acid sequence of SEQ ID No: 77 other than CDR1 (SEQ ID No: 9), CDR2 (SEQ ID No: 76), and CDR3 (SEQ ID No: 11,

(VH-9) the amino acid sequence of SEQ ID No: 79, or an amino acid sequence comprising one or several amino acid substitutions (e.g., conservative substitutions), insertions, or deletions in regions of the amino acid sequence of SEQ ID No: 79 other than CDR1 (SEQ ID No: 17), CDR2 (SEQ ID No: 78), and CDR3 (SEQ ID No: 19),

(VH-10) the amino acid sequence of SEQ ID No: 81 , or an amino acid sequence comprising one or several amino acid substitutions (e.g., conservative substitutions), insertions, or deletions in regions of the amino acid sequence of SEQ ID No: 81 other than CDR1 (SEQ ID No: 17), CDR2 (SEQ ID No: 80), and CDR3 (SEQ ID No: 19),

(VH-11) the amino acid sequence of SEQ ID No: 83 , or an amino acid sequence comprising one or several amino acid substitutions (e.g., conservative substitutions), insertions, or deletions in regions of the amino acid sequence of SEQ ID No: 83 other than CDR1 (SEQ ID No: 25), CDR2 (SEQ ID No: 82), and CDR3 (SEQ ID No: 27),

(VH-12) the amino acid sequence of SEQ ID No: 85 , or an amino acid sequence comprising one or several amino acid substitutions (e.g., conservative substitutions), insertions, or deletions in regions of the amino acid sequence of SEQ ID No: 85 other than CDR1 (SEQ ID No: 25), CDR2 (SEQ ID No: 84), and CDR3 (SEQ ID No: 27),

wherein, the numbers (VH-1) to (VH-12) correspond to the numbers (1) to (12) of the aforementioned combinations of the heavy chain CDR1 to CDR3 sequences. For example, the amino acid sequence of (VH-1) means the sequence including the amino acid sequences of heavy chain CDR1 to CDR3 identified in (1) SEQ ID No: 1 to SEQ ID No: 3 of SEQ ID No: 7. The branch numbers (e.g., (VH-1a) and (VH-1b)) for the respective heavy chain variable region VH domains mean that these sequences correspond to a variation of the heavy chain variable region having the CDR combination of the common sequence of (VH-1), and having a framework sequence consisting of various sequences.

In this case, the amino acid sequence of the framework of the heavy chain variable region can be that of an existing antibody, for example, derived from the amino acid sequence of the variable region of any human antibody or an amino acid sequence modified from such an amino acid sequence. Specifically, since, among the amino acid sequences of the heavy chain variable region, each of CDR1 to CDR3 is known to be essential for binding to the target antigen, while an amino acid sequences comprising one or several amino acid substitutions (e.g., conservative substitutions), insertions, or deletions in regions other than CDR1 to CDR3 can be used, an amino acid sequence comprising one or several amino acid substitutions (e.g., conservative substitutions, insertions, or deletions) in regions other than CDR1 to CDR3 can also be used in the present invention as long as they can constitute an antibody or an antibody derivative thereof that have binding activity against Tim-3 and enhance the immune activity against cancer cells.

In this embodiment of the present invention, the half-molecules of antibody etc. of the present invention can be identified as having an amino acid sequence selected from the group consisting of the following (VL-1) through (VL-6) (and the branch numbers of each number) of the light chain variable region VL domains:

(VL-1a) the amino acid sequence of SEQ ID No: 8 or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6),

(VL-1b) the amino acid sequence of SEQ ID No: 58 or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6),

(VL-1c) the amino acid sequence of SEQ ID No: 50 or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6),

(VL-2a) the amino acid sequence of SEQ ID No: 16 or an amino acid sequence comprising one or several amino acid substitutions (e.g., conservative substitutions), insertions, or deletions in regions of the amino acid sequence of SEQ ID No: 16 other than CDR1 (SEQ ID No: 12), CDR2 (SEQ ID No: 13), and CDR3 (SEQ ID No: 14),

(VL-2b) the amino acid sequence of SEQ ID No: 63 or an amino acid sequence comprising one or several amino acid substitutions (e.g., conservative substitutions), insertions, or deletions in regions of the amino acid sequence of SEQ ID No: 63 other than CDR1 (SEQ ID No: 12), CDR2 (SEQ ID No: 13), and CDR3 (SEQ ID No: 14),

(VL-2c) the amino acid sequence of SEQ ID No: 64 or an amino acid sequence comprising one or several amino acid substitutions (e.g., conservative substitutions), insertions, or deletions in regions of the amino acid sequence of SEQ ID No: 64 other than CDR1 (SEQ ID No: 12), CDR2 (SEQ ID No: 13), and CDR3 (SEQ ID No: 14),

(VL-2d) the amino acid sequence of SEQ ID No: 65 or an amino acid sequence comprising one or several amino acid substitutions (e.g., conservative substitutions), insertions, or deletions in regions of the amino acid sequence of SEQ ID No: 65 other than CDR1 (SEQ ID No: 12), CDR2 (SEQ ID No: 13), and CDR3 (SEQ ID No: 14),

(VL-3a) the amino acid sequence of SEQ ID No: 24 or an amino acid sequence comprising one or several amino acid substitutions (e.g., conservative substitutions), insertions, or deletions in regions of the amino acid sequence of SEQ ID No: 24 other than CDR1 (SEQ ID No: 20), CDR2 (SEQ ID No: 21), and CDR3 (SEQ ID No: 22),

(VL-3b) the amino acid sequence of SEQ ID No: 67 or an amino acid sequence comprising one or several amino acid substitutions (e.g., conservative substitutions), insertions, or deletions in regions of the amino acid sequence of SEQ ID No: 67 other than CDR1 (SEQ ID No: 20), CDR2 (SEQ ID No: 21), and CDR3 (SEQ ID No: 22),

(VL-3c) the amino acid sequence of SEQ ID No: 68 or an amino acid sequence comprising one or several amino acid substitutions (e.g., conservative substitutions), insertions, or deletions in regions of the amino acid sequence of SEQ ID No: 68 other than CDR1 (SEQ ID No: 20), CDR2 (SEQ ID No: 21), and CDR3 (SEQ ID No: 22),

(VL-3d) the amino acid sequence of SEQ ID No: 69 or an amino acid sequence comprising one or several amino acid substitutions (e.g., conservative substitutions), insertions, or deletions in regions of the amino acid sequence of SEQ ID No: 69 other than CDR1 (SEQ ID No: 20), CDR2 (SEQ ID No: 21), and CDR3 (SEQ ID No: 22),

(VL-4a) the amino acid sequence of SEQ ID No: 32 or an amino acid sequence comprising one or several amino acid substitutions (e.g., conservative substitutions), insertions, or deletions in regions of the amino acid sequence of SEQ ID No: 32 other than CDR1 (SEQ ID No: 28), CDR2 (SEQ ID No: 29), and CDR3 (SEQ ID No: 30),

(VL-4b) the amino acid sequence of SEQ ID No: 71 or an amino acid sequence comprising one or several amino acid substitutions (e.g., conservative substitutions), insertions, or deletions in regions of the amino acid sequence of SEQ ID No: 71 other than CDR1 (SEQ ID No: 28), CDR2 (SEQ ID No: 29), and CDR3 (SEQ ID No: 30),

(VL-4c) the amino acid sequence of SEQ ID No: 72 or an amino acid sequence comprising one or several amino acid substitutions (e.g., conservative substitutions), insertions, or deletions in regions of the amino acid sequence of SEQ ID No: 72 other than CDR1 (SEQ ID No: 28), CDR2 (SEQ ID No: 29), and CDR3 (SEQ ID No: 30),

(VL-4d) the amino acid sequence of SEQ ID No: 73 or an amino acid sequence comprising one or several amino acid substitutions (e.g., conservative substitutions), insertions, or deletions in regions of the amino acid sequence of SEQ ID No: 73 other than CDR1 (SEQ ID No: 28), CDR2 (SEQ ID No: 29), and CDR3 (SEQ ID No: 30),

(VL-5a) the amino acid sequence of SEQ ID No: 8 or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6),

(VL-5b) the amino acid sequence of SEQ ID No: 58 or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6),

(VL-5c) the amino acid sequence of SEQ ID No: 50 or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6),

(VL-6a) the amino acid sequence of SEQ ID No: 56 or an amino acid sequence comprising one or several amino acid substitutions (e.g., conservative substitutions), insertions, or deletions in regions of the amino acid sequence of SEQ ID No: 56 other than CDR1 (SEQ ID No: 52), CDR2 (SEQ ID No: 53), and CDR3 (SEQ ID No: 54),

(VL-6b) the amino acid sequence of SEQ ID No: 60 or an amino acid sequence comprising one or several amino acid substitutions (e.g., conservative substitutions), insertions, or deletions in regions of the amino acid sequence of SEQ ID No: 60 other than CDR1 (SEQ ID No: 52), CDR2 (SEQ ID No: 53), and CDR3 (SEQ ID No: 54),
(VL-6c) the amino acid sequence of SEQ ID No: 61 or an amino acid sequence comprising one or several amino acid substitutions (e.g., conservative substitutions), insertions, or deletions in regions of the amino acid sequence of SEQ ID No: 61 other than CDR1 (SEQ ID No: 52), CDR2 (SEQ ID No: 53), and CDR3 (SEQ ID No: 54),
wherein, the numbers (VL-1) to (VL-6) correspond to the numbers (1) to (6) of the aforementioned combinations of the light chain CDR1 to CDR3 sequences. For example, the amino acid sequence of (VL-1) means the sequence including the amino acid sequences of light chain CDR1 to CDR3 identified in (1) SEQ ID No: 4 to SEQ ID No: 6 of SEQ ID No: 8. The branch numbers (e.g., (VL-1a) and (VL-1b)) for the respective light chain variable region VL domains mean that these sequences correspond to a variation of the light chain variable region having the CDR combination of the common sequence (VL-1), and having a framework sequence consisting of various sequences.

In addition,

- The light chain variable region defined by the combination of the sequences of the light chain CDR1 to CDR3 in (7) and (8) described above is identical to the light chain variable region defined by the combination of the sequences of the light chain CDR1 to CDR3 in (2) described above (i.e., the light chain variable region defined by (VL-2) or its branch number),
- The light chain variable region defined by the combination of the sequences of the light chain CDR1 to CDR3 in (9) and (10) described above is identical to the light chain variable region defined by the combination of the sequences of the light chain CDR1 to CDR3 in (3) described above (i.e., the light chain variable region defined by (VL-3) or its branch number),
- The light chain variable region defined by the combination of the sequences of the light chain CDR1 to CDR3 in (11) and (12) described above is identical to the light chain variable region defined by the combination of the sequences of the light chain CDR1 to CDR3 in (4) described above (i.e., the light chain variable region defined by (VL-4) or its branch number).

In this case, the amino acid sequence of the framework of the light chain variable region can be that of existing antibodies, for example, derived from the amino acid sequence of the variable region of any human antibody or an amino acid sequence modified from such an amino acid sequence. Specifically, since, among the amino acid sequences of the light chain variable region, each of CDR1 to CDR3 is known to be essential for binding to the target antigen, while an amino acid sequences comprising one or several amino acid substitutions (e.g., conservative substitutions), insertions, or deletions in regions other than CDR1 to CDR3 can be used, an amino acid sequences comprising one or several amino acid substitutions (e.g., conservative substitutions, insertions, or deletions) in regions other than CDR1 to CDR3 can also be used in the present invention, as long as they can constitute an antibody etc. that have binding activity against Tim-3 and enhance the immune activity against cancer cells.

In the present invention, the antibody derivatives of the present invention also include functional fragments of the above described antibody etc. and modified antibodies thereof. Functional fragments of the antibody etc. in the present invention include F(ab')2, Fab', Fab, single chain Fv (scFv), and so on. Modified antibodies include Fc effector function modified antibody, IgG1LALA, IgG1_N297A, IgG4_S228P, and so on. Any types of these fragments or modified antibodies can be used as functional fragments or modified antibodies in the present invention, as long as the functional fragments or modified antibodies are characterized by their ability to bind to the Tim-3 antigen, and to inhibit binding between Tim-3-Galectin-9, resulting in acting as so-called immune checkpoint inhibitors which can remove the immunosuppressive signals based on the Tim-3-Galectin-9 pathway or in inducing cytotoxicity against cancer cells as a result. For example, single-chain Fv (scFv) can be used as such functional fragments.

For example, when a single-chain Fv (scFv) antibody is used as an antibody etc. of this invention, the half-molecule of the antibody etc. of this invention can be specified as a single chain by linking the amino acid sequence of the heavy chain variable region VH domain in (1) to (12) described below with the amino acid sequence of the light chain variable region VL domain described below:

(1) an amino acid sequence in which the heavy chain variable region of (VH-1a), (VH-1b) or (VH-1c) and the light chain variable region of (VL-1a), (VL-1b) or (VL-1c) are connected via a linker,
(2) an amino acid sequence in which the heavy chain variable region of (VH-2a) or (VH-2b) and the light chain variable region of (VL-2a), (VL-2b), (VL-2c) or (VL-2d) are connected via a linker,
(3) an amino acid sequence in which the heavy chain variable region of (VH-3a) or (VH-3b) and the light chain variable region of (VL-3a), (VL-3b), (VL-3c) or (VL-3d) are connected via a linker,
(4) an amino acid sequence in which the heavy chain variable region of (VH-4a) or (VH-4b) and the light chain variable region of (VL-4a), (VL-4b), (VL-4c) or (VL-4d) connected via a linker, and
(5) an amino acid sequence in which the heavy chain variable region of (VH-5a), (VH-5b) or (VH-5c) and the light chain variable region of (VL-5a), (VL-5b) or (VL-5c) are connected via a linker,

(6) an amino acid sequence in which the heavy chain variable region of (VH-6a), (VH-6b) or (VH-6c) and the light chain variable region of (VL-6a), (VL-6b) or (VL-6c) are connected via a linker,

(7) an amino acid sequence in which the heavy chain variable region of (VH-7) and the light chain variable region of (VL-2a), (VL-2b), (VL-2c) or (VL-2d) are connected via a linker,

(8) an amino acid sequence in which the heavy chain variable region of (VH-8) and the light chain variable region of (VL-2a), (VL-2b), (VL-2c) or (VL-2d) are connected via a linker,

(9) an amino acid sequence in which the heavy chain variable region of (VH-9) and the light chain variable region of (VL-3a), (VL-3b), (VL-3c) or (VL-3d) are connected via a linker,

(10) an amino acid sequence in which the heavy chain variable region of (VH-10) and the light chain variable region of (VL-3a), (VL-3b), (VL-3c) or (VL-3d) are connected via a linker,

(11) an amino acid sequence in which the heavy chain variable region of (VH-11) and the light chain variable region of (VL-4a), (VL-4b), (VL-4c) or (VL-4d) are connected via a linker,

(12) an amino acid sequence in which the heavy chain variable region of (VH-12) and the light chain variable region of (VL-4a), (VL-4b), (VL-4c) or (VL-4d) are connected via a linker.

wherein, the numbers (1) to (12) correspond to the numbers of the aforementioned combinations of the heavy chain CDR1-CDR3 sequences and the numbers of the aforementioned combinations of the light chain CDR1-CDR3 sequences. For example, the amino acid sequence of the variable region of the half-molecule of the 003 single-chain antibody in (1) comprises (1) the amino acid sequences of the heavy chain CDR1 to CDR3 identified by SEQ ID No: 1 to SEQ ID No: 3 and the light chain CDR1 to CDR3 identified by SEQ ID No: 4 to SEQ ID No: 6.

The heavy-chain variable region and the light-chain variable region in scFv may be linked so that either one is on the N-terminal side, and, that is to say, the order of these regions may be heavy-chain variable region-linker-light-chain variable region, or light chain variable region-linker-heavy-chain variable region, from N-terminal side to C-terminal side. Furthermore, the linker connecting between the heavy-chain variable region and the light-chain variable region may be any linker known in the art for use in the field. For example, in the examples described later in this application, GGGGS×4 (i.e., a linker composed of four consecutive GGGGS amino acid sequences) can be used.

When using scFv as a heterodimeric antibody etc. in the second embodiment, scFv antibodies, which are two different types of half molecules, are further connected via a linker to the Fc region of an antibody commonly used in the art to form scFv-Fc, which is then joined to form a heterodimeric The Fc region of the scFv antibody can also be connected via a linker to form scFv-Fc, which is a heterodimeric double chain. In this case, for example, the Fc region can be structured as a linker (e.g., a linker consisting of GGGG) attached to the C-terminal side of scFv.

Production of an antibody etc. or half-molecules of the antibody etc.

The antibody etc. of the present invention can be obtained by a method of administering the Tim-3 antigen as an immunogen to animals of the aforementioned species and fusing the antibody producing cells collected from the animals with myeloma cells to culture (called hybridoma method), by a method of designing a vector for protein expression containing a DNA sequence that can define the amino acid sequence of the antibody etc. and introducing the vector into protein-producing cells to recombinantly obtain the antibodies (called recombinant DNA method), or by a method of are expressing the variable regions of the antibody etc., on the surface of phage as single-chain antibodies (scFv) to obtain a phage that binds to antigens (called phage display method).

All of these methods are commonly used in the technical field,

- For the hybridoma method, see, for example, Kohler and Milstein et al., Nature(1975), Vol.256, p. 495-97, Hongo et al., Hybridoma (1995), Vol.14, No. 3, p. 253-260, Harlow et al., Antibodies: A Laboratory Manual, (Cold Spring Harbor Laboratory Press (1988), Vol.2) and Hammerling et al., Monoclonal Antibodies and T-Cell Hybridomas, p.563-681 (Elsevier, N.Y., 1981), etc.,
- For the recombinant DNA method, see, for example, US4816567,
- For the phage display method, see, for example, US5223409, US5403484, and US5571698 of Ladner et al., US5427908 and US5580717 of Dower et al., US5969108 and US6172197 of McCafferty et al. and US5885793, US6521404, US6544731, US6555313, US6582915 and US6593081 by Griffiths et al. and others.

When an antibody etc. of the present invention is produced by the recombinant DNA method or the phage display method, DNA sequences that can define the amino acid sequences of the antibody etc. of the present invention can be produced by a method of obtaining it from cells producing the desired antibody etc., a method of designing it based on codons optimized for the animal species used in the expression system based on the amino acid sequence, or a combination of these methods.

In the case of producing the antibody etc. of the present invention by the recombinant DNA method, the prepared DNA sequences can be obtained using methods well known to those skilled in the art, for example, using a method of obtaining the DNA sequence by incorporating it into an expression vector suitable for the protein-expressing cell type (e.g., CHO cells) in which the antibody etc. is to be expressed and introducing the expression vector into the protein-expressing cell type, or using any other methods well known to those skilled in the art.

When a homodimeric antibody is produced by the recombinant DNA method, since the structure of the homodimeric antibody is a combination of one type of the heavy chain and one type of the light chain, the antibody etc. of the present invention can be produced by introducing a vector containing a DNA sequence that defines a heavy-chain amino acid sequence and a vector containing a DNA sequence that defines a light-chain amino acid sequence into a protein-expressing cells and expressing both proteins intracellularly to produce an antibody etc. intracellularly, or by introducing a vector containing both a DNA sequence that defines a heavy-chain amino acid sequence and a DNA sequence that defines a light-chain amino acid sequence and expressing both proteins intracellularly to produce an antibody etc. intracellularly.

When a heterodimeric antibody is produced by the recombinant DNA method, since the structure of the heterodimeric antibody is a combination of two types of the heavy chains and two types of the light chains, the antibody etc. of the present invention can be produced by introducing a vector containing a DNA sequence that defines the first heavy chain amino acid sequence, a vector containing a DNA sequence that defines the second heavy chain amino acid sequence, a vector containing a DNA sequence that defines the first light chain amino acid sequence, a vector containing a DNA sequence that defines the second light chain amino acid sequence into the cells for protein expression intracellularly and expressing the four proteins (first and second heavy chains, first and second light chains) intracellularly to produce the antibody etc. intracellularly.

When an antibody etc. of the invention is produced by the phage display method, a library of phages which displays DNA sequences of the produced H- and L-chain variable regions connected by short amino acid sequences on the phages is used to select antibodies with affinity for the target molecules.

When a homodimeric antibody is produced by the phage display method, one type of the heavy chain and one type of the light chain can be connected via a short amino acid sequence and expressed as a single-chain Fv (scFv), and then the Fc region may be linked to the scFv to form a scFv-Fc antibody to produce a homodimeric antibody. Furthermore, a homodimeric antibody etc. can also be produced by separating the VH and VL of the scFv and connecting the constant regions (CH and CL) to each to assemble the antibody etc..

In the case of a method for producing a heterodimeric antibody etc., the probability of obtaining the desired heterodimeric antibody is low since the random combination of two types of proteins (the first heavy chain and the first light chain, the second heavy chain and the second light chain) are occurred. Therefore, it is preferred to use the "knobs-into-holes" technique for the production of a heterodimeric antibody (see, for example, the method described in US7183076B2). In this technique, the "Knob" structure is introduced into the CH3 domain of the constant region of the one half-molecule of the one antibody etc., and the "Hole" structure is introduced into the CH3 domain of the constant region of the other half-molecule of the other antibody etc.. By combining the half-molecule of the antibody etc. having the "Knob" structure in the constant region and the half-molecule of the antibody etc. having the "Hole" structure in the constant region, it is possible to facilitate the formation of the heavy chain heterodimers when two asymmetric heavy chains are formed. The "Knob" and "Hole" structures can be used in combination with the aforementioned heavy chain variable region or single chain Fv (scFv) antibody variable region.

Functions of the antibody etc. of the present invention

The antibody etc. of the present invention, whether in the first embodiment or the second embodiment, has an ability to inhibit the function of the Tim-3 antigen expressed on cancer cells, or to inhibit the function of the Tim-3 antigen expressed on immune cells (especially T cells and antigen-presenting cells), thereby removing immunosuppressive signals against cancer cells, releasing the exhaustion of immune cells (especially T cells), promoting the proliferation of immune cells (especially T cells), increasing the cytotoxicity of immune cells (especially T cells) against cancer cells, and enhancing the secretion of cytokines by immune cells (especially T cells), which results in activating immune cells (especially T cells) or reversing the suppression of the maturation and activation of immune cells (especially dendritic cells), to exhibit tumor growth inhibition effects and cancer treatment efficacy.

Tim-3 antigen (also known as HAVCR2) is known as an immune checkpoint molecule expressed on Th1 cells (type 1 T helper cells), dendritic cells, CD8+ T cells and other lymphocyte subsets. Tim-3 is a key regulator of T cell tolerance and has been shown to play a pivotal role in autoimmunity and T cell exhaustion during chronic viral infection. Tim-3 is known to interact with a lot of ligands such as Phosphatidylserine (PtdSer), Carcinoembryonic antigen-related cell adhesion molecule 1 (CEACAM1), Galectin-9, and high mobility group box 1 (HMGB1), and, among them, the Tim-3-Galectin-9 pathway is known to negatively regulate an immune function of Th1 cells (type 1 T helper cells).

Therefore, as a direct function, the antibody etc. of the present invention binds to the Tim-3 antigen, inhibits the function of the Tim-3 antigen and inhibits binding between Tim-3-Galectin-9, thereby exhibiting an ability to eliminate immunosuppressive signals that negatively regulate immunity by the Tim-3-Galectin-9 pathway, especially exhibiting an ability to eliminate immunosuppressive signals against cancer cells.

In other words, the antibody etc. of the present invention binds to Tim-3 antigen expressed on cancer cells or immune cells, and can function as an immune checkpoint inhibitor that can eliminate immunosuppressive signals based on the Tim-3-Galectin-9 pathway involving Tim-3 antigen.

As a direct function, the antibody etc. of the present invention binds to the Tim-3 antigen and inhibits the function of the Tim-3 antigen, thereby exhibiting an ability to inhibit the binding between Tim-3 and phosphatidylserine (PtdSer).

As mentioned above, the antibody etc. that binds to Tim-3 protein is classified into two groups: those that inhibit PtdSer (the antibodies belonging to Group 1 and Group 2) and those that do not inhibit or partially inhibit PtdSer (antibodies belonging to Group 3, Group 4 and Group 5). In addition, there is no known antibody that can completely inhibit Galectin-9. Therefore, among the antibody etc. of the present invention, a homodimeric antibody etc., which binds at a single site on Tim-3, can exhibit the following functions: partial inhibition of the binding of Tim-3 to Galectin-9; or partial inhibition of the binding of Tim-3 to Galectin-9 and inhibition of the binding of Tim-3 to Phosphatidylserine (PtdSer). On the other hand, among the antibody etc. of the present invention, a heterodimeric antibody etc. (a biparatopic antibody) can exhibit the ability to bind to two different sites on Tim-3 depending on their combination, thereby, for example, inhibiting the binding of Tim-3 to Galectin-9 and inhibiting the binding of Tim-3 to Phosphatidylserine (PtdSer).

As a result of the above-mentioned direct function of suppressing the function of Tim-3 antigen expressed on immune cells, as a secondary effect, the antibody etc. of the present invention can activate the immune cells by relieving the exhaustion of the immune cells (especially T cells), resulting in exhibition of tumor growth suppression and cancer treatment effects.

The immune checkpoint involving Tim-3 functions through a mechanism different from other known immune checkpoint molecules, such as PD-1/PD-L1 and CTLA-4/B7 (CD80/CD86), for example. Therefore, the antibody etc. of the present invention can be used as a backup medicament when the PD-1/PD-L1 inhibitors (e.g., Nivolumab, Pembrolizumab (anti-PD-1 antibodies), Atezolizumab, Durvalumab (anti-PD-L1 antibodies)) or CTLA-4/B7 (CD80/CD86) inhibitors (Ipilimumab (anti-CTLA-4 antibody)) are not effective, or the antibody etc. of the present invention can also be used in combination with these existing immune checkpoint inhibitors.

In this way, the antibody etc. of the present invention is preferably capable of suppressing the function of Tim-3 expressed on cancer cells or immune cells and relieving the exhaustion of the immune cells (especially T cells), thereby activating immune cells and exerting a cancer growth suppression effects and cancer therapeutic effects. Such activation of T cells may occur in vitro or in vivo. For example, to determine in advance whether an antibody against Tim-3 has the ability to activate T cells that are cytotoxic to such cancer cells in a live body (in vivo), the antibody having an ability to bind to the Tim-3 antigen can be screened in vitro by measuring whether the antibody actually activates T cells.

In the context of the present invention, the exhaustion of immune cells (especially T cells) refers to a state where immune cells have become dysfunctional. Specifically, it includes a decrease in cytokine secretion and cytotoxicity of T cells, a decrease in cytokine secretion and cytotoxicity of NK cells, a decrease in antigen-presenting ability of dendritic cells, and activation of regulatory T cells. In such a state, the individual's immune response against cancer cells does not have a function or has a decreased function, resulting in reduced activity to eliminate cancer cells. The exhaustion of immune cells is known to occur when checkpoint proteins (such as PD-1, CTLA-4, Tim-3, etc.) increase on the surface of T cells or when immune system including effector T cells are subjected to long-term stimulation by proliferating cancer cells. However, it is considered to be a reversible condition, and it is considered that the state of exhaustion can be relieved by activating the same cells.

In the context of the present invention, activation of T cells can be measured by indicators such as T cell proliferation, increased cytotoxicity of T cells against cancer cells, and secretion of cytokines from T cells. When the activation of T cells is observed by the antibody etc. of the present invention, it indicates that the antibody etc. of the present invention inhibits the activity of Tim-3 in T cells, leading to the cancer growth suppression effects and the cancer therapeutic effects.

The screening of the ability of the antibody etc. of the present invention to activate T cells can be performed in a live body (in vivo) or outside a live body (in vitro). In vivo screening can be conducted by measuring changes in tumor mass size in the body or by anatomically examining the infiltration of T cells around tumor mass, when the antibody etc. of the present invention are administered to the wild-type mice or the immunodeficient mice such as nude mice or SCID mice, where the target cancer cells have been transplanted. In vitro screening can be carried out by contacting peripheral blood T cells with the antibody etc. of the present invention in the culture condition to measure T cell proliferation, cytokine secretion, or assessing whether T cells induce cell death in cancer cells.

Applications of antibody etc.

The antibody etc. of the present invention, based on its aforementioned functionality of ability to activate immune cells (especially, T cells) with cytotoxicity against cancer cells, can be used to provide a pharmaceutical composition for the treatment of cancer, comprising the antibody etc. of the present invention, by activating immune cells (especially T cells) with cytotoxicity against cancer cells, in a subject requiring cancer treatment or prevention.

The antibody etc. of the present invention specifically binds to cells expressing Tim-3 protein on their surface and has an ability to selectively inhibit the binding between Tim-3 and its ligand. Consequently, the antibody etc. of the present invention is characterized by its ability to resolve negative immune regulation, particularly by inhibiting the binding of Tim-3 to Galectin-9. Therefore, the antibody etc. of the present invention induce cytotoxicity against cancer cells but do not induce cytotoxicity against normal cells, which are non-target cells since the antibody etc. of the present invention eliminate the immune inhibitory signals directed against cancer cells which are occurred through Tim-3 antigen expressed on immune cells (especially T cells), thereby exhibiting anti-cancer effects.

In the present invention, cancer cells that can be therapeutically targeted by the antibody etc. of the present invention cancer cells that express Tim-3 protein, or cancer cells that avoid monitoring and attack by immune cells expressing Tim-3. Examples of such cancer cells may include cells selected from the group consisting of: leukemia (including acute myeloid leukemia, chronic myeloid leukemia, chronic lymphocytic leukemia, acute lymphocytic leukemia), lymphoma (including non-Hodgkin lymphoma, Hodgkin lymphoma, T-cell lymphoma, B-cell lymphoma, Burkitt lymphoma, malignant lymphoma, diffuse lymphoma, follicular lymphoma), myeloma (including multiple myeloma), melanoma, lung cancer, breast cancer, colon cancer, kidney cancer, stomach cancer, ovarian cancer, pancreatic cancer, cervical cancer, uterine cancer, endometrial cancer, esophageal cancer, liver cancer, head and neck cancer, head and neck squamous cell carcinoma, skin cancer, urinary tract cancer, prostate cancer, choriocarcinoma, pharyngeal cancer, laryngeal cancer, myoma, male germinoma,, endometrial hyperplasia, endometriosis, embryonal carcinoma, fibrosarcoma, Kaposi's sarcoma, hemangioma, cavernous hemangioma, hemangioblastoma, retinoblastoma, astrocytoma, neurofibroma, oligodendromatosis, medulloblastoma, neuroblastoma, glioma, rhabdomyosarcoma, glioblastoma,, osteogenic sarcoma, leiomyosarcoma, thyroid sarcoma, and Wilms tumor.

The antibody etc. of the present invention activates immune cells (especially T cells) with cytotoxicity against target cancer cells, as described above. Therefore, when administered in vivo, the antibody etc. exhibits the activation of immune cells (especially T cells) only expressing the target Tim-3 antigen and having cytotoxicity against cancer cells, but is required not to induce clinically problematic cytotoxicity against normal cells that express the Tim-3 antigen.

The antibody etc. of the present invention can also be combined with other drugs, such as other antibodies or anticancer agents, to provide a composition. In one embodiment, the antibody etc. of the present invention can also be combined with drugs to form an antibody-drug conjugate (ADC).

In the present invention, the antibody etc. of the present invention can also be formulated with a physiologically acceptable diluent or carrier. Suitable carriers include, but not limited to, buffer (such as phosphate buffer, citrate buffer, acetate buffer), salts (such as sodium chloride), sugars (such as glucose, trehalose, mannitol, sorbitol), additives (such as amino acids such as arginine, surfactants such as polysorbates), and others. Alternatively, the antibody etc. of the present invention can be lyophilized (freeze-dried) and can be used after reconstitution with an aqueous buffer solution, as mentioned above, when necessary. A formulation containing the antibody etc. of the present invention can be administered in various dosage forms, including parenteral drugs such as injections and intravenous injections drips.

The dosage of the antibody etc. of the present invention varies depending on symptoms, age, weight, etc., but typically can be administered in the range of 0.01 mg to 1000 mg per kg body weight per dose for parenteral administration, preferably, in the range of, for example, 0.05 mg to 500 mg, 0.05 mg-100 mg, 0.05 mg-50 mg, or 0.05 mg-20 mg per kg body weight per day, more preferably 0.1 mg to 10 mg per kg body weight per day, and can be administered via a suitable administration route such as intraperitoneal injection, subcutaneous injection, intramuscular injection, intratumoral injection, or intravenous injection, depending on the type of cancer.

In another embodiment, the present invention may also provide a method of treating or preventing cancer in a subject in need, in which an effective amount of the antibody etc. of the present invention is administered to the subject, based on the characteristics of the antibody etc. of the present invention having a cytotoxic activity against the aforementioned cancer cells. Treatment or prevention of cancer with the antibody etc. of the present invention occurs due to the activation of immune cells (especially T cells) with cytotoxicity against cancer cells in the body.

In the case of administering the antibody etc. of the present invention to a living body for the treatment or prevention of cancer, the antibody etc. can be administered in combination with an adjuvant (e.g., as described in Clin. Microbiol. Rev., 7:277-289, 1994) to effectively generate cellular or humoral immunity in the body, or can be administered in the particle dosage such as liposome formulations, particle-based formulations with diameters in the range of a few micrometers, lipid-bound formulations.

Application of the antibody etc. outside the body

The antibody etc. of the present invention, based on its ability to specifically bind to Tim-3 antigen, can be used to detect Tim-3 antigen in a sample. Specifically, the antibody etc. of the present invention can be used when conducting various detection methods for Tim-3 antigen that can be performed using an antibody, purification methods using an antibody such as immunoprecipitation method, aggregation reaction method, and magnetic beads method, immunoassays such as ELISA method, , Western blot method, and immunohistochemistry method, and immunocytochemistry such as flow cytometry method, on samples that include cancer cells or immune cells (e.g., T cells) collected from subjects. In each case, the antibody etc. of the present invention can be detected using detection labels commonly known to those skilled in the art (such as fluorescence, enzymes, etc.).

The antibody etc. of the present invention, based on its ability to selectively bind to Tim-3 antigens and activates immune cells (especially T cells) with cytotoxicity against cancer cells, can be used to measure the cytotoxicity of the antibody etc. against cancer cells in a subject. Examples of a method for predicting the enhanced cytotoxicity against cancer cells in such a subject, can include, as an example, a method for using the antibody etc. of the present invention to predict enhanced cytotoxicity to cancer cells in a subject, comprising the following steps:

a step of contacting cancer cells collected from the subject to the antibody etc. of the present invention under the culture condition (i.e., in vitro).
a step of measuring whether survival rate of the cancer cells decreases under the culture conditions or whether the secretion of immune activation substances is enhanced.

In the context of the secretion of immune activation substances as the target of measurement, this can be examined by measuring cytokines secreted by T cells, including IL-2, IFN-$\gamma$, and TNF$\alpha$. Preferably, IFN-$\gamma$ is measured.

Lymphocytes cause a decrease in the survival rate of cancer cells in vivo. Using this phenomenon in one embodiment of the present invention, cytotoxicity can be examined by contacting cancer cells collected from a subject with the antibody etc. of the present invention in vitro, and under the culture conditions, measuring the reduction in the cell survival rate of the cancer cells. More specifically, it is possible to predict the enhancement of cytotoxicity against cancer cells in the subject (in vivo) when the antibody etc. of the present invention are administered, by the steps of: contacting cancer cells collected from the subject with the antibody etc. of the present invention under the presence of peripheral blood lymphocytes from the same subject, and measuring the decrease in the cell survival rate of cancer cells under the culture conditions (i.e., in vitro).

It is known that the enhancement of the secretion of immune-activating substances from lymphocytes leads to the activation of T cells and the shrinking of tumors in vivo, which is considered to indicate the activation of antitumor immunity against cancer cells. Using this phenomenon in one embodiment of the present invention, cytotoxicity against cancer cells can be examined by contacting cancer cells collected from a subject with the antibody etc. of the present invention in vitro, and measuring whether the secretion of immune-activating substances is enhanced. More specifically, it is possible to predict the activation of immune cells such as lymphocytes that exhibit cytotoxicity in the subject (in vivo) and the resulting enhancement of cytotoxicity against cancer cells when the antibody etc. of the present invention are administered, by the steps of: contacting cancer cells collected from the subject with the antibody etc. of the present invention under the presence of peripheral blood lymphocytes from the same subject, . and measuring, under cultured conditions, whether the secretion of immune-activating substances from peripheral blood lymphocytes is enhanced.

In this embodiment, IFN-$\gamma$ and TNF$\alpha$ are listed as examples of immune activating substances that are measured to determine cytotoxicity to cancer cells. IFN-$\gamma$ is known to activate immune cells such as NK cells and to exert anti-tumor effects.

The present invention can also provide a measurement kit comprising an antibody etc. of the invention, for measuring, in vitro, cytotoxicity against cancer cells taken from a subject, secretion of immune activating substances, or activation of immune cells.

In addition to the antibody etc. of the invention, the measurement kit for measuring cytotoxicity can include a known means of measuring cell proliferation (e.g., thymidine incorporation, BrdU incorporation, measurement of free lactate dehydrogenase (LDH) activity, measurement of substances derived from living cells (reductase activity, esterase activity, ATP, etc.).

The measurement kit for measuring the secretion of immunoactivating substances can include, in addition to the antibody etc. of the invention, a means for detecting the immunoactivating substance to be measured (e.g., a primary antibody against the immunoactivating substance and a secondary antibody for detection of the primary antibody).

The measurement kit for measuring the activation of immune cells can include a labeled reagent for measuring proliferation of the immune cells using a flow cytometer, in addition to the antibody etc. of the present invention.

The following examples are provided to illustrate the invention in detail. The examples shown below are not intended to limit the invention in any way.

Examples

Example 1: Generation of Anti-Human Tim-3 Monoclonal Antibody

In this example, the genes for antibodies were obtained from the spleen of mice immunized with human Tim-3, and a phage display method was used to produce monoclonal antibodies that bind specifically to human Tim-3.

Mice (MRL/lpr) were immunized with recombinant human Tim-3 (rhTim-3-His: SinoBological, 0390-H08H) and Expi293 cells transiently expressing human Tim-3 antigen (Accession No. JX049979.1), and the spleens were collected after confirmation of elevated serum antibody titers.

Total RNA was extracted from the spleens of the immunized mice, converted to cDNA, and then the antibody gene (VH and Vκ) were amplified by PCR. The PCR products of the amplified VH gene and Vκ gene were connected by a linker (GGGGS $\times$ 4) to form a single chain Fv (hereafter referred to as scFv), which was inserted into a phagemid (ThermoFisher Scientific) derived from pTZ19R. The phagemid inserted with scFv was transformed into E. coli DH12S strain (ThermoFisher Sceintific) followed by infecting with helper phage (M13KO7, New England Biolabs), to construct scFv phage library (size: about 1 $\times$ 10$^8$ clones).

The scFv antibody phage library was subjected to panning with recombinant human Tim-3, and after panning, the phage-infected E. coli was used to express scFv with IPTG-induction, which was screened for scFv clones that bind specifically to human Tim-3 (Accession No. JX049979.1) stable-expressing Jurkat cells.

The nucleotide sequences of the scFvs that were positive in the screening were then sequenced and connected by a linker to a modified antibody Fc region (Fc-LALA with L234A and L235A mutations in the Fc region of IgG1) to form scFv-Fc-LALA antibodies or to a modified antibody Fc region (LALA with L234A mutation and L235A mutation in the Fc region of IgG1) to form IgG1-LALA type antibodies, which were expressed in ExpiCHO cells (Thermo Fisher Scientific) and were affinity purified using Protein A column.

Example 2: Epitope Binning

In this example, epitope binning was performed on the antibodies obtained in Example 1 to group the anti-human Tim-3 antibodies by their epitope sites and to confirm their competitive relationship.

Epitope binning was analyzed by molecular interaction analysis of surface plasmon resonance (SPR) using Biacore8K (Cytiva). Commercially available recombinant human Tim-3 (SinoBological, 10390-H08H) was captured on a Ni-NTA Sensor chip, to which a monoclonal antibody of the scFv-Fc-LALA type obtained in Example 1 (primary antibody) was bound until saturation. Then, a monoclonal antibody of scFv-Fc-LALA type (secondary antibody) different from the primary antibody obtained in Example 1 was added and the binding reaction was determined.

Figure 1 shows the results of the epitope binning. As shown in this figure, the tested antibodies can be classified into Group 1 through Group 5 according to the results of competition and non-competition between the antibodies. Among these classified antibodies, Group 2 antibodies and Group 4 antibodies were found to be non-competitive, and Group 2 antibodies and Group 5 antibodies were found to be non-competitive. In other words, the results indicate that Group 4 antibodies and Group 5 antibodies do not compete with the Group 2 antibody, Ch003 antibody, and can therefore be used as a combination in the formation of biparatopic antibodies.

Among these antibodies, Ch003 antibody in Group 2, and Ch149 antibody, Ch428 antibody, and Ch621 antibody in Group 5 were selected. The CDRs (CDR1 to CDR3 of heavy and light chains) of each antibody were as follows (Table 1).

[Table 1]

| VH | CDR1 | CDR2 | CDR3 |
|---|---|---|---|
| 003 | SYYMS (SEQ ID NO: 1) | TISNSGGSIYYLDSVKD (SEQ ID NO: 2) | DPYYSNYVPMDY (SEQ ID NO: 3) |
| 149 | DYYMD (SEQ ID NO: 9) | YIYPNNGGTSYNQKFKG (SEQ ID NO: 10) | SGYGNYYTMDY (SEQ ID NO: 11) |
| 428 | DYYMD (SEQ ID NO: 17) | YIYPNNGGTSYNQKFKG (SEQ ID NO: 18) | GGYYSYYSYDY (SEQ ID NO: 19) |
| 621 | DYYMD (SEQ ID NO: 25) | YIYPNNGGTSYNQKFKG (SEQ ID NO: 26) | SGYKAYYAMDY (SEQ ID NO: 27) |

| VL | CDR1 | CDR2 | CDR3 |
|---|---|---|---|
| 003 | KASQYVDTYVA (SEQ ID NO: 4) | SASTRHT (SEQ ID NO: 5) | AQYSSSPLT (SEQ ID NO: 6) |
| 149 | KASQSVGNNVA (SEQ ID NO: 12) | YASNRYT (SEQ ID NO:13) | OOHYSSPST (SEQ ID NO:14) |

(continued)

| VL | CDR1 | CDR2 | CDR3 |
|---|---|---|---|
| 428 | KASQSVGNNVA (SEQ ID NO: 20) | YASNRYT (SEQ ID NO: 21) | OOHYSSPYT (SEQ ID NO: 22) |
| 621 | KASQSVGNNVA (SEQ ID NO: 28) | YASNRYT (SEQ ID NO:29) | OOHYSSPYT (SEQ ID NO: 30) |

The amino acid sequences of the VH and VL regions of these antibodies were as follows (underlines indicate CDR sequences).

Ch003 antibody:

VH Region: EVMLMESGGG LVKLGGSLKL SCAASGFTFS SYYMSWVRQT PEKRLEWVAT ISNSGGSIYY LDSVKDRFTI SRDNAKNTLY LQMSSLNSED TAVYYCARDP YYSNYVPMDY WGQGTSVTVS S (SEQ ID NO: 7)

VL Region: SIVMTQTPKF MSTSVGDRVS VTCKASQYVD TYVAWYQQKP GQSPKPLIYS ASTRHTGVPA RFTGSGSGTD FTLTISNVQS EDLAEYFCAQ YSSSPLTFGA GTKLELK (SEQ ID NO: 8)

Ch149 antibody:

VH Region: QVQLQQSGPE RVKPGDSVKM SCKASGYTFT DYYMDWVKQS HGKSLEWIGY IYPNNGGTSY NQKFKGKATL TVDKSSSTAY MELHSLTSED SAVYYCARSG YGNYYTMDYW GQGTSVTVSA (SEQ ID No: 15)

VL Region: SIVMTQTPKF MPVSAGDRVT MTCKASQSVG NNVAWYQQKP GQSPKLLIYY ASNRYTGVPD RFTGSGSGTD FTFTISSVQV EDLAVYFCQQ HYSSPSTFGT GTKLEIK (SEQ ID No: 16)

Ch428 antibody:

VH Region: QVQLQQSGPE LVKPGDSVKM SCKASGYTFT DYYMDWVKQS HGKSLEWIGY IYPNNGGTSY NQKFKGKATL TVDKSSSTAY MELHSLTSED SAVYYCARGG YYSYYSYDYW GQGTTLTVSS (SEQ ID No: 23)

VL Region: SIVMTQTPKF LPVSAGDRVT MTCKASQSVG NNVAWYQQKP GQSPKLLIYY ASNRYTGVPD RFTGSGSGTD FTFTISSVQV EDLAVYFCQQ HYSSPYTFGT GTKLEIK (SEQ ID No: 24)

Ch621 antibody:

VH Region: QIQLQQSGPE LVKPGDSVKM SCKASGYTFT DYYMDWVKQS HGKSLEWIGY IYPNNGGTSY NQKFKGKATL TVDKSSSTAY MELHSLTSED SAVYYCARSG YKAYYAMDYW GQGTTLTVSS (SEQ ID No: 31)

VL Region: SIVMTQTPKF LPVSAGDRVT MTCKASQSVG NNVAWYQQKP GQSPKLLIYY ASNRYTGVPD RFTGSGSGTD FTFTISSVQV EDLAVYFCQQ HYSSPYTFGS GTKLEIK (SEQ ID No: 32)

Example 3: Binding analysis of monoclonal antibody

In this example, binding analysis was performed on the monoclonal antibodies produced in Example 1 and selected in Example 2.

(3-1) Binding affinity analysis

First, IgG1_LALA type antibody (Ch003-LA antibody) and scFv-Fc-LALA type antibody (Ch003_sc antibody, Ch149_sc antibody, Ch428_sc antibody, and Ch621_sc antibody) prepared in Example 1 were used to analyze the binding to recombinant human Tim-3.

The binding affinity of these antibodies to human Tim-3 was measured by surface plasmon resonance (SPR) interaction analysis using Biacore8K. Anti-human Tim-3 antibodies were captured on a sensor chip immobilized with anti-human IgG antibody (Cytiva, cat#29234600), and recombinant human Tim-3 (rhTim-3-His: SinoBological, 10390-H08H) was used as the analyte. The dissociation constant (KD = kd/ka) was calculated based on the binding rate constant (ka) and dissociation rate constant (kd) of each antibody to human Tim-3 obtained by surface plasmon resonance.

The results are shown in Table 2. The monoclonal antibodies of the invention were shown to bind to recombinant human Tim-3 with the following dissociation constants (KD values).

[Table 2]

| scFv-Fc_LALA type antibody | ka (1/Ms) | kd (1/s) | KD (M) |
|---|---|---|---|
| Ch003_sc | 4.63E+ 05 | 6.82E-03 | 1.47E-08 |
| Ch149_sc | 7.58E+ 04 | 2.43E-02 | 3.20E-07 |
| Ch428_sc | 4.44E+ 05 | 9.85E-03 | 2.22E-08 |
| Ch621_sc | 5.83E+ 05 | 6.64E-02 | 1.14E-07 |

| IgG1_LALA type antibody | ka (1/Ms) | kd (1/s) | KD (M) |
|---|---|---|---|
| Ch003-LA | 5.79E+ 05 | 7.27E-03 | 1.26E-08 |

(3-2) Binding analysis to Human Tim-3

Next, binding analysis was performed on the monoclonal antibodies produced in Example 1 and selected in Example 2, using Jurkat cells stably expressing human Tim-3 or Expi293 cells transiently expressing human Tim-3.

The scFv-Fc-LALA type antibodies (Ch003_sc antibody, Ch149_sc antibody, Ch428_sc antibody, and Ch621_sc antibody) prepared in Example 1 were used as antibodies. An isotype control antibody was used as a negative control.

Binding analysis was performed as follows. Cells expressing human Tim-3, such as Jurkat cells stably expressing human Tim-3 or Expi293 cells transiently expressing human Tim-3, were aliquoted in 96 well V-bottom plates at $2 \times 10^5$ cells/well and washed once with 200 $\mu$L (microliter) of PBS (-). Fifty $\mu$L (microliter) of Zombie NIR™ dye (Biolegend, 423106), diluted 500-1000 fold in PBS (-), was added to stain dead cells for 15 minutes at room temperature. Then, 50 $\mu$L (microliter) of Human TruStain FcX (Biolegend, 422302), diluted 20-fold in PBS (-), was added and treated for 20 minutes at room temperature, and washed once with staining buffer [PBS (-), 1% BSA, 0.09% NaN$_3$].

Next, the dilution series of anti-human Tim-3 antibody and the isotype control antibody was adjusted in 5 steps of 10-fold dilution in staining buffer and added to cells at 100 $\mu$L (microliter)/well. After 1 hour incubation at 4°C, washed once with staining buffer, and then the secondary antibody (PE-Goat Anti-human IgG/Jackson Immuno Research Laboratories, 109-116-170) diluted 500-fold in staining buffer was added to cells at 100 $\mu$L (microliter)/well. After 30 min incubation at 4°C followed by washing with staining buffer twice, the fixation buffer (Biolegend, 420801) was added to the cells, and the cells were fixed at room temperature for 20 minutes. The stained cells were suspended in staining buffer and analyzed by flow cytometer. From the results, the mean fluorescence intensity (MFI) corresponding to the antibody concentration was plotted and the KD value was calculated.

The results of the flow cytometry analysis are shown in Figure 2, and the dissociation constant KD values (nM) for each antibody calculated based on the data are shown in Table 3. The monoclonal antibodies of the invention were shown to bind specifically to human Tim-3 expressed on the cell membrane.

[Table 3]

| Ab | Jurkat cells expressing human Tim-3 | Expi293 cells expressing human Tim-3 |
|---|---|---|
|  | KD (nM) | KD (nM) |
| Ch003_sc | 0.08394 | 0.4271 |
| Ch149_sc | 0.5898 | 0.5979 |
| Ch428_sc | 0.07183 | 0.4324 |
| Ch621_sc | 0.2612 | 0.3653 |

(3-3) Activated T cell binding analysis

In addition, the binding of the anti-human Tim-3 monoclonal antibody of the invention, produced in Example 1 then selected in Example 2, to human Tim-3 on activated T cell membranes was analyzed by flow cytometry.

The scFv-Fc-LALA type antibodies (Ch003_sc antibody, Ch149_sc antibody, Ch428_sc antibody, and Ch621_sc antibody) prepared in Example 1 were used as antibodies. The isotype control antibody was used as a negative control.

Activated T cells were prepared as follows. Peripheral blood mononuclear cells (PBMC) $1 \times 10^7$ cells collected from healthy human volunteers were subjected to the Pan T cells isolation Kit, human (Miltenyi Biotec, 130-096-535) to isolate T cells. T cells were suspended in RPMI-1640 medium supplemented with 10% FCS, 100 units/mL of penicillin, and 100 $\mu$g (microgram)/mL of streptomycin at a concentration of $1 \times 10^6$ cells/mL, to which Dynabeads™ Human T-Activator CD3/CD28 (VERITAS, DB11132) were added at a 1:1 ratio, and the cells were cultured for four days at 37°C in a 5% $CO_2$ atmosphere.

Binding analysis was conducted as follows: Activated T cells were plated in 96-well V-bottom plates at a density of $1 \times 10^5$ cells per well, and they were washed once with 200 $\mu$L (microliter) of PBS (-). For dead cell staining, 50 $\mu$L (microliter) of Zombie Violet™ Dye (BioLegend, 423114), diluted 500-1000 times with PBS (-), was added to the cells, which were stained for 15 minutes at room temperature. Afterward, 50 $\mu$L (microliter) of Human TruStain FcX, diluted 20 times with PBS (-), was added to the cells, which were incubated for 20 minutes at room temperature. Finally, the cells were washed once with staining buffer.

Next, dilution series of anti-Tim-3 and isotype-control antibodies were prepared in 5 steps of 10-fold dilution in staining buffer and added to cells at 100 $\mu$L (microliter)/well. After 1 hour incubation at 4°C, cells were washed once with the staining buffer, and incubated with 100 $\mu$L (microliter)/well of the secondary antibody (PE-Goat Anti-human IgG) diluted appropriately in staining buffer and 100 $\mu$L (microliter)/well of T cell marker staining antibody (Biolegend, anti-CD3:UCHT1, anti-CD4:RPA-T4, anti-CD8a:HIT8a) was added to cells. After incubation at 4°C for 30 minutes, the cells were then washed twice with staining buffer and fixed by adding 50 $\mu$L (microliter) of Fixation buffer and allowing to stand at room temperature for 20 minutes. Stained cells were suspended in staining buffer and analyzed by flow cytometer.

From the results of analysis, the mean fluorescence intensity (MFI) corresponding to the antibody concentration was plotted and the KD value was calculated.

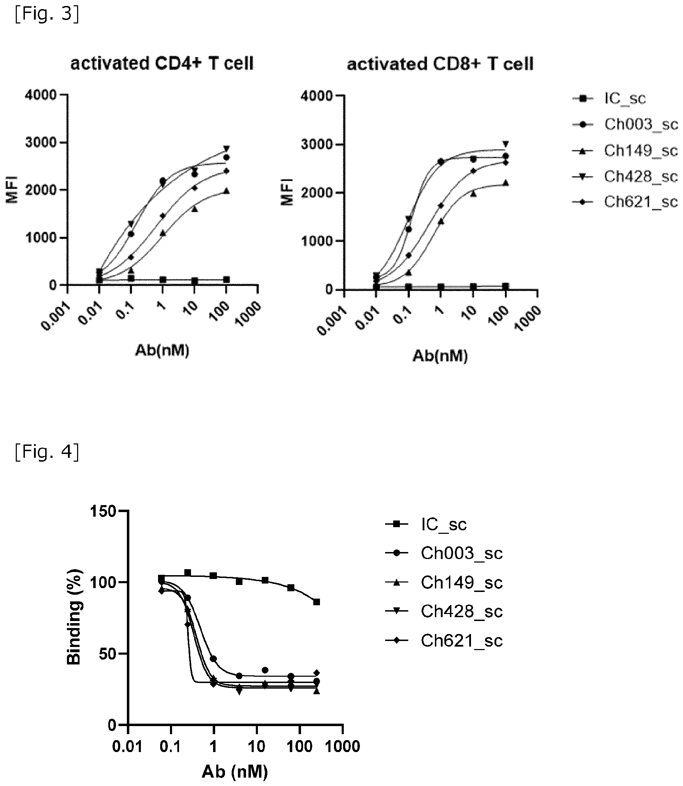

The results of the flow cytometry analysis are shown in Figure 3, and the dissociation constant KD values (nM) for each antibody calculated based on the data are shown in Table 4. The monoclonal antibodies of the present invention specifically bound to human Tim-3 expressed on primary T cells.

[Table 4]

| Ab | activated CD4 cells | activated CD8 cells |
|---|---|---|
|  | KD (nM) | KD (nM) |
| Ch003_sc | 0.1397 | 0.1254 |
| Ch149_sc | 0.7462 | 0.5508 |
| Ch428_sc | 0.1080 | 0.0988 |
| Ch621_sc | 0.4947 | 0.4014 |

Example 4: Inhibitory activity on Ligand binding of monoclonal antibody

In this example, experiments were performed with respect to the monoclonal antibodies produced in Example 1 and selected in Example 2 for the purpose of functional analysis.

(4-1) Galectin-9 binding inhibition

The anti-human Tim-3 antibodies produced in Example 1 and selected in Example 2 of the present invention were analyzed for inhibitory activity against binding between human Tim-3 and human Galectin-9 using ELISA.

The scFv-Fc-LALA type antibodies (Ch003_sc antibody, Ch149_sc antibody, Ch428_sc antibody, and Ch621_sc antibody) prepared in Example 1 were used as antibodies. An isotype control antibody was used as a negative control.

Human Galectin-9 (R&D Systems, 2045-GA) was adjusted to 2.5 $\mu$g (microgram)/mL in 50 mM Carbonate Buffer, pH 9.4, and added at 50 $\mu$L (microliter)/well to 96 well ELISA plates (Corning, 9018) then left at 4°C overnight. The plates were washed with washing buffer [PBS (-), 0.1% Tween 20] three times and blocked for 2 hours at room temperature (Nacalai Tesque, Blocking One:0395395).

During blocking, a 7-step of 4-fold dilution series of each anti-human Tim-3 antibodies were prepared in binding buffer [PBS (-), 0.05% Tween 20, 1/5 vol. Blocking One] and were mixed 1:1 with 200 ng/mL of biotinylated human Tim-3-Fc antigen (in-house preparation, SEQ ID No: 86), which were allowed to incubate for 1 hour at room temperature. The antigen/antibody mixture was added to the plate after blocking at 50 $\mu$L (microliter)/well and allowed to incubate for 1 hour at room temperature.

The plates were then washed three times with washing buffer, and Streptavidin-HRP (Abcam, ab7403) diluted 15,000-fold in binding buffer was added to the plates at 50 $\mu$L (microliter)/well and allowed to incubate for 30 minutes at room temperature. The plates were then washed three times with washing buffer, and TMB+ (Dako, S1599) was added to the plates at 50 $\mu$L/well and allowed to incubate for 10 minutes at room temperature. Finally, 0.5 N sulfuric acid was added to the plates at 50 $\mu$L (microliter)/well to stop color development reaction, and absorbance at 450 nM was measured.

The results of human Tim-3-Fc antigen binding (%) to human Galectin-9 are shown in Figure 4. Although none of the monoclonal antibodies produced in this invention were able to completely inhibit the binding between human Tim-3 and human Galectin-9, the monoclonal antibodies exhibit high inhibitory activity at the maximum inhibition rates ranging from 69.5% to 77.0% (specifically, 69.5% by Ch003_sc, 76.1% by Ch149_sc, 77.0% by Ch428_sc 77.0%, and 74.0% by Ch621_sc).

(4-2) Phosphatidylserine binding inhibition

Next, the anti-human Tim-3 antibodies of the present invention, prepared in Example 1 and selected in Example 2, were analyzed for inhibitory activity against the binding between human Tim-3 and phosphatidylserine using flow cytometry.

The scFv-Fc-LALA type antibodies (Ch003_sc antibody, Ch149_sc antibody, Ch428_sc antibody, and Ch621_sc antibody) prepared in Example 1 were used as antibodies. An isotype control antibody was used as a negative control.

Jurkat cells were adjusted to $5 \times 10^5$ cells/mL in RPMI-1640 medium supplemented with 10% FCS, 100 units/mL penicillin, and 100 $\mu$g (microgram)/mL streptomycin, and were reacted with 100 ng/ mL of Anti-Fas (CD95) mAb (MBL, SY-001) in a 37°C incubator for 3 hours to induce apoptosis.

During apoptosis induction, a 7-step dilution series of each anti-human Tim-3 antibodies were prepared in Annexin V binding buffer (Biolegend) and were mixted 1:1 with 8 $\mu$g (microgram)/mL of biotinylated human Tim-3-Fc antigen, which were allowed to incubate for 1 hour at room temperature.

After the cells after induction of apoptosis being aliquoted into 96 well V-bottom plates, washed with PBS (-),50 $\mu$L (microliter)of Zombie Violet™ Dye diluted 1000-fold in PBS (-) was added to the plates, which were allowed to incubate for 15 minutes at room temperature. After washing once the plates with PBS (-),50 $\mu$L (microliter)/well of Human TruStain FcX was added to the plates, which was allowed to incubate at room temperature for 10 minutes.

Next, 50 $\mu$L (microliter)/well of the antigen/antibody mixture was added to the plates and allowed to incubate at room temperature for 30 minutes. Then, the plates were washed once with Annexin V binding buffer, and 50 $\mu$L (microliter) of PE-Streptavidin (Biolegend, 405204) which were adjusted to 0.3 $\mu$L (microliter)/$1 \times 10^6$ cells with Annexin V binding buffer was added to the plates and allowed to incubate for 15 minutes at room temperature.

Then, the plates were washed once with Annexin V binding buffer, and 100 $\mu$L (microliter) of APC-Annexin V (Biolegend, 640920) which was adjusted with Annexin V binding buffer (Biolegend) to 2 $\mu$L (microliter)/$1 \times 10^6$ cells was added to the plates and allowed to incubate for 15 minutes at 4°C. Finally, 200 $\mu$L (microliter) of Annexin V binding buffer was added to the plates and analyzed by flow cytometer.

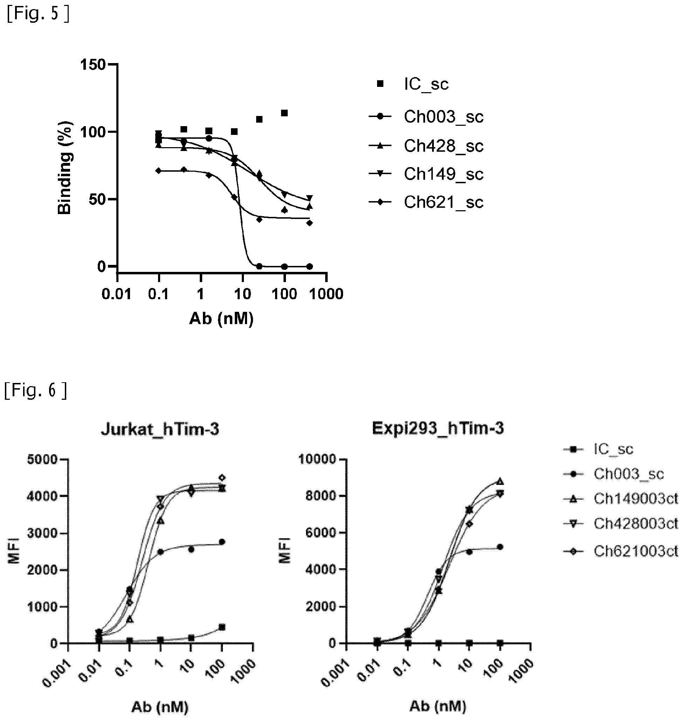

The results of flow cytometry analysis are shown in Figure 5, and the $IC_{50}$ values (nM) for each antibody, calculated based on the results, are shown in Table 5. Among the monoclonal antibodies produced in the present invention, the Ch003_sc antibody almost completely inhibited the binding between human Tim-3 and Phosphatidylserine. The Ch149_sc antibody, the Ch428_sc antibody, and the Ch621_sc antibody exhibited partial inhibition. Compared to the epitope binning results in Example 2, which indicates that the Ch003 antibody belonging to Group 2 and the Ch149 antibody, Ch428 antibody, and Ch621 antibody belonging to Group 5 target non-competing epitope sites, the results of this example demonstrate the correlation between the epitope sites and the functions of each antibody.

[Table 5]

| Ab | IC50 (nM) |
|---|---|
| Ch003_sc | 8.396 |
| Ch149_sc | Weak inhibition |
| Ch428_sc | Weak inhibition |
| Ch621_sc | Weak inhibition |

Example 5: Preparation of Anti-Human Tim-3 Biparatopic Antibody

In this example, biparatopic antibodies that bind to human Tim-3 were produced based on the monoclonal antibodies produced in Example 1 and selected in Example 2.

Among the scFvs that were positive in the screening of Example 1, the Ch003 antibody of Group 2, and the Ch149 antibody, the Ch428 antibody, and the Ch621 antibody of Group 5 that do not compete for binding to Tim-3 as shown in Example 2 were selected and modified using the Knob-into-Hole technology (US7183076B2). For the Ch003 antibody in Group 2, the Fc domain was modified to be a Knob-type, and a C-tag was added at the C-terminus of the antibody. For the Group 5 antibodies, the Ch149 antibody, the Ch428 antibody, and the Ch621 antibody, the Fc domain was modified to be a Hole-type. Then, two different types of DNA encoding these antibodies were inserted into the vector. Specifically, expression vectors were prepared for a combination of Ch003 antibody-coding DNA and Ch149 antibody-coding DNA, a combination of Ch003 antibody-coding DNA and Ch428 antibody-coding DNA, and a combination of Ch003 antibody-coding DNA and Ch621 antibody-coding DNA. Each of these expression vectors was transfected into ExpiCHO cells (Thermo Fisher Scientific) to express and produce the scFv-Fc heterodimeric biparatopic antibodies in the cells.

Biparatopic antibodies were purified directly using an anti-C-tag column.

As a result, Ch149003ct antibody (a combination of a half molecule of Ch149 antibody and a half molecule of Ch003 antibody), Ch428003ct antibody (a combination of a half molecule of Ch428 antibody and a half molecule of Ch003 antibody), Ch621003ct antibody (a combination of a half molecule of Ch621 antibody and a half molecule of Ch003 antibody) were obtained.

Example 6: Binding analysis of biparatopic antibody

In this example, experiments were performed with respect to the anti-human Tim-3 biparatopic antibodies of the invention produced in Example 5 for the purpose of the binding analysis.

(6-1) Binding affinity analysis

First, the biparatopic antibodies (Ch149003ct, Ch428003ct, and Ch621003ct antibodies) prepared in Example 5 were used as antibodies for binding analysis to recombinant human Tim-3.

The analysis of the binding affinity of these biparatopic antibodies to human Tim-3 was carried out using the same method as described in Example 3 (3-1), except that the antibodies used were the biparatopic antibodies (Ch149003ct antibody, Ch428003ct antibody, Ch621003ct antibody).

The results are shown in Table 6. The biparatopic antibodies of the present invention exhibited improved binding affinity compared to the monoclonal antibodies of their origin (Example 3 (3-1) Table 2). This improvement is considered to be exhibited due to an avidity effect because the biparatopic antibodies can bind to the analyte at two different sites, and the results confirm the successful generation of biparatopic antibodies.

[Table 6]

| biparatopic antibody | ka (1/Ms) | kd (1/s) | KD (M) |
|---|---|---|---|
| Ch149003ct | 4.18E+05 | 1.16E-04 | 2.78E-10 |
| Ch428003ct | 4.61E+05 | 1.11E-04 | 2.40E-10 |
| Ch621003ct | 6.48E+05 | 9.05E-05 | 1.39E-10 |

(6-2) Binding analysis to Human Tim-3

Next, the binding analysis was performed on the biparatopic antibodies targeting human Tim-3 produced in Example 5 (Ch149003ct, Ch428003ct, and Ch621003ct) using Jurkat cells stably expressing human Tim-3 or Expi293 cells transiently expressing human Tim-3.

The analysis of the binding affinity of these biparatopic antibodies to human Tim-3 was performed using the same method as described in Example 3 (3-2), except that the antibodies used were the biparatopic antibodies (Ch149003ct antibody, Ch428003ct antibody, and Ch621003ct antibody). The Ch003_sc antibody, for which binding analysis was performed in Example 3 (3-2), was used as a control antibody.

The results of the flow cytometry analysis are shown in Figure 6, and the dissociation constant KD values (nM) for each antibody calculated based on the data are shown in Table 7. The biparatopic antibodies of the present invention, like the monoclonal antibody in Example 3, exhibited specific binding to human Tim-3 expressed on the cell membrane. Additionally, the biparatopic antibodies exhibited increased mean fluorescence intensity (MFI) of saturation binding compared to the original monoclonal antibodies. This increase is considered to be exhibited because one molecule of the biparatopic antibody binds to one molecule of the antigen on the cell membrane, while one molecule of the monoclonal antibody binds to two molecules of the antigen on the cell membrane. Similar to Example 6 (6-1), the results confirm the successful production of the biparatopic antibodies.

[Table 7]

| Ab | Jurkat cells expressing human Tim-3 | Expi293 cells expressing human Tim-3 |
|---|---|---|
|  | KD (nM) | KD (nM) |
| Ch003_sc | 0.08394 | 0.4271 |
| Ch149003ct | 0.4044 | 2.172 |
| Ch428003ct | 0.1939 | 1.370 |
| Ch621003ct | 0.2556 | 1.817 |

(6-3) Furthermore, the binding of the anti-human Tim-3 biparatopic antibodies of the present invention prepared in Example 5 (Ch149003ct antibody, Ch428003ct antibody, Ch621003ct antibody) to human Tim-3 on activated T cell membranes was analyzed by flow cytometry.

The analysis of the binding affinity of the biparatopic antibodies to human Tim-3 was performed using the same method as described in Example 3 (3-3), except that the antibodies used were biparatopic antibodies (Ch149003ct antibody, Ch428003ct antibody, and Ch621003ct antibody). The Ch003_sc antibody, for which binding analysis was performed in Example 3 (3-3), was used as a control antibody.

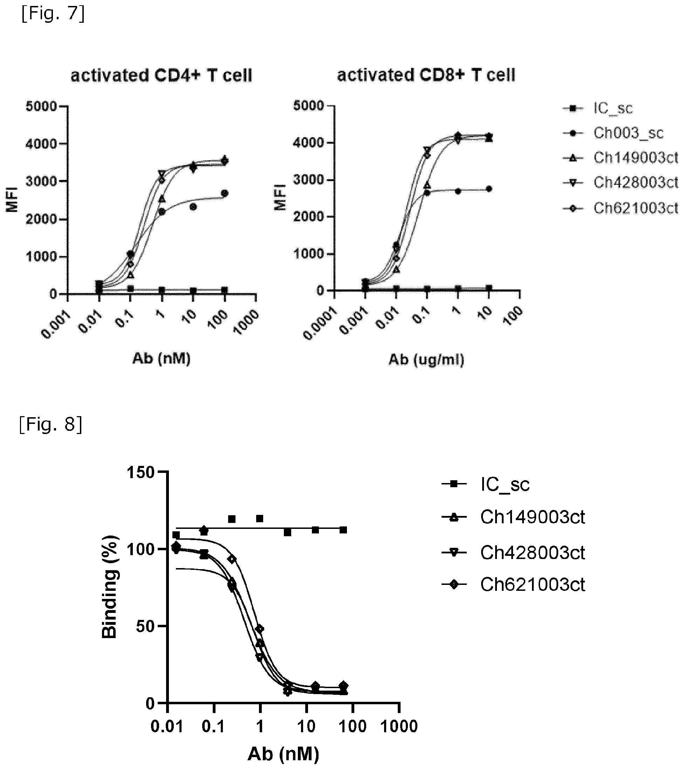

The results of the flow cytometry analysis are shown in Figure 7, and the dissociation constant KD values (nM) for each antibody calculated based on this data are shown in Table 8. The biparatopic antibodies of the present invention, like the monoclonal antibodies of the present invention that demonstrated binding activity in Example 3 (3-3), exhibited specific binding to human Tim-3 expressed on primary T cells. Furthermore, compared to the original monoclonal antibodies, the biparatopic antibodies exhibited increased mean fluorescence intensity (MFI) of saturation binding. These results demonstrate that the biparatopic antibodies produced in the present invention are also functional in primary cells.

[Table 8]

| Ab | Activated CD4 T cells | Activated CD8 T cells |
|---|---|---|
|  | KD (nM) | KD (nM) |
| Ch003_sc | 0.1397 | 0.1254 |

(continued)

| Ab | Activated CD4 T cells | Activated CD8 T cells |
|---|---|---|
| | KD (nM) | KD (nM) |
| Ch149003ct | 0.5079 | 0.5581 |
| Ch428003ct | 0.2185 | 0.2365 |
| Ch621003ct | 0.2870 | 0.3076 |

Example 7: Inhibitory Activity on Ligand Binding of Biparatopic Antibodies

In this example, experiments were conducted with respect to the biparatopic antibodies produced in Example 5 for the purpose of functional analysis.

(7-1) Galectin-9 binding inhibition

The anti-human Tim-3 biparatopic antibodies (Ch149003ct antibody, Ch428003ct antibody, Ch621003ct antibody) of the present invention, produced in Example 5 were analyzed for inhibitory activity against the binding between human Tim-3 and human Galectin-9 using ELISA.

The inhibitory activity of these biparatopic antibodies to the binding between human Tim-3 and human Galectin-9 was confirmed using the same method as described in Example 4 (4-1), except that the antibodies used were the biparatopic antibodies (Ch149003ct antibody, Ch428003ct antibody, Ch621003ct antibody). The Ch003_sc antibody, which was used for binding analysis in Example 4 (4-1), was used as a control antibody.

The results of binding (%) of human Tim-3-Fc antigen to human Galectin-9 are shown in Figure 8 and the $IC_{50}$ values (nM) of each biparatopic antibody calculated based on this analysis are shown in Table 9. Biparatopic antibodies produced in this invention were able to inhibit the binding between human Tim-3 and human Galectin-9 by 90% and above. From these results, it is demonstrated that the biparatopic antibodies produced in this invention exhibit stronger inhibitory activity of the binding between human Tim-3 and human Galectin-9, compared to the monoclonal antibodies of this invention.

[Table 9]

| Ab. ID | IC50 (nM) |
|---|---|
| Ch149003ct | 0.5891 |
| Ch428003ct | 0.4539 |
| Ch621003ct | 0.7512 |

(7-2) Phosphatidylserine binding inhibition

Next, the anti-human Tim-3 biparatopic antibodies of the present invention (Ch149003ct antibody, Ch428003ct antibody, Ch621003ct antibody) produced in Example 5 were analyzed for inhibitory activity against the binding between human Tim-3 and Phosphatidylserine using flow cytometry.

The inhibitory activity of these biparatopic antibodies to the binding between human Tim-3 and Phosphatidylserine was confirmed using the same method as described in Example 4 (4-2), except that the antibodies used were the biparatopic antibodies (Ch149003ct antibody, Ch428003ct antibody, Ch621003ct antibody). The Ch003_sc antibody for which binding analysis was performed in Example 4 (4-2) was also used as a control antibody.

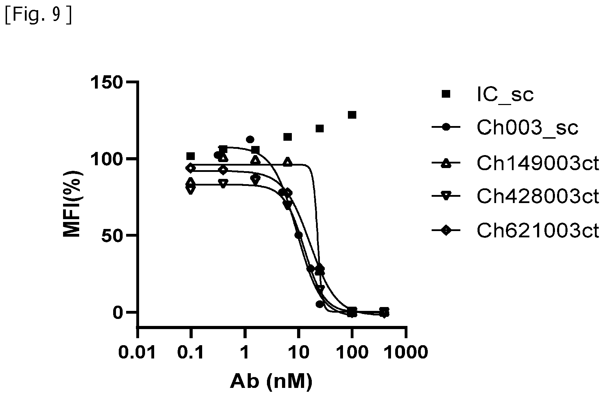

The results of the flow cytometry analysis are shown in Figure 9, and the $IC_{50}$ values (nM) for each antibody calculated based on the analysis data are shown in Table 10. The biparatopic antibodies of the present invention, when compared to the Ch003_sc antibody as described in Example 4 (4-2), exhibited an approximately two-fold increase in $IC_{50}$ values, while they were able to almost completely inhibit the binding between human Tim-3 and Phosphatidylserine.

From the results of Example 7 (7-1) and (7-2), it has been demonstrated that the biparatopic antibodies produced in the present invention are antibodies that strongly inhibit the binding between human Tim-3 and human Galectin-9, and between human Tim-3 and Phosphatidylserine, simultaneously.

[Table 10]

| Ab. ID | IC$_{50}$ (nM) |
|---|---|
| Ch003_sc | 9.321 |
| Ch149003ct | 22.86 |
| Ch428003ct | 12.79 |
| Ch621003ct | 16.32 |

Example 8: Peripheral Blood Mononuclear Cell (PBMC) Stimulation Assay with Staphylococcus Enterotoxin B (SEB)

In this example, the effect of the anti-human Tim-3 monoclonal antibodies produced in Example 1 and the anti-human Tim-3 biparatopic antibodies produced in Example 5 of the present invention on the activation of human peripheral blood T cell was evaluated based on the activation of peripheral blood mononuclear cell (PBMC) induced by Staphylococcus Enterotoxin B (SEB).

As subject antibodies, the scFv-Fc-LALA type antibodies produced in Example 1 (Ch003_sc antibody, Ch149_sc antibody, Ch428_sc antibody, Ch621_sc antibody) and the biparatopic antibodies produced in Example 5 (Ch149003ct antibody, Ch428003ct antibody, Ch621003ct antibody) were used. An isotype control antibody was used as a negative control.

PBMCs collected from healthy human volunteer blood were suspended in RPMI-1640 medium supplemented with 10% FCS, 100 units/mL penicillin, 100 $\mu$g (microgram)/mL streptomycin, and 1×GlutaMax (Gibco, 35050-061), which were seeded in 96-well U-bottom plates at a density of 0.7-0.8×10$^5$ cells/well. The anti-human Tim-3 antibodies were added to the plates to achieve a final concentration of 10 $\mu$g (microgram)/mL and were incubated with the PBMCs for 30 minutes at 37°C under 5% CO$_2$. Subsequently, Staphylococcus Enterotoxin B (SEB) (Toxin Technology Inc., BT202RED) was added to the plates to achieve a final concentration of 1 ng/mL, and the cells were cultured for 10 days at 37°C under 5% CO$_2$.

On the 10th day of the culture, Brefeldin A (Biolegend) was added at a final concentration of 1×, and SEB was added at a final concentration of 10 ng/mL, which was mixed by pipetting and further incubated for 8 hours. The cells were collected in a 96-well V-bottom plate and washed once with 200 $\mu$L (microliter) of PBS (-). To stain dead cells, 50 $\mu$L (microliter) of Zombie-NIR™ (Biolegend) diluted 500-1000 times in PBS (-) was added to the cell culture, which were allowed to incubate for 15 minutes at room temperature. Then, 50 $\mu$L (microliter) of Human TruStain FcX (Biolegend), diluted 20 times in PBS (-), was added to the plates which was allowed to incubate for 20 minutes at room temperature.

Then the biotinylated anti-human Tim-3 antibody (in-house product) which are not compete with the subject antibodies were diluted to 1 $\mu$g (microgram)/mL in staining buffer, and 50 $\mu$L (microliter) of the aliquot was added to the cells, which were allowed to incubate for 30 minutes at 4°C. After washing once the cells with staining buffer, 100 $\mu$L (microliter) each of T cell marker staining antibodies (Biolegend, anti-CD3:UCHT1, anti-CD4: RPA-T4, anti-CD8a: HIT8a) and fluorescently labeled streptavidin that were appropriately diluted in staining buffer was added to the plates. After incubating in the dark at 4°C for 30 minutes, the cells were washed once with the staining buffer, and then 50 $\mu$L (microliter) of the fixation buffer was added to the plates, which was allowed to incubate at room temperature for 20 minutes to fix he cells. After washing twice with Intracellular Staining Permeabilization Wash Buffer (Biolegend), 100 uL of IFN-$\gamma$ staining antibody (Biolegend, anti-IFN-$\gamma$: B27), diluted appropriately in Intracellular Staining Permeabilization Wash Buffer, was added to the cells, then incubated in the dark at 4°C for 40 minutes. After washing twice with Intracellular Staining Permeabilization Wash Buffer, the cells were suspended in staining buffer containing counting beads (ThermoFisher Scientific, C36950) for cell counting, which were analyzed by flow cytometry.

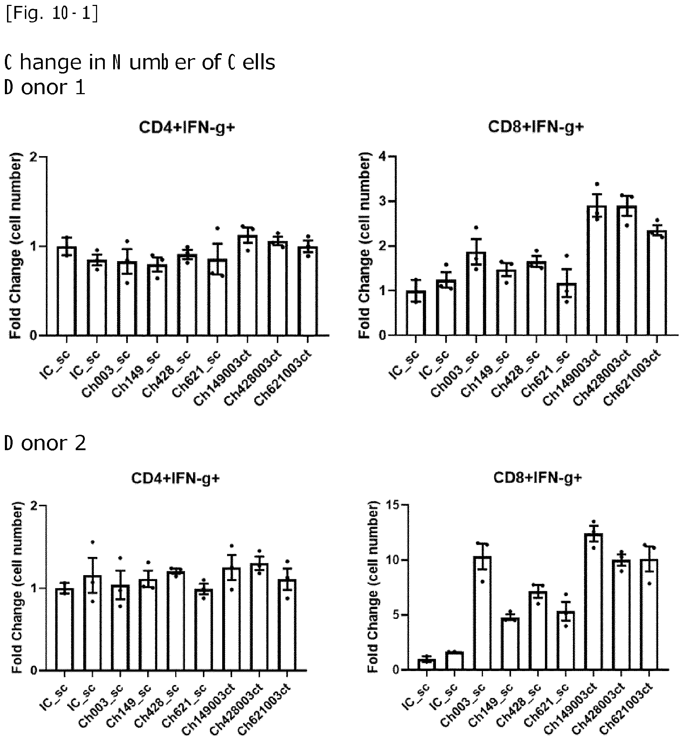

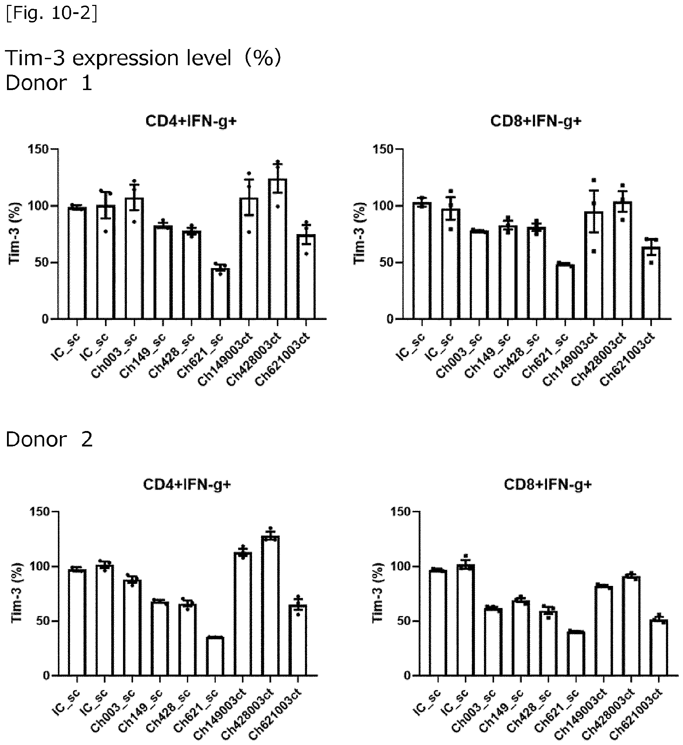

The results of flow cytometry analysis are shown in Figure 10. Figure 10-1 reveals the changes in the number of CD4-positive IFN-$\gamma$-positive cells and that of CD8-positive IFN-$\gamma$-positive cells, and Figure 10-2 reveals the percentage of the Tim-3 expression level on the cell surface (MFI value) of CD4-positive IFN-$\gamma$-positive cells and CD8-positive IFN-$\gamma$-positive cells, with the MFI value of the isotype control being considered as 100% and the MFI value of the staining negative control being considered as 0%. The anti-human Tim-3 monoclonal antibodies produced in the present invention increased the number of CD8-positive IFN-$\gamma$-positive cells. The effect was further enhanced with the anti-human Tim-3 biparatopic antibodies produced in the present invention. This demonstrates that the activation of effector T cells is enhanced by inhibiting not only the binding between human Tim-3 and Phosphatidylserine but also the binding between human Tim-3 and human Galectin-9.

Example 9: Evaluation of Inhibition of T Cell Apoptosis Induction by Galectin-9

In this example, the anti-human Tim-3 monoclonal antibodies and the anti-human Tim-3 biparatopic antibodies of the present invention were evaluated for their ability to inhibit the induction of apoptosis in human peripheral blood T cells by Galectin-9.

PBMCs collected from healthy volunteer blood were suspended in RPMI-1640 medium supplemented with 10% FCS, 100 units/mL penicillin, 100 $\mu$g (microgram)/mL streptomycin, and 1×GlutaMax (Gibco, 35050-061), to which Dynabeads™ Human T-Activator CD3/CD28 were added at a 1:1 ratio, and the cells were cultured for 4 days. The cells were then seeded in a 96-well U-bottom plate at a density of $1.0×10^5$ cells/well, to which anti-human Tim-3 antibodies were added at a final concentration of 10 $\mu$g (microgram)/mL. Then, the cells were incubated for 30 minutes at 37°C under 5% $CO_2$, to which Galectin-9 was then added at a final concentration of 62.5 nM, and the cells were cultured for 8 hours at 37°C under 5% $CO_2$.

The cells were collected in a 96-well V-bottom plate and washed once with 200 $\mu$L (microliter) of PBS (-). To stain dead cells, 50 $\mu$L (microliter) of Zombie-NIR™ (Biolegend), diluted 500-1000 times in PBS (-) was added, which were allowed to incubate for 15 minutes at room temperature. Then, 50 $\mu$L (microliter) of Human TruStain FcX (Biolegend), diluted 20 times in PBS (-), was added to the plates which was allowed to incubate for 20 minutes at room temperature. Next, the biotinylated anti-human Tim-3 antibody (in-house product) which are not compete with the subject antibodies were diluted to 1 $\mu$g (microgram)/mL in staining buffer, and 50 $\mu$L (microliter) aliquot was added to the cells, which were allowed to incubate for 30 minutes at 4°C. After washing once the cells with staining buffer, 100 uL each of T cell marker staining antibodies (Biolegend, anti-CD3:UCHT1, anti-CD4:RPA-T4, anti-CD8a:HIT8a) and fluorescently labeled streptavidin that were appropriately diluted in staining buffer was added to the plates. After incubating in the dark at 4°C for 30 minutes, the cells were washed once with the staining buffer, and then, were washed further using Annexin V Binding Buffer (Biolegend). Next, 100 $\mu$L (microliter) of Fluorescently labeled Annexin V (Biolegend) which was diluted appropriately in Annexin V Binding Buffer was added to the cells. After incubating at room temperature for 15 minutes in the dark, the cells were washed once with Annexin V Binding Buffer. Finally, 50 $\mu$L (microliter) of the fixation buffer was added to the cells, which were allowed to incubate at room temperature for 20 minutes to fix the cells, which were suspended in the staining buffer, and analyzed by flow cytometry.

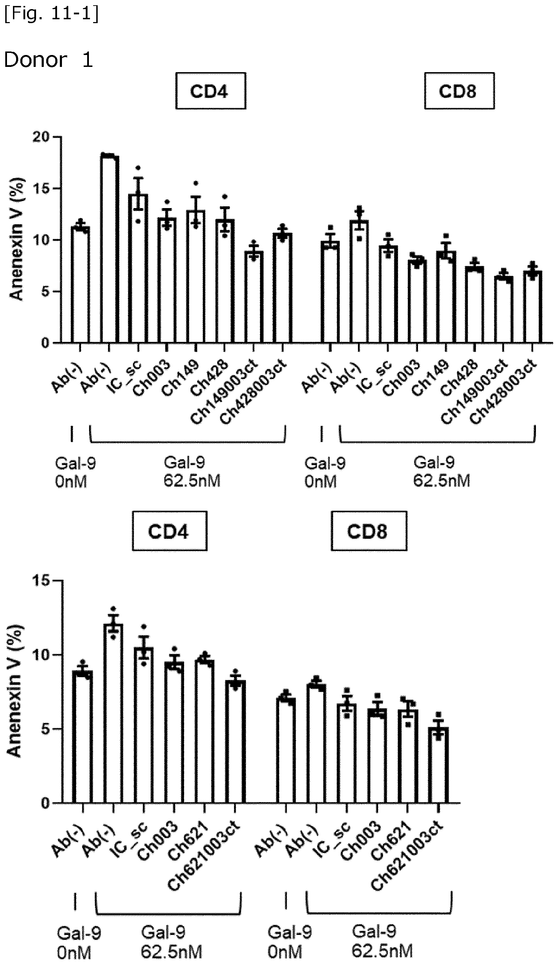

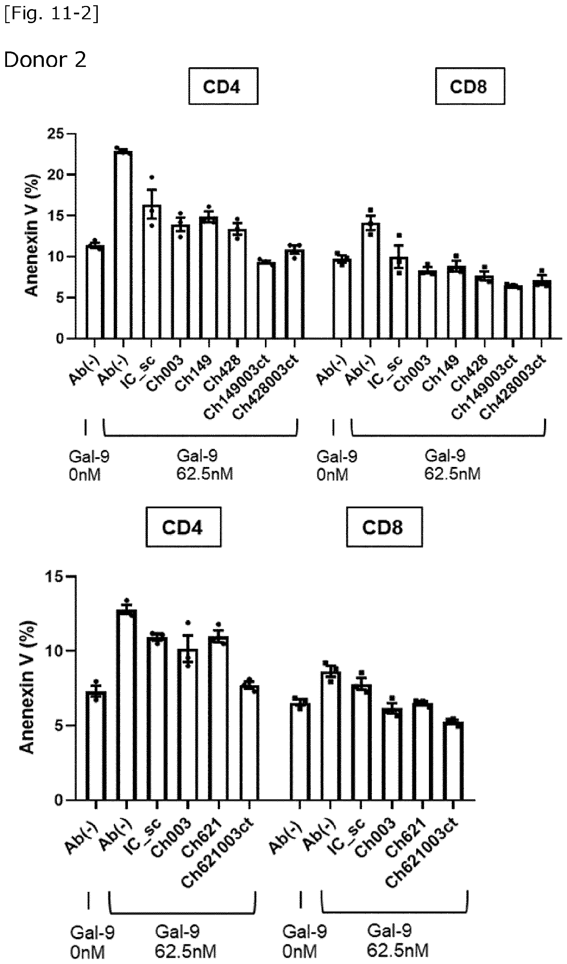

The results are shown in Figure 11. The anti-human Tim-3 monoclonal antibodies produced in the present invention inhibited apoptosis induction of T cells by Galctin-9. This effect was further enhanced with the biparatopic antibodies produced in Example 5; particularly in CD4-positive T cells, the effect reached a level similar to that of the group without Galectin-9 added, indicating that the antibodies effectively neutralize all the added Galectin-9. Further, in CD8-positive T cells, the apoptosis was more suppressed than that of the group without Galectin-9 added, indicating that not only binding to the added Galectin-9, but also Galectin-9 secreted from the cells was also blocked by the antibodies. The enhanced inhibition of binding between human Tim-3 and human Galctin-9 by the biparatopic antibodies was also confirmed on the T cells as enhanced inhibition of apoptosis-induced signaling.

Example 10: CDR Optimization of Ch003 Antibody

Multiple variations in $V_H$ and V$\kappa$ CDR sequences of the anti-human Tim-3 antibody, Ch003 antibody, obtained in this invention had been generated with a high degree of similarity. Therefore, in this example, antibodies were produced in an scFv-Fc-LALA format for all possible combinations of five variations for the heavy chain variable region (a to e) and three variations for the light chain variable region (A1, A2, and B2), and the combination with the higher binding affinity was determined.

The $V_H$ and V$\kappa$ CDR sequences are shown in Table 11, and the KD values for the binding affinity to human Tim-3 for each combination are shown in Table 12. As a result, it was revealed that the combination of the heavy chain variable region d and the light chain variable regions A1 or A2 showed particularly high binding affinity (see, Table 12). In the following examples, an antibody with the heavy chain variable region d and the light chain variable regions A1 was used as Ch003_F20 antibody, and an antibody with the heavy chain variable region d and the light chain variable region B2 was used as Ch072 antibody.

[Table 11]

| VH# | VH-CDR1 | VH-CDR2 | VH-CDR3 |
|---|---|---|---|
| a | SYYMS (SEQ ID NO: 1) | TISNSGGSIYYLDSVKD (SEQ ID NO:2) | DPYYSNYVPMDY (SEQ ID NO: 3) |
| b | SYYMS (SEQ ID NO: 37) | TISNSGGSTYYPDSVKD (SEQ ID NO: 38) | DPYYSNYVPMDY (SEQ ID NO: 39) |
| c | SYYMS (SEQ ID NO: 40) | TISNSGGSIYYPDSVKD (SEQ ID NO: 41) | DPYYSNYVPMDY (SEQ ID NO: 42) |
| d | SYYMS | TISNSGGSTYYPDSVKD | DPYYTNYVPMDY |
|  | (SEQ ID NO: 33) | (SEQ ID NO: 34) | (SEQ ID NO: 35) |
| e | SYYMS (SEQ ID NO: 43) | TVSNSGGSTYYPDSVKD (SEQ ID NO: 44) | DPYYTNYVPMDY (SEQ ID NO: 45) |

| VK# | VL-CDR1 | VL-CDR2 | VL-CDR3 |
|---|---|---|---|
| A1 | KASQYVDTYVA (SEQ ID NO: 4) | SASTRHT (SEQ ID NO: 5) | AQYSSSPLT (SEQ ID NO: 6) |
| A2 | KASQYVGTYVA (SEQ ID NO: 46) | SASTRHT (SEQ ID NO: 47) | EQYSSSPLT (SEQ ID NO: 48) |
| B2 | KASENVGTYVS (SEQ ID NO: 52) | GASNRYT (SEQ ID NO: 53) | GQSYSYPLT (SEQ ID NO: 54) |

[Table 12]

| KD value of each antibody | | | | | | |
|---|---|---|---|---|---|---|
| KD (M) | | VH# | | | | |
| | | a | b | c | d | e |
| VK# | A1 | 1.33E-08 | 9.54E-09 | 7.87E-09 | 4.68E-09 | 3.94E-09 |
| | A2 | 1.17E-08 | 9.07E-09 | 8.40E-09 | 5.01E-09 | 3.87E-09 |
| | B2 | 1.1 2E-06 | 9.00E-07 | 1.21E-06 | 7.92E-07 | 6.66E-07 |

The amino acid sequences of the VH and VL regions of the Ch003_F20 and CH072 antibodies were as follows (underlines indicate CDR sequences). Ch003_F20 antibody:

VH Region: EVMLMESGGG LVKLGGSLKL SCAASGFTFS SYYMSWVRQT PEKRLEWVAT ISNSGGSTYY PDSVKDRFTI SRDNAKNTLY LQMSSLNSED TAVYYCARDP YYTNYVPMDY WGQGTSVTVS S (SEQ ID No: 36)
VL region: SIVMTQTPKF MSTSVGDRVS VTCKASQYVD TYVAWYQQKP GQSPKPLIYS ASTRHTGVPA RFTGSGSGTD FTLTISNVQS EDLAEYFCAQ YSSSPLTFGA GTKLELK (SEQ ID No: 8)
Ch072 antibody:
VH Region: EVKLVESGGG LVKLGGSLKL SCAASGFTFS SYYMSWVRQT PEKRLEWVAT ISNSGGSTYY PDSVKDRFTI SRDNAKNTLY LQMSSLNSED TAVYYCARDP YYTNYVPMDY WGQGTSVTVS S (SEQ ID No: 55)

VL region: NIVMTQSPKS MSMSVGERVT LSCKASENVG TYVSWYQQKP EQSPKLLIYG ASNRYTGVPD RFTGSGSATD FTLTISSVQA EDLADYYCGQ SYSYPLTFGA GTKLELK (SEQ ID No: 56)

Example 11: Humanization of mouse antibodies.

In this example, mouse antibodies obtained in Example 1 and Example 10 were humanized using the CDR grafting method.

(11-1) Humanization of Ch003 antibody and Ch003_F20 antibody.

In this example, mouse antibodies obtained in Example 1 and in Example 10 were humanized using CDR grafting method.

The Ch003 antibody analyzed in Example 2 and the Ch003_F20 antibody with CDR optimization in Example 10 were selected as antibodies and were humanized. CDRs were identified by the Kabat numbering method. Sequences with high homology to the VH and Vκ framework sequences of each mouse antibody were selected from known human antibody sequences, to which the CDRs of the mouse antibodies were grafted, to produce the humanized VH and Vκ sequences If necessary, structurally important sites in the framework (Canonical, Vernier, Interface) were replaced with sequences from the originating mouse antibody.

Since the VH and Vκ sequences of the mouse antibodies from which two antibodies mentioned above (Ch003 antibody and Ch003_F20 antibody) are derived are similar with each other, Ch003 antibody was selected as the representative clone and used to create multiple humanized VH and Vκ sequences and to combine with the originated mouse VH and Vκ sequences. Relating to these produced antibodies, humanized VH1 and VH2, and humanized Vκ2 and Vκ3 were chosen based on maintaining equal or higher binding affinities to human Tim-3 compared to the original mouse antibodies. All of these humanized VH and Vκ combinations (refer to Table 13 below) exhibited equal or higher binding affinities to human Tim-3 compared to the original mouse antibodies.

Humanization of the Ch003_F20 antibody was performed by selecting the VH1 and Vκ3 framework sequences from the humanized sequences of Ch003 (see Table 13).

[Table 13]

| Fw (003) | VK2 | VK3 |
|---|---|---|
| VH1 | Hu003_12 | Hu003_13 |
| VH2 | Hu003_22 | Hu003_23 |
|  |  |  |
| Fw (003_F20) | Vκ2 | Vκ3 |
| VH1 | Hu003_12_F20 | Hu003_13_F20 |
| VH2 | Hu003_22_F20 | Hu003_23_F20 |

The sequences of the heavy and light chains for the humanized antibodies (Hu003_12 antibody, Hu003_13 antibody, Hu003_22 antibody, Hu003_23 antibody, and Hu003_13_F20 antibody) produced based on the Ch003 antibody and Ch003_F20 antibody are presented in the following Table 14 (the Hu003 antibody sequences are shown in Table 14-1, the Hu003_F20 antibody sequences are shown in Table 14-2 and Table 14-3).

[Table 14-1]

| VH | VR1 | CDR1 | VR2 | CDR2 |
|---|---|---|---|---|
| Hu003 VH1 (Hu003_12 & Hu003_13) | QVQLQESGGG LVKPGGSLKL SCAASGFTFS | SYYMS | WVRQTPDKRL EWVA | TISNSGGSIY YLDSVKD |

| Hu003 VH2 (Hu003_22 & Hu003_23) | **E**VQLVESGGG LVKPGGSL**RL** SCAASGFTFS | SYYMS | WVRQTPEKRL EWVA | TISNSGGSIY YLDSVKD |
|---|---|---|---|---|

| VH (continued) | VR3 | CDR3 | VR4 | |
|---|---|---|---|---|
| Hu003 VH1 (Hu003_12 & Hu003_13) | RFTISRDNAK NTLYLQMSSL KSEDTAMYYC AR | DPYYSNYVPM DY | WGQGTMVTVS S | SEQ ID No: 49 |
| Hu003 VH2 (Hu003_22 & Hu003_23) | RFTISRDNAK N**S**LYLQMN**S**L **RA**EDTA**V**YYC AR | DPYYSNYVPM DY | WGQGT**T**VTVS S | SEQ ID No: 57 |

| Vκ | VR1 | CDR1 | VR2 | CDR2 |
|---|---|---|---|---|
| Hu003 Vκ2 (Hu003_12 & Hu003_22) | DIVMTQTPSS LSASVGDRVT ITC | KASQYVDTYV A | WYQQKPGKAP KPLIY | SASTRHT |
| Hu003 Vκ3 (Hu003_13 & Hu003_23) | DIVMTQSPSF LSASVGDRVT ITC | KASQYVDTYV A | WYQQRPGKAP KPLIY | SASTRHT |

| Vκ (continued) | VR3 | CDR3 | VR4 | |
|---|---|---|---|---|
| Hu003 Vκ2 (Hu003_12 & Hu003_22) | GVPSRFSGSG SGTDFTLTIS SLQPEDFATY FC | AQYSSSPLT | FGQGTKLELK | SEQ ID No: 58 |
| Hu003 Vκ3 (Hu003_13 & Hu003_23) | GVPSRFSGSG SGTEFTLTIS SLQPEDFATY FC | AQYSSSPLT | FGGGTKLELK | SEQ ID No: 50 |

[Table 14-2]

| VH | VR1 | CDR1 | VR2 | CDR2 |
|---|---|---|---|---|
| Hu003_F20 VH1 (Hu003_12_F20 & Hu003_13_F20) | QVQLQESGGG LVKPGGSLKL SCAASGFTFS | SYYMS | WVRQTPDKRL EWVA | TISNSGGSTY YPDSVKD |

| Hu003_F20 VH2 (Hu003_22_F20& Hu003_23_F20) | **E**VQLVESGGG LVKPGGSL**RL** SCAASGFTFS | SYYMS | WVRQTPEKRL EWVA | TISNSGGSTY YPDSVKD |
|---|---|---|---|---|

| VH (continued) | VR3 | CDR3 | VR4 | |
|---|---|---|---|---|
| Hu003_F20 VH1 (Hu003_12_F20& Hu003_13_F20) | RFTISRDNAK NTLYLQMSSL KSEDTAMYYC AR | DPYYTNYVPM DY | WGQGTMVTVS S | SEQ ID No: 51 |
| Hu003_F20 VH2 (Hu003_22_F20& Hu003_23_F20) | RFTISRDNAK N**S**LYLQMN**S**L **RA**EDTA**V**YYC AR | DPYYTNYVPMDY | WGQGTTVTVSS | SEQ ID No: 59 |

[Table 14-3]

| VK | VR1 | CDR1 | VR2 | CDR2 |
|---|---|---|---|---|
| Hu003_F20 VK2 (Hu003_12_F20& Hu003_22_F20) | DIVMTQTPSS LSASVGDRVT ITC | KASQYVDTYV A | WYQQKPGKAP KPLIY | SASTRHT |
| Hu003_F20 VK3 (Hu003_13_F20& Hu003_23_F20) | DIVMTQSPSF LSASVGDRVT ITC | KASQYVDTYV A | WYQQRPGKAP KPLIY | SASTRHT |

| VK (continued) | VR3 | CDR3 | VR4 | |
|---|---|---|---|---|
| Hu003_F20 VK2 (Hu003_12_F20& Hu003_22_F20) | GVPSRFSGSG SGTDFTLTIS SLQPEDFATY FC | AQYSSSPLT | FGQGTKLELK | SEQ ID No: 58 |
| Hu003_F20 VK3 (Hu003_13_F20& Hu003_23_F20) | GVPSRFSGSG SGTEFTLTIS SLQPEDFATY FC | AQYSSSPLT | FGGGTKLELK | SEQ ID No: 50 |

(11-2) Humanization of Ch072 antibody

In this example, mouse antibody was humanized by CDR grafting on the Ch072 antibody (heavy chain variable region d and light chain variable region B2) obtained in Example 10.

Humanization of this antibody was performed in the same method as described in (1), except that the target antibody was a Ch072 antibody.

Finally, relating to these produced antibodies, humanized VH1, Vκ1, and Vκ3 were chosen based on maintaining equal or higher binding affinities to human Tim-3 compared to the original mouse antibodies (see Table 15). All of these humanized VH and Vκ combinations exhibited equal or higher binding affinities to human Tim-3 compared to the original mouse antibodies.

[Table 15]

| Fw (072) | Vκ1 | Vκ3 |
|---|---|---|
| VH1 (= 003_F20 VH1) | Hu072_11 | Hu072_13 |
| VH2 (=003_F20 VH2) | Hu072_21 | Hu072_23 |

The resulting sequences of the heavy and light chains of the humanized antibodies (Hu072_11 antibody and Hu072_13 antibody) produced based on the Ch072 antibody are shown in Table 16.

[Table 16]

| VH | VR1 | CDR1 | VR2 | CDR2 |
|---|---|---|---|---|
| Hu072 VH1 (Hu072_11 & Hu072_13) | QVQLQESGGG LVKPGGSLKL SCAASGFTFS | SYYMS | WVRQTPDKRL EWVA | TISNSGGSTY YPDSVKD |
| Hu072 VH2 (Hu072_21 & Hu072_23) | EVQLVESGGG LVKPGGSLRL SCAASGFTFS | SYYMS | WVRQTPEKRL EWVA | TISNSGGSTY YPDSVKD |

| VH (continued) | VR3 | CDR3 | VR4 | |
|---|---|---|---|---|
| Hu072 VH1 (Hu072_11 & Hu072_13) | RFTISRDNAK NTLYLQMSSL KSEDTAMYYC AR | DPYYTNYVPM DY | WGQGTMVTVS S | SEQ ID NO: 51 |
| Hu072 VH1 (Hu072_11 & Hu072_13) | RFTISRDNAK NSLYLQMNSL RAEDTAVYYC AR | DPYYTNYVPM DY | WGQGTTVTVS S | SEQ ID NO: 59 |

| Vκ | VR1 | CDR1 | VR2 | CDR2 |
|---|---|---|---|---|
| Hu072 Vκ (Hu072_11) | DIVMTQSPDS LAVSLGERVT LSC | KASENVGTYVS | WYQQKPRQPP KLLIY | GASNRYT |

| Hu072 Vκ (Hu072_13) | NIVMTQSPDS LAVSLGERAT INC | KASENVGTYVS | WYQQKPGQPP KLLIY | GASNRYT |
|---|---|---|---|---|

| Vκ (continued) | VR3 | CDR3 | VR4 | |
|---|---|---|---|---|
| Hu072 Vκ (Hu072_11) | GVPDRFSGSG SGTDFTLTIS SLQAEDVALY YC | GQSYSYPLT | FGQGTRLEIK | SEQ ID NO: 60 |
| Hu072 Vκ (Hu072_13) | GVPDRFSGSG SGTDFTLTIS SLQAEDVAVY YC | GQSYSYPLT | FGQGTKLEIK | SEQ ID NO: 61 |

(11-3) Humanization and heavy chain CDR2 sequence modification of Ch149, Ch428 and Ch621 antibodies

In this example, mouse antibodies were humanized by CDR grafting on Ch149, Ch428 and Ch621 antibodies produced in Example 1 and selected in Example 2.

Humanization of these antibodies was performed in the same method as described in (1), except that the target antibodies were Ch149, Ch428, and Ch621 antibodies.

Since the VH and Vκ sequences of the mouse antibodies from which three antibodies above (Ch149, Ch428, and Ch621 antibodies) are derived are similar, Ch428 antibody was used as representatives clone. Specifically, relating to the Ch428 antibody, humanized sequences of the heavy and light chains were created, and antibodies were produced by combining the humanized light chain against the chimeric sequence heavy chain and the chimeric sequence light chain against the humanized heavy chain, respectively. Finally, for the resulting antibodies, combinations of the chimeric sequence heavy chain with humanized Vκ1, Vκ3, or Vx4, and combinations of humanized VH4 with chimeric sequence light chain were chosen based on maintaining binding equal or higher binding affinity compared to that of the original mouse antibody (see Table 17).

[Table 17]

| Fw(428) | Vκ1 | Vκ3 | Vκ4 |
|---|---|---|---|
| Chimera HC | Hu428_C1 | Hu428_C3 | Hu428_C4 |

| Fw (428) | Chimera Vκ |
|---|---|
| VH4 | Hu428_4C |

Based on these results, humanized Ch428 antibodies, Hu428_41, Hu428_43, and Hu428_44 antibodies were produced by combining the VH4 sequence of Hu428 antibody as the heavy chain and Vκ1, Vκ3, or Vκ4 of Hu428 antibody as the light chain (see Table 18). The amino acid sequences of the heavy and light chains of the resulting Hu428_41, Hu428_43, and Hu428_44 antibodies, respectively, were as described in the following table (see Table 19).

[Table 18]

| Fw(428) | Vκ1 | Vκ3 | Vκ4 |
|---|---|---|---|
| VH4 | Hu428_41 | Hu428_43 | Hu428_44 |

46

[Table 19]

| VH | VR1 | CDR1 | VR2 | CDR2 |
|---|---|---|---|---|
| Hu428 VH4 (Hu428_41, Hu428_43 & Hu428_44) | QVQLVQSGAEVK KPGASVKVSCKA SGYTFT | DYYMD | WVRQAPGQGLE WLG | YIYPNNGGTSYN QKFKG |

| VH (continued) | VR3 | CDR3 | VR4 | |
|---|---|---|---|---|
| Hu428 VH4 (Hu428_41, Hu428_43 & Hu428_44) | RVTMTTDKSTST AYMELRSLRSDD TAVYYCAR | GGYYSYYSYDY | WGQGTTVTVSS | SEQ ID NO: 66 |

| Vκ | VR1 | CDR1 | VR2 | CDR2 |
|---|---|---|---|---|
| Hu428 Vκ1 (Hu428_41) | DIVMTQSPDSLA VSLGERVTINC | KASQSVGNNVA | WYQQKPGQSPKL LIY | YASNRYT |
| Hu428 Vκ3 (Hu428_43) | DIVMTQSQKFMS TSVGDRVSITC | KASQSVGNNVA | WYQQKPGQSPKL LIY | YASNRYT |
| Hu428 Vκ4 (Hu428_44) | DIVMTQSPDSLP VSLGERATINC | KASQSVGNNVA | WYQQKPGQPPKL LIY | YASNRYT |

| Vκ (continued) | VR3 | CDR3 | VR4 | |
|---|---|---|---|---|
| Hu428 Vκ1 (Hu428_41) | GVPDRFSGSGSG TDFTLTISSLQAE DVAVYYC | QQHYSSPYT | FGQGTKLEIK | SEQ ID NO: 67 |
| Hu428 Vκ3 (Hu428_43) | GVPDRFTGSGSG TDFTLTISNMQSE DLADYFC | QQHYSSPYT | FGGGTKLEIK | SEQ ID NO: 68 |
| Hu428 Vκ4 (Hu428_44) | GVPDRFSGSGSG TDFTLTISSLQAE DVAVYYC | QQHYSSPYT | FGQGTKLEIK | SEQ ID NO: 69 |

The framework obtained for the Hu428 antibody was subsequently combined with the CDR sequences of the Ch149 and Ch621 antibodies to humanize the Ch149 and Ch621 antibodies (see Table 20). The sequences of the heavy and light chains of the humanized antibodies for Ch149 and Ch621 antibodies (Hu149 and Hu621 antibodies) obtained based on the sequences obtained for the Hu428 antibody are shown in Table 21.

[Table 20]

| Fw (149) | Vκ1 | Vκ3 | Vκ4 |
|---|---|---|---|
| VH4 | Hu149_41 | Hu149_43 | Hu149_44 |
| | | | |
| Fw (621) | Vκ1 | Vκ3 | Vκ4 |
| VH4 | Hu621_41 | Hu621_43 | Hu621_44 |

[Table 21-1]

| VH | VR1 | CDR1 | VR2 | CDR2 |
|---|---|---|---|---|
| Hu149 VH4 (Hu149_41, Hu149_43 & Hu149_44) | QVQLVQSGAEVK KPGASVKVSCKAS GYTFT | DYYMD | WVRQAPGQGLE WLG | YIYPNNGGTSYNQ KFKG |

| VH (continued) | VR3 | CDR3 | VR4 | |
|---|---|---|---|---|
| Hu149 VH4 (Hu149_41, Hu149_43 & Hu149_44) | RVTMTTDKSTSTA YMELRSLRSDDTA VYYCAR | SGYGNYYTMDY | WGQGTTVTVSS | SEQ ID NO: 62 |

| Vκ | VR1 | CDR1 | VR2 | CDR2 |
|---|---|---|---|---|
| Hu149 Vκ1 (Hu149_41) | DIVMTQSPDSLAV SLGERVTINC | KASQSVGNNVA | WYQQKPGQSPKL LIY | YASNRYT |
| Hu149 Vκ3 (Hu149_43) | DIVMTQSQKFMS TSVGDRVSITC | KASQSVGNNVA | WYQQKPGQSPKL LIY | YASNRYT |
| Hu149 Vκ4 (Hu149_44) | DIVMTQSPDSLPV SLGERATINC | KASQSVGNNVA | WYQQKPGQPPKL LIY | YASNRYT |

| Vκ (continued) | VR3 | CDR3 | VR4 | |
|---|---|---|---|---|
| Hu149 Vκ1 (Hu149_41) | GVPDRFSGSGSGT DFTLTISSLQAED VAVYYC | QQHYSSPST | FGQGTKLEIK | SEQ ID NO: 63 |
| Hu149 Vκ3 (Hu149_43) | GVPDRFTGSGSGT DFTLTISNMQSED LADYFC | QQHYSSPST | FGGGTKLEIK | SEQ ID NO: 64 |
| Hu149 Vκ4 (Hu149_44) | GVPDRFSGSGSGT DFTLTISSLQAED VAVYYC | QQHYSSPST | FGQGTKLEIK | SEQ ID NO: 65 |

[Table 21-2]

| VH | VR1 | CDR1 | VR2 | CDR2 |
|---|---|---|---|---|
| Hu621 VH4 (Hu621_41, Hu621_43 & Hu621_44) | QVQLVQSGAEVK KPGASVKVSCKAS GYTFT | DYYMD | WVRQAPGQGLE WLG | YIYPNNGGTSYNQ KFKG |

| VH (continued) | VR3 | CDR3 | VR4 | |
|---|---|---|---|---|
| Hu621 VH4 (Hu621_41, Hu621_43 & Hu621_44) | RVTMTTDKSTSTA YMELRSLRSDDTA VYYCAR | SGYKAYYAMDY | WGQGTTVTVSS | SEQ ID NO: 70 |

| Vκ | VR1 | CDR1 | VR2 | CDR2 |
|---|---|---|---|---|
| Hu621 V1 (Hu621_41) | DIVMTQSPDSLAV SLGERVTINC | KASQSVGNNVA | WYQQKPGQSPKL LIY | YASNRYT |
| Hu621 Vκ3 (Hu621_43) | DIVMTQSQKFMS TSVGDRVSITC | KASQSVGNNVA | WYQQKPGQSPKL LIY | YASNRYT |
| Hu621 Vκ4 (Hu621_44) | DIVMTQSPDSLPV SLGERATINC | KASQSVGNNVA | WYQQKPGQPPKL LIY | YASNRYT |

| Vκ (continued) | VR3 | CDR3 | VR4 | |
|---|---|---|---|---|

| Hu621 Vκ1 (Hu621_41) | GVPDRFSGSGSGT DFTLTISSLQAED VAVYYC | QQHYSSPYT | FGQGTKLEIK | SEQ ID NO: 71 |
|---|---|---|---|---|
| Hu621 Vκ3 (Hu621_43) | GVPDRFTGSGSGT DFTLTISNMQSED LADYFC | QQHYSSPYT | FGGGTKLEIK | SEQ ID NO: 72 |
| Hu621 Vκ4 (Hu621_44) | GVPDRFSGSGSGT DFTLTISSLQAED VAVYYC | QQHYSSPYT | FGQGTKLEIK | SEQ ID NO: 73 |

In addition, modifications of CDR2 sequence in the humanized antibodies (Hu149, Hu428, and Hu621 antibodies) were performed on the heavy chains (heavy chain VH4 for each antibody):

- CDR2 of heavy chain VH4 of the Hu149 antibody was modified to YIYPNNAGTS YNQKFKG (SEQ ID No: 74) to form heavy chain VH7 (SEQ ID No: 75) or to YIYPNKGGTS YNQKFKG (SEQ ID No: 76) to form heavy chain VH8 (SEQ ID No: 77),
- CDR2 of heavy chain VH4 of the Hu428 antibody was modified to YIYPNNAGTS YNQKFKG (SEQ ID No: 78) to form heavy chain VH7 (SEQ ID No: 79) or to YIYPNKGGTS YNQKFKG (SEQ ID No: 80) to form heavy chain VH8 (SEQ ID No: 81), and
- CDR2 of heavy chain VH4 of the Hu621 antibody was modified to YIYPNNAGTS YNQKFKG (SEQ ID No: 82) to form heavy chain VH7 (SEQ ID No: 83) or to YIYPNKGGTS YNQKFKG (SEQ ID No: 84) to form heavy chain VH8 (SEQ ID No: 85) .

For each modified antibody obtained based on the heavy chain of each humanized antibody, based on maintaining equal or higher binding affinities compared to the original mouse antibody, the following antibodies were prepared (see Table 22):

- Hu149_71, Hu149_81, Hu149_73, Hu149_83, Hu149_74, and Hu149_84 antibodies for Hu149 antibody,
- Hu428_71, Hu428_81, Hu428_73, Hu428_83, Hu428_74, and Hu428_84 antibodies for Hu428 antibody,
- Hu621_71, Hu428_81, Hu621_73, Hu428_83, Hu428_74, and Hu428_84 antibodies for Hu621 antibody.

[Table 22]

| Fw (149) | Vκ1 | Vκ3 | Vκ4 |
|---|---|---|---|
| VH4 | Hu149_41 | Hu149_43 | Hu149_44 |
| VH7 (Fw=4, HCDR2 NG→NA) | Hu149_71 | Hu149_73 | Hu149_74 |
| VH8 (Fw=4, HCDR2 NG→KG) | Hu149_81 | Hu149_83 | Hu149_84 |
| | | | |
| Fw (428) | Vκ1 | Vκ3 | Vκ4 |
| VH4 | Hu428_41 | Hu428_43 | Hu428_44 |
| VH7 (Fw=4, HCDR2 NG→NA) | Hu428_71 | Hu428_73 | Hu428_74 |
| VH8 (Fw=4, HCDR2 NG→KG) | Hu428_81 | Hu428_83 | Hu428_84 |
| | | | |
| Fw (621) | Vκ1 | Vκ3 | Vκ4 |
| VH4 | Hu621_41 | Hu621_43 | Hu621_44 |
| VH7 (Fw=4, HCDR2 NG→NA) | Hu621_71 | Hu621_73 | Hu621_74 |

(continued)

| Fw (621) | Vκ1 | Vκ3 | Vκ4 |
|---|---|---|---|
| VH8 (Fw=4, HCDR2 NG→KG) | Hu621_81 | Hu621_83 | Hu621_84 |

The heavy chain sequences of heavy-chain CDR2-modified humanized antibodies are shown in Table 23 (Table 23-1 for the Hu149 series antibodies, Table 23-2 for the 428 series antibodies, and Table 23-3 for the Hu621 series antibodies).

[Table 23-1]

| VH | VR1 | CDR1 | VR2 | CDR2 |
|---|---|---|---|---|
| Hu149 VH7 (Hu149_71, Hu149_73 & Hu149_74) | QVQLVQSGAEVK KPGASVKVSCKAS GYTFT | DYYMD | WVRQAPGQGLE WLG | YIYPNNAGTSYNQ KFKG |
| Hu149 VH8 (Hu149_81, Hu149_83 & Hu149_84) | QVQLVQSGAEVK KPGASVKVSCKAS GYTFT | DYYMD | WVRQAPGQGLE WLG | YIYPNKGGTSYNQ KFKG |

| VH (continued) | VR3 | CDR3 | VR4 | |
|---|---|---|---|---|
| Hu149 VH7 (Hu149_71, Hu149_73 & Hu149_74) | RVTMTTDKSTSTA YMELRSLRSDDTA VYYCAR | SGYGNYYTMDY | WGQGTTVTVSS | SEQ ID NO: 75 |
| Hu149 VH8 (Hu149_81, Hu149_83 & Hu149_84) | RVTMTTDKSTSTA YMELRSLRSDDTA VYYCAR | SGYGNYYTMDY | WGQGTTVTVSS | SEQ ID NO: 77 |

[Table 23-2]

| VH | VR1 | CDR1 | VR2 | CDR2 |
|---|---|---|---|---|
| Hu428 VH7 (Hu428_71, Hu428_73 & Hu428_74) | QVQLVQSGAEVK KPGASVKVSCKAS GYTFT | DYYMD | WVRQAPGQGLE WLG | YIYPNNAGTSYNQ KFKG |
| Hu428 VH8 (Hu428_81, Hu428_83 & Hu428_84) | QVQLVQSGAEVK KPGASVKVSCKAS GYTFT | DYYMD | WVRQAPGQGLE WLG | YIYPNKGGTSYNQ KFKG |

| VH (continued) | VR3 | CDR3 | VR4 | |
|---|---|---|---|---|
| Hu428 VH7 (Hu428_71, Hu428_73 & Hu428_74) | RVTMTTDKSTSTA YMELRSLRSDDTA VYYCAR | GGYYSYYSYDY | WGQGTTVTVSS | SEQ ID NO: 79 |
| Hu428 VH8 (Hu428_81, Hu428_83 & Hu428_84) | RVTMTTDKSTSTA YMELRSLRSDDTA VYYCAR | GGYYSYYSYDY | WGQGTTVTVSS | SEQ ID NO: 81 |

[Table 23-3]

| VH | VR1 | CDR1 | VR2 | CDR2 |
|---|---|---|---|---|
| Hu621 VH7 (Hu621_71, Hu621_73 & Hu621_74) | QVQLVQSGAEVK KPGASVKVSCKAS GYTFT | DYYMD | WVRQAPGQGLE WLG | YIYPNNAGTSYNQ KFKG |
| Hu621 VH8 (Hu621_81, Hu621_83 & Hu621_84) | QVQLVQSGAEVK KPGASVKVSCKAS GYTFT | DYYMD | WVRQAPGQGLE WLG | YIYPNKGGTSYNQ KFKG |

| VH (continued) | VR3 | CDR3 | VR4 | |
|---|---|---|---|---|

| Hu621 VH7 (Hu621_71, Hu621_73 & Hu621_74) | RVTMTTDKSTSTA YMELRSLRSDDTA VYYCAR | SGYKAYYAMDY | WGQGTTVTVSS | SEQ ID NO: 83 |
|---|---|---|---|---|
| Hu621 VH8 (Hu621_81, Hu621_83 & Hu621_84) | RVTMTTDKSTSTA YMELRSLRSDDTA VYYCAR | SGYKAYYAMDY | WGQGTTVTVSS | SEQ ID NO: 85 |

Example 12: Binding Analysis of Humanized Antibody

In this example, experiments were performed with respect to the humanized anti-human Tim-3 antibodies of the invention produced in Example 11 for the purpose of the binding analysis.

(12-1) Binding affinity analysis

First, the humanized antibody (Hu003_13_F20antibody) prepared in Example 11 was used as an antibody for binding analysis to recombinant human Tim-3. The Hu003_13_F20 antibody was prepared by the same method as described in Example 1, except that the sequence of the variable region was that of Hu003_13_F20 as described in Example 11.

The analysis of the binding affinity of these humanized antibodies to human Tim-3 was carried out using the same method as described in Example 3 (3-1), except that the antibodies used were humanized antibodies (Hu003_13_F20_sc antibody and Hu003_13_F20_LA antibody).

The results are shown in Table 24. The humanized antibodies obtained in the present invention have lower KD values compared to the binding affinity of the original monoclonal antibodies (Ch003-LA and Ch003_sc antibodies) to human recombinant Tim-3, confirming their improved affinity.

[Table 24]

|  | KD (M) |
|---|---|
| Hu003_13_F20_LA | 4.71E-09 |
| Hu003_13_F20_sc | 6.13E-09 |
| Ch003-LA | 1.26E-08 |
| Ch003_sc | 1.47E-08 |

Next, the binding affinities of other humanized antibodies produced in Example 11 to recombinant human Tim-3 were analyzed in the same method as described above. Each humanized antibody was produced by the same method as described in Example 1, except that the sequence of the variable region was that of the humanized antibody sequence described in Example 11.

The binding affinities of Hu003_12_sc, Hu003_13_sc, Hu003_22_sc, and Hu003_23_sc antibodies, as Hu003 series antibodies, to human Tim-3 are shown in Table 25. The humanized antibodies obtained in the present invention have comparable KD values compared to the binding affinity of the original monoclonal antibody (Ch003_sc antibody) to human recombinant Tim-3, confirming that the affinity is maintained.

[Table 25]

| < Hu003 series antibodies> | |
|---|---|
|  | KD (M) |
| Ch003_sc | 1.03E-08 |
| Hu003_12_sc | 9.07E-09 |

(continued)

| < Hu003 series antibodies> | |
|---|---|
| | KD (M) |
| Hu003_13_sc | 8.89E-09 |
| Hu003_22_sc | 9.26E-08 |
| Hu003_23_sc | 1.15E-08 |

The binding affinities of the Hu072_11 and Hu072_13 antibodies, as Hu072 series antibodies, to human Tim-3 are shown in Table 26. The humanized antibodies obtained in the present invention have comparable KD values compared to the binding affinity of the original monoclonal antibody (Ch072_sc antibody) to human recombinant Tim-3, confirming that the affinity is maintained.

[Table 26]

| < Hu072 series antibodies> | |
|---|---|
| | KD (M) |
| Ch072_sc | 6.19E-07 |
| Hu072_11_sc | 8.80E-07 |
| Hu072_13_sc | 8.23E-07 |

The binding affinities of Hul49_41_sc, Hu149_43_sc, and Hu149_44_sc antibodies, as Hu149 series antibodies, as well as Hu149_71_sc, Hul49_73_sc, Hu149_74_sc, Hu149_81_sc, Hu149_83_sc, and Hu149_84_sc antibodies, as heavy-chain CDR2-modified Hu149 series antibodies, to human Tim-3 are shown in Table 27. These antibodies have comparable or lower KD values compared to the binding affinity of the original monoclonal antibody (Ch149_sc antibody) to human recombinant Tim-3, confirming that the affinity was maintained and improved.

[Table 27]

| < Hu149 series antibodies> | |
|---|---|
| | KD (M) |
| Hu149_41_sc | n.t. |
| Hu149_43_sc | 3.05E-07 |
| Hu149_44_sc | n.t. |
| Hu149_71_sc | n.t. |
| Hu149_73_sc | 4.31E-07 |
| Hu149_74_sc | n.t. |
| Hu149_81_sc | n.t. |
| Hu149_83_sc | 1.59E-07 |
| Hu149_84_sc | n.t. |
| n.t.: not tested | |

The binding affinities of the Hu428_41_sc, Hu428_43_sc, and Hu428_44_sc antibodies, as Hu428 series antibodies, as well as the Hu428_71_sc, Hu428_73_sc, Hu428_74_sc, Hu428_81_sc, Hu428_83_sc, and Hu428_84_sc antibodies, as heavy-chain CDR2-modified Hu428 series antibodies, to human Tim-3 are shown in Table 28. These antibodies have almost equivalent KD values compared to the binding affinity of the original monoclonal antibody (Ch428_sc antibody) to human recombinant Tim-3, confirming that the affinity is maintained.

54

[Table 28]

| < Hu428 series antibodies> | |
|---|---|
| | KD (M) |
| Hu428_41_sc | n.t. |
| Hu428_43_sc | 3.51E-08 |
| Hu428_44_sc | 4.84E-08 |
| Hu428_71_sc | n.t. |
| Hu428_73_sc | 3.92E-08 |
| Hu428_74_sc | 5.01E-08 |
| Hu428_81_sc | n.t. |
| Hu428_83_sc | 1.02E-08 |
| Hu428_84_sc | 1.54E-08 |
| n.t.: not tested | |

The binding affinities of Hu621_41_sc, Hu621_43_sc, and Hu621_44_sc antibodies, as Hu621 series antibodies, as well as Hu621_71_sc, Hu621_73_sc, Hu621_74_sc, Hu621_81_sc, Hu621_83_sc, and Hu621_84_sc antibodies, as heavy-chain CDR2-modified Hu621 series antibodies, to human Tim-3 are shown in Table 29. These antibodies have lower KD values compared to the binding affinity of the original monoclonal antibody (Ch621_sc antibody) to human recombinant Tim-3, confirming the improved affinity.

[Table 29]

| < Hu621 series antibodies> | |
|---|---|
| | KD (M) |
| Hu621_41_sc | n.t. |
| Hu621_43_sc | 8.21E-08 |
| Hu621_44_sc | n.t. |
| Hu621_71_sc | n.t. |
| Hu621_73_sc | 8.33E-08 |
| Hu621_74_sc | n.t. |
| Hu621_81_sc | n.t. |
| Hu621_83_sc | 4.91E-08 |
| Hu621_84_sc | n.t. |
| n.t.: not tested | |

(12-2) Binding analysis to Human Tim-3.

Next, the binding analysis was performed on the humanized anti-human Tim-3 antibodies of the invention (Hu003_13_F20, Hu149_83, Hu428_83, and Hu621_83 antibodies) produced in Example 11 using Jurkat cells stably expressing human Tim-3 or Expi293 cells transiently expressing human Tim-3.

The analysis of the binding affinity of these humanized antibodies to human Tim-3 was performed using the same method as described in Example 3 (3-2), except that the antibodies used were the humanized antibodies (Hu003_13_F20_sc antibody, Hu003_13_F20_LA antibody, Hu149_83_sc antibody, Hu428_83_sc antibody, and Hu621_83_sc antibody). The Ch003_sc antibody was used as a control antibody for Hu003_13_F20_sc antibody, Ch003_LA antibody was used as a control antibody for Hu003_13_F20_LA antibody, and an isotype control antibody was used as a negative control. In addition, for Hu149_83 sc, Hu428_83_sc, and Hu621_83_sc antibodies, Hu003_13_F20_sc antibody was used as a control to confirm that it showed similar results of Hu003_13_F20_sc and Hu003_13_F20_LA antibodies obtained at different date and time, and an isotype control antibody as a negative control.

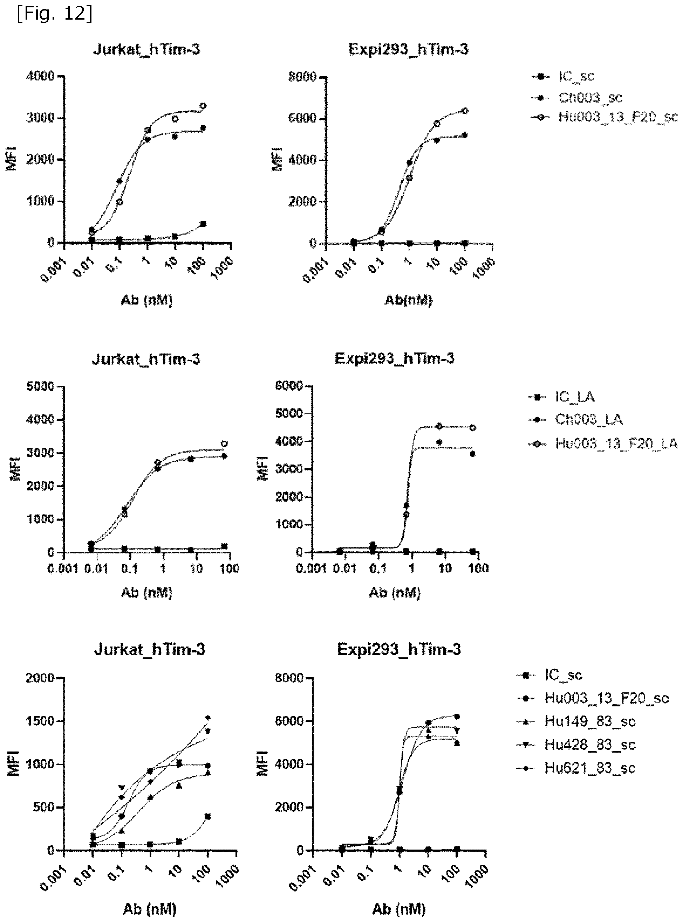

The results of the flow cytometry analysis are shown in Figure 12 (upper and middle rows of Figure 12 for Hu003_13_F20_sc and Hu003_13_F20_LA antibodies; lower row of Figure 12 for Hu149_83_sc, Hu428_83_sc, and Hu621_83_sc antibodies),the dissociation constant KD values (nM) for Hu003_13_F20_sc and Hu003_13_F20_LA antibodies calculated based on the data are shown in Table 30-1, and the dissociation constant KD values (nM) for Hul49_83_sc, Hu428_83_sc, and Hu621_83_sc antibodies are shown in Table 30-2. The humanized antibodies of this invention specifically bound to human Tim-3 expressed on the cell membrane at the similar level to the monoclonal antibodies used as a control. The results confirm the successful production of the humanized antibodies.

[Table 30-1]

| Ab | Jurkat cells expressing human Tim-3 | Expi293 cells expressing human Tim-3 |
|---|---|---|
| | KD (nM) | KD (nM) |
| Hu003_13_F20_sc | 0.2147 | 1.033 |
| Hu003_13_F20_LA | 0.1051 | 1.485 |
| Ch003_sc | 0.08394 | 0.4271 |
| Ch003_LA | 0.08284 | 0.8914 |

[Table 30-2]

| Ab | Jurkat cells expressing human Tim-3 | Expi293 cells expressing human Tim-3 |
|---|---|---|
| | KD (nM) | KD (nM) |
| Hu003_13_F20_sc | 0.1693 | 1.422 |
| Hu149_83_sc | 0.3375 | 0.982 |
| Hu428_83_sc | 0.0470 | 1.120 |
| Hu621_83_sc | 0.0568 | 0.897 |

(12-3) Furthermore, the binding of the humanized anti-human Tim-3 antibodies of the invention (Hu003_13_F20, Hu149_83, Hu428_83, and Hu621_83 antibodies) prepared in Example 11 to human Tim-3 on activated T cell membranes was analyzed by flow cytometry.

The analysis of the binding affinity of these humanized antibodies to human Tim-3 was performed in the same method as described in Example 3 (3-3), except that the antibodies used were the humanized anti-human Tim-3 antibodies of the invention produced in Example 11 (Hu003_13_F20_sc antibody, Hu003_13_F20_LA antibody, Hu149_83_sc antibody, Hu428_83_sc antibody and Hu621_83_sc antibodies). The Ch003_sc antibody was used as a control for Hu003_13_F20_sc antibody, the Ch003_LA antibody was used as a control for Hu003_13_F20_LA antibody, and an isotype control antibody was used as a negative control. In addition, for Hu149_83_sc antibody, Hu428_83_sc antibody, and Hu621_83_sc antibody, Hu003_13_F20_sc antibody was used as a control to confirm that it showed similar results of Hu003_13_F20_sc and Hu003_13_F20_LA antibodies obtained at different date and time, and an isotype control antibody was used as a negative control.

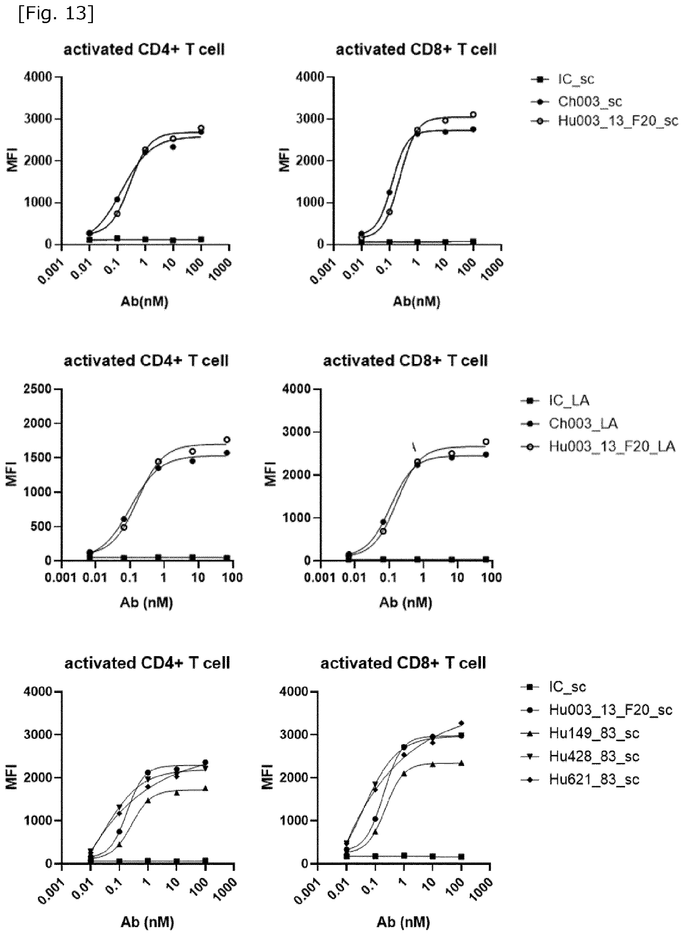

The results of the flow cytometry analysis are shown in Figure 13 (upper and middle rows of Figure 13 for Hu003_13_F20_sc and Hu003_13_F20_LA antibodies, and lower row of Figure 13 for Hu149_83_sc, Hu428_83_sc, and Hu621_83_sc antibodies), the dissociation constant KD values(nM) of Hu003_13_F20_sc and Hu003_13_F20_LA antibodies calculated based on the data are shown in Table 31-1, and the dissociation constant KD values(nM) of Hu149_83 sc, Hu428_83_sc, and Hu621_83_sc antibodies Table 31-2. The humanized antibody of the present invention specifically bound to human Tim-3 expressed on primary T cells, similar to the monoclonal antibody of the present invention, which is used as a control.

[Table 31-1]

| Ab | Activated CD4 T cells | Activated CD8 T cells |
| --- | --- | --- |
|  | KD (nM) | KD (nM) |
| Hu003_13_F20_sc | 0.2644 | 0.2546 |
| Hu003_13_F20_LA | 0.1601 | 0.1639 |
| Ch003_sc | 0.1397 | 0.1254 |
| Ch003_LA | 0.1052 | 0.1122 |

[Table31-2]

| Ab | Activated CD4 T cells | Activated CD8 T cells |
| --- | --- | --- |
|  | KD (nM) | KD (nM) |
| Hu003_13_F20_sc | 0.1960 | 0.1952 |
| Hu149_83_sc | 0.2679 | 0.2261 |
| Hu428_83_sc | 0.0661 | 0.0634 |
| Hu621_83_sc | 0.0845 | 0.0735 |

Example 13: Inhibitory activity on Ligand binding of humanized antibody

In this example, experiments were performed with respect to the humanized anti-human Tim-3 antibody of the invention produced in Example 11 for the purpose of functional analysis.

(13-1) Galectin-9 binding inhibition

The humanized anti-human Tim-3 antibodies (Hu003_13_F20, Hu149_83, Hu428_83, and Hu621_83 antibodies) produced in Example 11 were analyzed for their inhibitory activity against the binding between human Tim-3 and human Galectin-9 using ELISA.

The inhibitory activity of these humanized antibodies to the binding between human Tim-3 and human Galectin-9 was confirmed using the same method as described in Example 4 (4-1), except that the antibodies used were the humanized antibodies (Hu003_13_F20_sc antibody, Hu003_13_F20_LA antibody, Hu149_83_sc antibody, Hu428_83_sc antibody, and Hu621_83_sc antibody). The Ch003_sc antibody was used as a control for Hu003_13_F20_sc antibody, the Ch003_LA antibody was used as a control for Hu003_13_F20_LA antibody, and an isotype control antibody was used as a negative control. In addition, for Hu149_83_sc antibody, Hu428_83_sc antibody, and Hu621_83_sc antibody, Hu003_13_F20_sc antibody was used as a control to confirm that it showed similar results of Hu003_13_F20_sc and Hu003_13_F20_LA antibodies obtained at different date and time, and an isotype control antibody was used as the negative control.

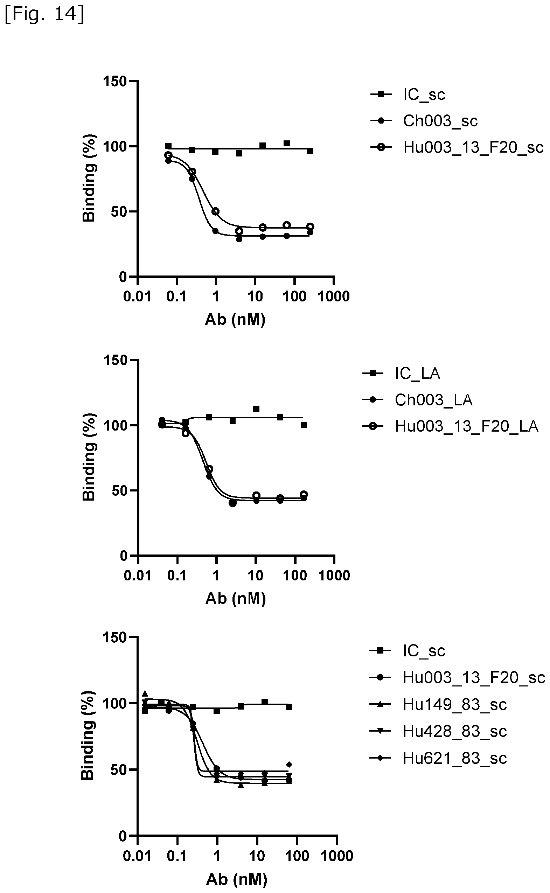

The results of binding (%) of human Tim-3-Fc antigen to human Galectin-9 are shown in Figure 14. The humanized antibodies produced in the present invention were able to inhibit the binding between human Tim-3 and human Galectin-9 to the same extent as the monoclonal antibodies of the present invention, which are the origin of each humanized antibody (the maximum inhibition rate was 71.2% for Ch003_sc antibody, 65.1% for Hu003_13_F20_sc antibody, 58.8% for Ch003_LA antibody, 59.6% for Hu003_13_F20_LA_sc antibody, 61.5% for Hu149_83_sc antibody, 56.8% for Hu428_83_sc antibody, and 53.1% for Hu621_83_sc antibody).

(13-2) Phosphatidylserine binding inhibition

Next, the humanized anti-human Tim-3 antibodies of this invention (Hu003_13_F20 antibody, Hu149_83 antibody, Hu428_83 antibody, and Hu621_83 antibody) produced in Example 11 were analyzed for inhibitory activity against binding between human Tim-3 and Phosphatidylserine using flow cytometry.

The inhibitory activity of these humanized antibodies to the binding between human Tim-3 and Phosphatidylserine was confirmed using the same method as described in Example 4 (4-2), except that the antibodies used were the humanized antibodies (Hu003_13_F20_sc antibody, Hu003_13_F20_LA antibody, Hu149_83_sc antibody, Hu428_83_sc antibody, and Hu621_83_sc antibody). The Ch003_sc antibody was used as a control for Hu003_13_F20_sc antibody, the Ch003_LA antibody was used as a control for Hu003_13_F20_LA antibody, and an isotype control antibody was used as a negative control. In addition, for Hu149_83_sc antibody, Hu428_83_sc antibody, and Hu621_83_sc antibody, Hu003_13_F20_sc antibody was used as a control to confirm that it showed similar results of Hu003_13_F20_sc and Hu003_13_F20_LA antibodies obtained at different date and time, and an isotype control antibody was used as the negative control.

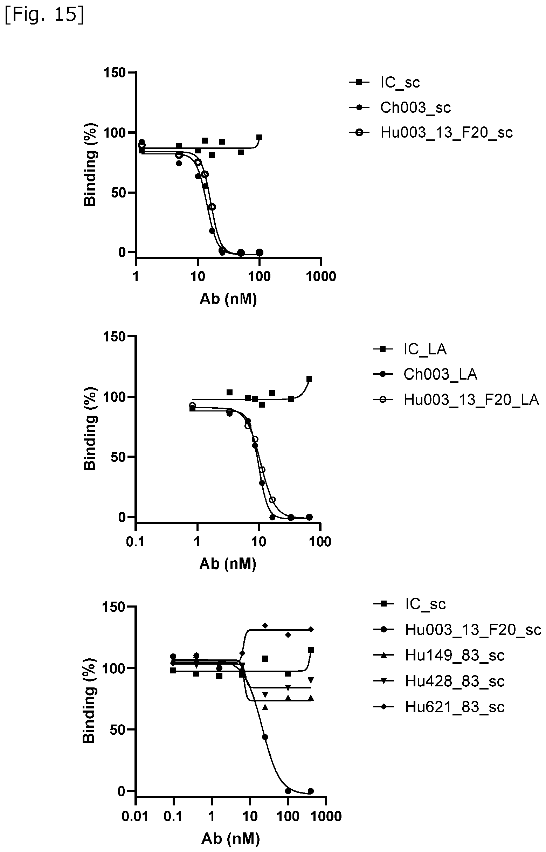

The results of the flow cytometry analysis are shown in Figure 15, the $IC_{50}$ values (nM) for Hu003_13_F20_sc and Hu003_13_F20_LA antibodies calculated based on the analysis data are shown in Table 32-1, and the $IC_{50}$ values (nM) for Hu149_83_sc antibody, Hu428_83_sc antibody, and Hu621_83_sc antibody are shown in Table 32-2. The humanized antibodies produced in the present invention were able to inhibit the binding between human Tim-3 and phosphatidylserine to the same level as the monoclonal antibodies of this invention, which are the origin of each humanized antibody.

[Table 32-1]

| Ab | IC50 (nM) |
| --- | --- |
| Hu003_13_F20_sc | 16.29 |
| Hu003_13_F20_LA | 10.73 |
| Ch003_sc | 14.04 |
| Ch003_LA | 9.909 |

[Table 32-2]

| Ab | IC50 (nM) |
| --- | --- |
| Hu003_13_F20_sc | 20.88 |
| Hu149_83_sc | Weak inhibition |
| Hu428_83_sc | Weak inhibition |
| Hu621_83_sc | No inhibition |

Example 14: Peripheral blood mononuclear cell (PBMC) stimulation assay with Staphylococcus aureus enterotoxin B (SEB)

In this example, the effect of the humanized anti-human Tim-3 antibodies of the present invention, produced in Example 11, on the activation of human peripheral blood T cells was evaluated based on the activation of peripheral blood mononuclear cells (PBMC) induced by Staphylococcal enterotoxin B (SEB).

The experiments were performed using the same method as described in Example 8, except that the antibodies used were the humanized anti-human Tim-3 antibody of the present invention produced in Example 11. The Ch003_sc antibody was used as a control, an isotype control antibody was used as a negative control, and an antibody specified in US9605070B2 (ABTIM3-hum21 VH: Seq No.26/VL: Seq No.20) was used as a reference antibody.

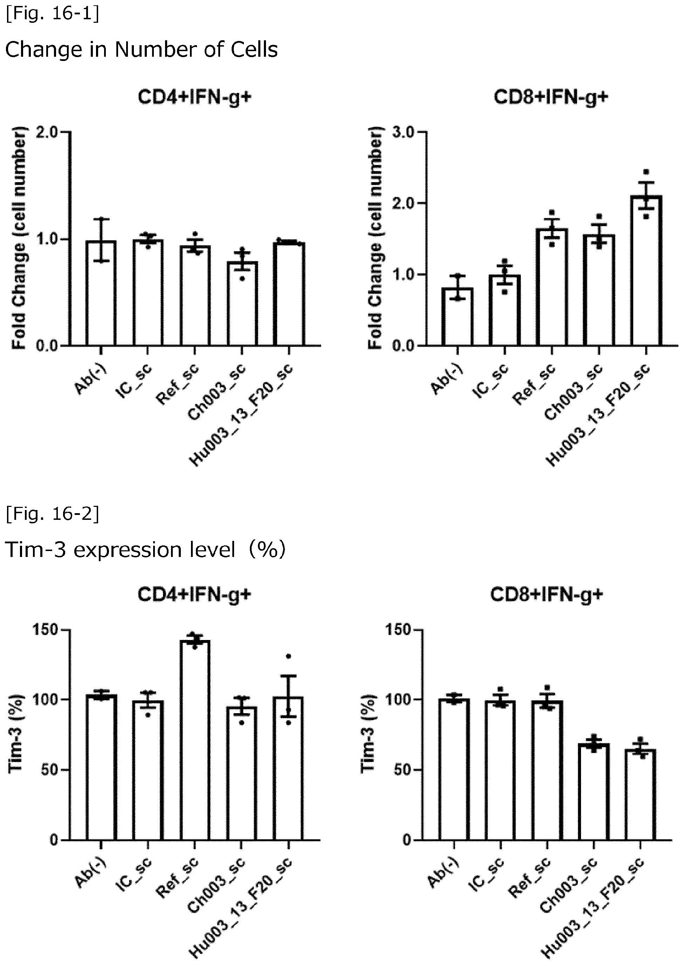

The results of flow cytometry analysis are shown in Figure 16. Figure 16-1 reveals the changes in the number of CD4-positive IFN-γ-positive cells and that of CD8-positive IFN-γ-positive cells, and Figure 16-2 reveals the percentage of Tim-3 expression level on the cell surface (MFI value) of CD4-positive IFNγ-positive cells and CD8-positive IFNγ-positive cells, with the MFI value of the isotype control being considered as 100% and the MFI value of the staining negative control being considered as 0%. The humanized anti-human Tim-3 antibodies produced in this invention increased the number of CD8-positive IFN-γ-positive cells. This effect was significantly higher than the reference antibody used as a positive control. Furthermore, the humanized anti-human Tim-3 antibodies in this invention reduced the expression level of human Tim-3 on CD8-positive IFN-γ-positive cells. The features of the antibodies were not observed by the reference antibody used as a positive control. The humanized anti-human Tim-3 antibodies produced in this invention inhibit the binding between human Tim-3 and human Galectin-9 and the binding between human Tim-3 and Phosphatidylserine, thereby contributing to the activation of effector T cells.

Example 15: Preparation of Anti-Human Tim-3 Biparatopic Antibody Using Humanized Antibody.

In this example, biparatopic antibodies that bind to human Tim-3 were prepared based on the humanized monoclonal antibodies produced in Example 11 and verified in Example 12 and Example 13.

Among the antibodies produced in Example 11, biparatopic antibodies were produced using half-molecules from Hu003 series antibodies and Hu072 series antibodies belonging to Group 2, and half-molecules from Hu149 series, Hu428 series, and Hu621 series antibodies belonging to Group 5, which do not compete with each other in binding to Tim-3. Specifically, using Hu003_13_F20 antibody as one half-molecule, and selecting Hu149_83 antibody, Hu428_83 antibody, or Hu621_83 antibody as the other half-molecule, these biparatopic antibodies were produced using the Knob-into-Hole technology (US7183076B2).

Specifically, for the half-molecule of Hu003_13_F20 antibody belonging to Group 2, the Fc domain was modified to be a Knob-type, and a C-tag was added to the C-terminus of the antibody. For the half-molecules of Hu149_83 antibody, Hu428_83 antibody, and Hu621_83 antibody belonging to Group 5, the Fc domain was modified to be a Hole-type, and two different types of DNA encoding these antibodies were inserted into the vector. In other words, expression vectors were prepared for the following combinations:

- a combination of DNA encoding Hu003_13_F20 antibody and DNA encoding Hu149_83 antibody
- a combination of DNA encoding Hu003_13_F20 antibody and DNA encoding Hu428_83 antibody
- a combination of DNA encoding Hu003_13_F20 antibody and DNA encoding Hu621_83 antibody.

Each of these expression vectors was transfected into ExpiCHO cells (Thermo Fisher Scientific) to express and produce the biparatopic antibodies as heterodimers of scFv-Fc. These biparatopic antibodies were directly purified using an anti-C-tag column.

As a result, Hu149003ct antibody (a combination of a half-molecule of Hu149_83 antibody and a half molecule of Hu003_13_F20 antibody), Hu428003ct antibody (a combination of a half-molecule of Hu428_83 antibody and a half molecule of Hu003_13_F20 antibody), and Hu621003ct antibody (a combination of a half-molecule of Hu621_83 antibody and a half molecule of Hu003_13_F20 antibody) were obtained.

Example 16: Binding Analysis of Biparatopic Antibodies Derived from Humanized Antibodies

In this example, experiments were conducted with respect to the biparatopic antibodies, which were produced in Example 15, for the purpose of the binding analysis.

(16-1) Binding Affinity Analysis

First, the humanized biparatopic antibodies (Hu149003ct antibody, Hu428003ct antibody, and Hu621003ct antibody) produced in Example 15 were used as antibodies for binding analysis to recombinant human Tim-3.

The analysis of binding affinity of the humanized biparatopic antibodies to human Tim-3 was conducted using the same method as described in Example 3 (3-1), except that the antibodies used were the humanized biparatopic antibodies (Hu149003ct antibody, Hu428003ct antibody, and Hu621003ct antibody).

The results are shown in Table 33. The humanized biparatopic antibodies of the present invention exhibited improved binding affinity, compared to the original humanized monoclonal antibodies (Example 12, Table 24-29). This improvement is considered to be exhibited due to an avidity effect because the biparatopic antibodies can bind to the analyte at two different sites, and this confirms the successful production of biparatopic antibodies.

[Table 33]

| biparatopic antibody | KD (M) |
|---|---|
| Hu149003ct | 7.34E-10 |
| Hu428003ct | 3.63E-10 |
| Hu621003ct | 3.19E-10 |

(16-2) Binding analysis to Human Tim-3

Next, the binding analysis was performed on the humanized anti-human Tim-3 biparatopic antibodies of the present invention produced in Example 15 (Hu149003ct antibody, Hu428003ct antibody, and Hu621003ct antibody), using Jurkat cells stably expressing human Tim-3 or Expi293 cells transiently expressing human Tim-3.

The analysis of the binding affinity of these humanized biparatopic antibodies to human Tim-3 was performed using the same method as described in Example 3 (3-2), except that the antibodies used were the humanized biparatopic antibodies (Hu149003ct antibody, Hu428003ct antibody, and Hu621003ct antibody). Hu003_13_F20_sc antibody, from which the half-molecules of these humanized biparatopic antibodies are originated and which was used for binding analysis in Example 12 (12-2), was used as a control antibody.

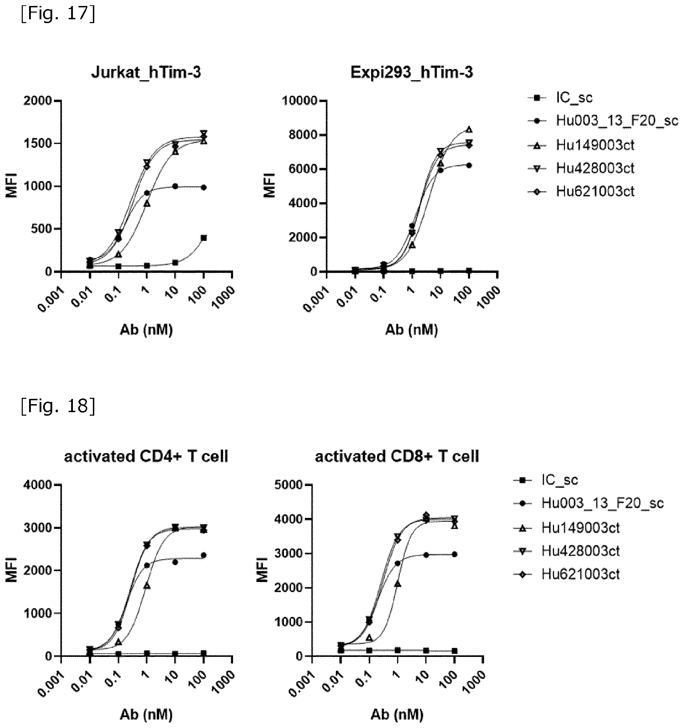

The results of flow cytometry analysis are shown in Figure 17, and the dissociation constants KD values (nM) are shown in Table 34. The humanized biparatopic antibodies of the present invention specifically bound to human Tim-3 expressed on the cell membrane, similar to the humanized monoclonal antibodies of Example 12. Additionally, the humanized biparatopic antibodies exhibited increased MFI of saturation binding compared to the original humanized monoclonal antibodies. This increase is considered to be exhibited because one molecule of the biparatopic antibody binds one molecule of the antigen on the cell membrane, while one molecule of the humanized monoclonal antibody molecule binds to two molecules of the antigen on the cell membrane. Similar to the results in Example 16 (16-1), these results confirm the successful production of the humanized biparatopic antibodies.

[Table 34]

| Ab | Jurkat cells expressing human Tim-3 | Expi293 cells expressing human Tim-3 |
|---|---|---|
| | **KD (nM)** | **KD (nM)** |
| Hu003_13_F20_sc | 0.1693 | 1.422 |
| Hu149003ct | 0.9041 | 4.161 |
| Hu428003ct | 0.2321 | 2.301 |
| Hu621003ct | 0.2827 | 2.270 |

(16-3) Furthermore, the binding of the humanized anti-human Tim-3 biparatopic antibodies of the present invention (Hu149003ct antibody, Hu428003ct antibody, and Hu621003ct antibody) to human Tim-3 on the membrane of activated T cells was analyzed using flow cytometry.

The analysis of the binding affinity of these humanized biparatopic antibodies to human Tim-3 was performed using the same method as described in Example 3 (3-3), except that the antibodies used were the humanized biparatopic antibodies (Hu149003ct antibody, Hu428003ct antibody, and Hu621003ct antibody). Hu003_13_F20_sc antibody, which is derived from half-molecules of these biparatopic antibodies and was used for binding analysis in Example 12 (12-3), was used as a control antibody.

The results of the flow cytometry analysis are shown in Figure 18, and the dissociation constant KD values (nM) for each antibody calculated based on the data are shown in Table 35. The humanized biparatopic antibodies of the present invention specifically bound to human Tim-3 expressed on primary T cells, similar to the humanized monoclonal antibodies of the present invention that were shown to bind in Example 12. Furthermore, the humanized biparatopic antibody also exhibited an increased MFI of saturation binding, compared to the original humanized monoclonal antibody. These results indicate that the humanized biparatopic antibodies produced in the present invention are also functional in primary cells.

[Table 35]

| Ab | Activated CD4 T cells | Activated CD8 T cells |
|---|---|---|
| | **KD (nM)** | **KD (nM)** |
| Hu003_13_F20_sc | 0.1960 | 0.1952 |
| Hu149003ct | 0.9168 | 0.9699 |
| Hu428003ct | 0.2789 | 0.2628 |
| Hu621003ct | 0.2970 | 0.3053 |

Example 17: Inhibitory Activity on Ligand Binding of Humanized Biparatopic Antibodies

In this example, experiments were conducted with respect to the humanized biparatopic antibodies generated in Example 15 for the purpose of functional analysis.

(17-1) Galectin-9 binding inhibition

The humanized anti-human Tim-3 biparatopic antibodies (Hu149003ct, Hu428003ct, and Hu621003ct antibodies) produced in Example 15 were analyzed for inhibitory activity against the binding between human Tim-3 and human Galectin-9 by ELISA.

The inhibitory activity of these humanized biparatopic antibodies to the binding between human Tim-3 and human Galectin-9 was confirmed using the same method as described in Example 4 (4-1), except that the antibodies used were the humanized biparatopic antibodies (Hu149003ct antibody, Hu428003ct antibody, and Hu621003ct antibody). The Hu003_13_F20_sc antibody, which is derived from half-molecules of these biparatopic antibodies and was used for binding analysis in Example 12 (12-2) and Galectin-9 binding inhibition evaluation in Example 13 (13-1), was used as a control antibody.

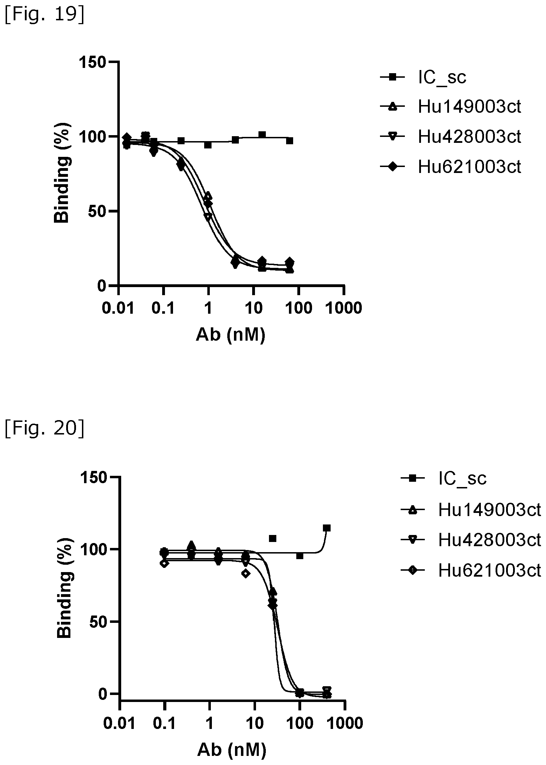

The results of binding (%) of human Tim-3-Fc antigen to human Galectin-9 are shown in Figure 19, and the $IC_{50}$ values (nM) for each humanized biparatopic antibody calculated based on this analysis are shown in Table 36. The humanized biparatopic antibodies produced in this invention were able to inhibit the binding between human Tim-3 and human Galectin-9 by 90% and above. These results indicate that the humanized biparatopic antibodies produced in this invention exhibit stronger inhibitory activity compared to the humanized monoclonal antibodies produced in Example 11 and are equivalent inhibitory activity the binding between human Tim-3 and human Galectin-9 compared to the chimera biparatopic antibodies demonstrated in Example 7 (7-1).

[Table 36]

| Ab. ID | $IC_{50}$ (nM) |
|---|---|
| Hu149003ct | 1.1200 |
| HU428003ct | 0.7118 |
| Hu621003ct | 0.8192 |

(17-2) Phosphatidylserine binding inhibition

Next, the humanized anti-human Tim-3 biparatopic antibodies produced in Example 15 (Hu149003ct antibody, Hu428003ct antibody, and Hu621003ct antibody) were analyzed for inhibitory activity against the binding between human Tim-3 and Phosphatidylserine was analyzed using flow cytometry.

The inhibitory activity of the humanized biparatopic antibodies to the binding between human Tim-3 and Phosphatidylserine was confirmed using the same method as described in Example 4 (4-2), except that the antibodies used were the humanized biparatopic antibodies (Hu149003ct antibody, Hu428003ct antibody, and Hu621003ct antibody). The Hu003_13_F20_sc antibody, which is derived from half-molecules of these biparatopic antibodies and was used for binding analysis in Example 12 (12-2) and phosphatidylserine binding inhibition evaluation in Example 13 (13-2), was used as a control antibody.

The results of flow cytometry analysis are shown in Figure 20, and the $IC_{50}$ values (nM) for each antibody calculated based on the analysis data are shown in Table 37. The humanized biparatopic antibodies of the present invention exhibited an approximately two-fold increase in $IC_{50}$ values, compared to the Hu003_13_F20_sc antibody as demonstrated in Example 13 (13-2), while they were able to almost completely inhibit the binding between human Tim-3 and Phosphatidylserine, similar to the chimeric biparatopic antibodies shown in Example 7 (7-2).

[Table 37]

| Ab. ID | IC50 (nM) |
|---|---|
| Hu149003ct | 32.31 |
| Hu428003ct | 27.30 |
| Hu621003ct | 32.31 |

From the results of Example 17 (17-1) and (17-2), it has been demonstrated that the humanized biparatopic antibodies produced in the present invention are antibodies that strongly inhibit the binding between human Tim-3 and human Galectin-9 and between human Tim-3 and Phosphatidylserine, which are the ligands for human Tim-3, simultaneously.

Example 18: Peripheral Blood Mononuclear Cells (PBMC) Stimulation Assay with Staphylococcal Enterotoxin B (SEB) using Humanized Antibodies

In this example, the effect of the humanized anti-human Tim-3 monoclonal antibodies produced in Example 11 and the humanized anti-human Tim-3 biparatopic antibodies produced in Example 15 on the activation of peripheral blood T cells was evaluated based on the activation of peripheral blood mononuclear cell (PBMC) induced by Staphylococcal Enterotoxin B (SEB).

The experiments were performed using the same method as described in Example 8, except that the antibodies used were the humanized anti-human Tim-3 monoclonal antibodies produced in Example 11 (Hu003_13_F20_sc antibody, Hu149_83_sc antibody, Hu428_83_sc antibody, and Hu621_83_sc antibody) and the humanized anti-human Tim-3 biparatopic antibodies produced in Example 15 (Hu149003ct antibody, Hu428003ct antibody, and Hu621003ct antibody). An isotype control antibody was used as a negative control.

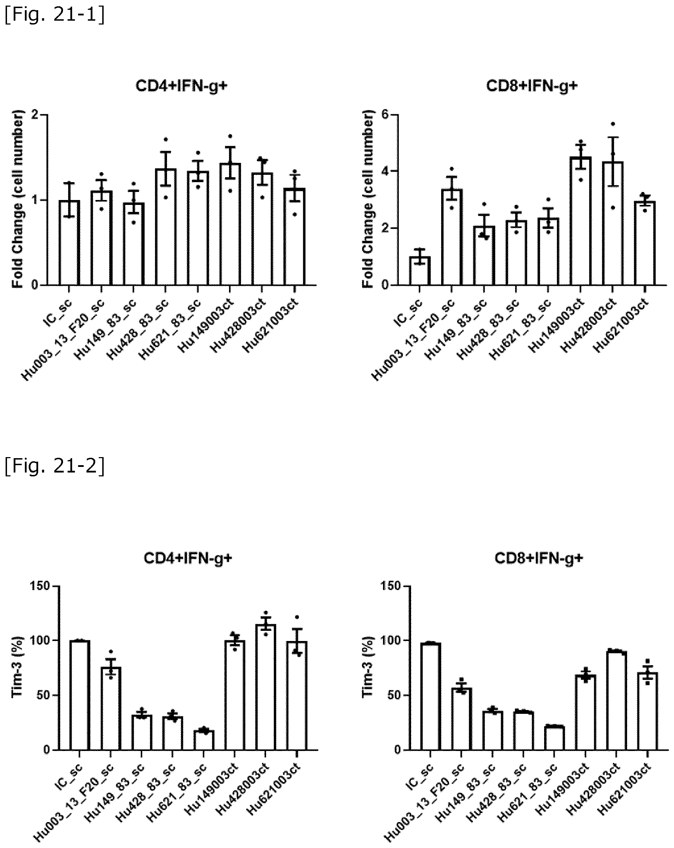

The results of flow cytometry analysis are shown in Figure 21. Figure 21-1 reveals the changes in the number of CD4-positive IFNγ-positive cells and that of CD8-positive IFNγ-positive cells, and Figure 21-2 reveals the Tim-3 expression level (MFI value) on the cell surface of CD4-positive IFNγ-positive cells and CD8-positive IFNγ-positive cells, with the MFI value of the isotype control being considered 100% and the MFI value of the staining negative control being considered 0%.

The humanized anti-human Tim-3 monoclonal antibodies produced in the present invention increased the number of CD8-positive IFN-γ-positive cells, and the effect was further enhanced by the humanized anti-human Tim-3 biparatopic antibodies. Thus, both the humanized anti-human Tim-3 monoclonal antibodies (Hu003_13_F20_sc antibody, Hu149_83_sc antibody, Hu428_83_sc antibody, and Hu621_83_sc antibody) and the humanized anti-human Tim-3 biparatopic antibodies (Hu149003ct antibody, Hu428003ct antibody, and Hu621003ct antibody) produced in the present invention have been demonstrated to enhance the activation of effector T cells against human Tim-3, similar to the chimeric monoclonal antibodies produced in Example 2 and the chimeric biparatopic antibodies produced in Example 5.

Example 19: Evaluation of inhibition of T cell apoptosis induction by Galectin-9

In this example, the humanized anti-human Tim-3 biparatopic antibodies of the present invention were evaluated for their ability to inhibit the induction of apoptosis in human peripheral blood T cells by Galectin-9.

The experiments were performed by the same method as described in Example 9, except that the antibodies used were the humanized anti-human Tim-3 monoclonal antibody (Hu003_13_F20_sc antibody) prepared in Example 11 and the humanized anti-human Tim-3 biparatopic antibodies (Hu149003ct, Hu428003ct, and Hu621003ct antibodies) prepared in Example 15 and Galectin-9 was added at the concentration of 125 nM. An isotype control antibody was used as a negative control. The chimeric anti-human Tim-3 monoclonal antibodies produced in Example 1 (Ch003_sc antibody) and the chimeric anti-human Tim-3 biparatopic antibodies produced in Example 5 (Ch149003ct antibody, Ch428003ct antibody, and Ch621003ct antibody) were used under the same conditions as that using the above described biparatopic antibodies.

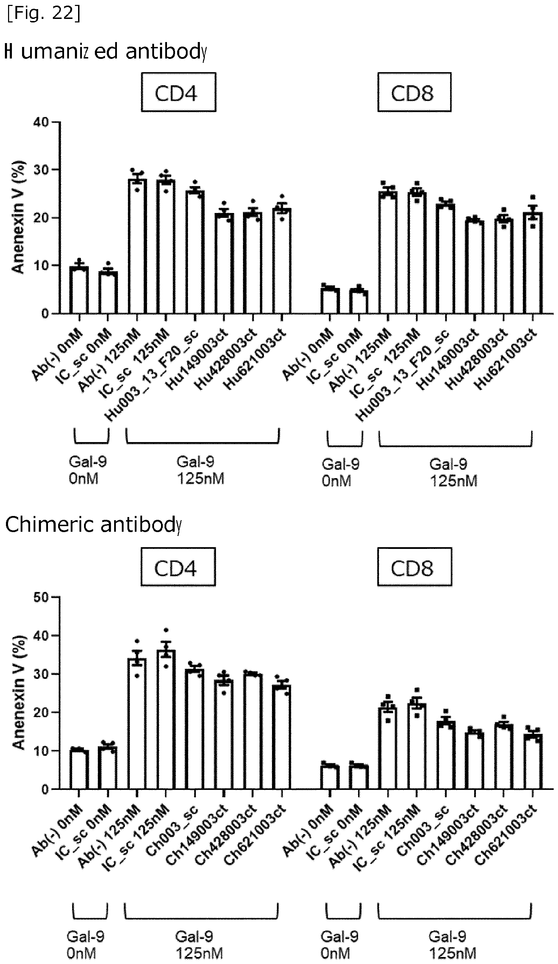

The results are shown in Figure 22. The humanized anti-human Tim-3 monoclonal antibodies produced in the present invention (Hu003_13_F20_sc antibody) slightly inhibited apoptosis induction of T cells by Galectin-9. This effect was further enhanced with the humanized biparatopic antibodies (Hu149003ct, Hu428003ct, and Hu621003ct antibodies) produced in Example 15. Similar results were observed for the chimeric antibodies, with the chimeric anti-human Tim-3 monoclonal antibody (Ch003_sc antibody) slightly inhibiting apoptosis induction of T cells by Galectin-9, while the chimeric anti-human Tim-3 biparatopic antibodies (Ch149003ct antibody, Ch428003ct antibody, and Ch621003ct antibody) exhibiting a stronger effect.

The humanized biparatopic antibodies, similar to the chimeric biparatopic antibodies, was confirmed to suppress apoptosis induction signals of T cells by enhancing the inhibition of the binding between human Tim-3 and human Galectin-9.

Example 20: Evaluation of Effects on T Cell Responses Specific for CMV Peptide Antigen

This example evaluated the effect of the anti-human Tim-3 biparatopic antibody on T cell responses specific for CMV peptide antigen.

In this example, as subject antibodies, Hu149003ct, Hu428003ct, and Hu621003ct were used as biparatopic antibodies, while Hu003_13_F20_sc and Hul49_83_sc, from which the half-molecules of these biparatopic antibodies are originated, and a combination of these two types of antibodies were used for as control antibodies.

Peripheral blood mononuclear cells (PBMC) from healthy human volunteers with HLA type HLA-A02 were suspended in RPMI-1640 medium supplemented with 10% FCS, 100 units/mL penicillin, 100 μg (microgram)/mL streptomycin, 1× GlutaMax (Gibco, 35050-061), and 10 mM HEPES, and these cells were seeded into 96-well U-bottom plates (at a density of 2-5×10$^5$ cells/well), to which the subject antibodies were added at the final concentration of 10 μg (microgram)/mL. After incubating at 37°C with 5% CO$_2$ for 30 minutes, HLA-specific CMV antigen peptides (HLA-A02: NLVPMVATV, HLA-DRB1*11: EHPTFTSQYRIQGKL, HLA-DQB1*06: LLQTGIHVRVSQPSL) were added at a concentration of 1 μg (microgram)/mL each and incubated for 6 days under 37°C with 5% CO$_2$.

Following the incubation, cells were stained using HLA-A*02:01 CMV pp65 Tetramer-NLVPMVATV-PE (MBL, TS-0010-1C) and T-cell marker staining antibodies (Biolegend, anti-CD3: UCHT1, anti-CD4: RPA-T4, anti-CD8a: HIT8a). The stained cells were subjected to Flow cytometry analysis, and the percentage of CMV antigen-specific T cells in the CD8-positive cells was calculated. Additionally, the concentration of IFNγ and TNFα in the collected culture supernatant was quantified using the Luminex method.

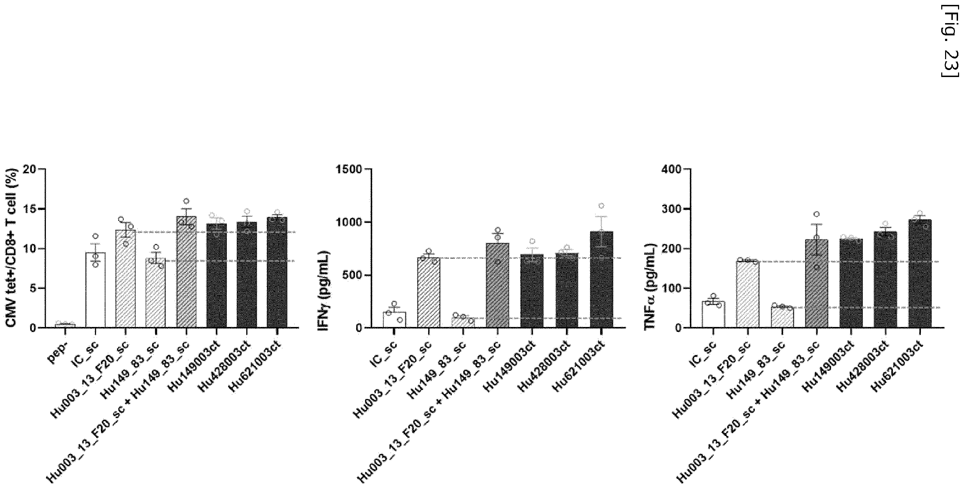

The results of the percentage of T cells specific for CMV antigen in the CD8-positive cells and the concentration of IFNγ and TNFα in the culture supernatant, as measured by Luminex, are shown in Figure 23.

The humanized anti-human Tim-3 monoclonal antibody, Hu003_13_F20_sc, which was evaluated as a control antibody, increased the percentage of T cells specific for CMV antigen and the concentration of IFNγ and TNFα in the culture supernatant, compared to the group with an isotype control antibody, while Hu149_sc showed a similar effect to the isotype control antibody. In the case of the group where both Hu003_13_F20_sc and Hu149_83_sc were added, the percentage of CMV antigen-specific T cells and the concentration of IFNγ and TNFα in the culture supernatant were further increased, compared to the group where Hu003_13_F20_sc was only added.

Hu149003ct, Hu428003ct, and Hu621003ct showed increases in the percentage of CMV antigen-specific T cells and the concentration of IFNγ and TNFα in the culture supernatant that were equal to or greater than those of the group where Hu003_13_F20_sc was only added, similar to the group where both monoclonal antibodies were added simultaneously.

Example 21: Evaluation of the effect of antigen-specific T cell responses and antitumor activity by endogenous antigens

In this example, the effect of the anti-human Tim-3 biparatopic antibodies on the activation of PBMC by endogenous antigens presented on HLA on the cell surface and their antitumor activity were evaluated using a co-culture system of PBMC and tumor cells expressing CMV antigens.

The subject antibodies used in this example were the anti-human Tim-3 biparatopic antibody Ch428003ct, and an isotype control antibody CN2_sc was used as a control.

On the day before the starting day of evaluation, MDA-MB-231 cells (ATCC: HTB-26) stably expressing pp65 protein (accession number: P06725) and mRFP (Origene: PS100041) derived from Human cytomegalovirus (CMV) (strain AD169) were suspended in RPMI-1640 medium supplemented with 10% FCS, 100 units/mL penicillin, 100 μg (microgram)/mL streptomycin, 1× GlutaMax (Gibco, 35050-061), and 10 mM HEPES , and seeded into a 96-well plate to incubate for one day at 37°C with 5% CO$_2$.

On the starting day of evaluation, PBMCs from healthy human volunteers with HLA type HLA-A02 were suspended in RPMI-1640 medium supplemented with 10% FCS, 100 units/mL penicillin, 100 μg (microgram)/mL streptomycin, 1× GlutaMax (Gibco, 35050-061), and 10 mM HEPES, and added to a 96-well plates where the number of PBMCs added to the 96-well plates was five times the number of tumor cells seeded the day before. The subject antibodies or an isotype control antibody at a final concentration of 10 μg (microgram)/mL were added to the wells which contain the tumor cells and PBMCs, which were co-cultured at 37°C with 5% CO$_2$ for 5 days.

After the co-culture, the culture supernatant and non-adherent cells were collected, followed by washing twice with PBS, and 70 μL (microliter) of RPMI-1640 medium supplemented with 10% FCS, 100 unit/mL penicillin, 100 μg (microgram)/mL streptomycin, 1× GlutaMax (Gibco, 35050-061), and 10 mM HEPES was added to the wells. Subsequently, 70 μL (microliter) of Cell Titer Glo solution (Promega: G7570) was added, and after the contents of each well were shaken at room temperature for 10 minutes, the contents of each well (100 μL (microliter) each) were then dispensed into a white 96-well plate, and the luminescence was measured using a luminescence plate reader.

The culture supernatants collected were centrifuged to remove cellular components and were used to quantitate IFNγ and TNFα concentrations using Luminex.

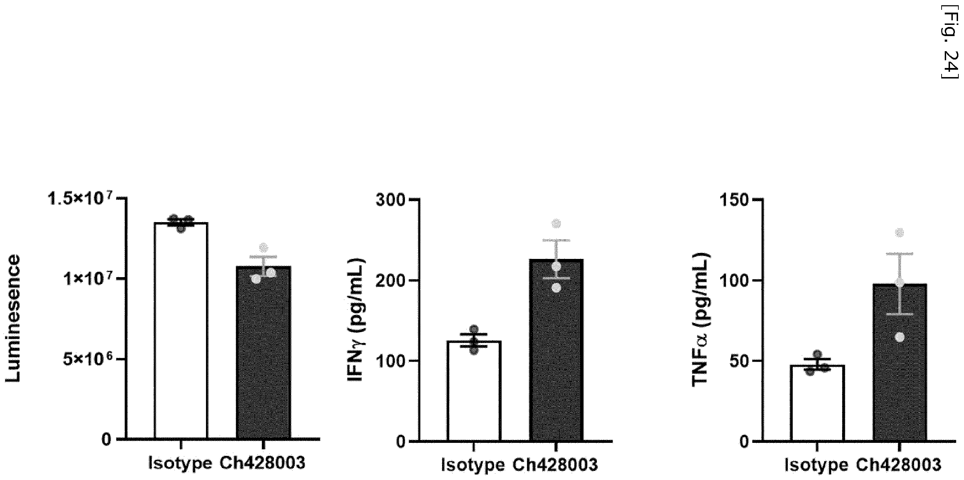

The concentrations of IFNγ and TNFα in the culture supernatant on day 5 of co-culture, as well as the luminescence from ATP derived from the live tumor cells on day 5 of co-culture are shown in Figure 24.

The concentrations of IFNγ and TNFα in the culture supernatant of the group treated with the anti-human Tim-3 biparatopic antibody Ch428003ct of the present invention significantly increased, compared to that of the group treated with an isotype control antibody, confirming that the anti-human Tim-3 biparatopic antibody also enhances the activation of antigen-specific T cells by the endogenous antigens presented on the cell membrane.

Furthermore, Ch428003ct inhibited the proliferation of tumor cells by 20% in the co-culture condition with PBMCs, compared to the isotype control antibody, showing that the anti-human Tim-3 biparatopic antibodies enhance the antitumor activity of the T cells activated by endogenous antigens against tumor cells expressing the relevant antigen.

Example 22: Efficacy study using human Tim-3 knock-in mice

In this example, the antitumor effect of the humanized anti-human Tim-3 biparatopic? antibody of the present invention was evaluated in a mouse model.

Since the humanized anti-human Tim-3 biparatopic antibodies in the present invention bind to human Tim-3, the evaluation was conducted using the human Tim-3 knock-in mice (Biocytogen: B-hTim3 mice) in which mouse Tim-3 gene was genetically replaced with human Tim-3 gene.

On day 0 of the study, MC38_OVA cells in 1 mL of HBSS containing Matrigel were suspended to be at a concentration of $5\times10^7$ cells, and subsequently, 100 μL (microliter) of the cell suspension ($5\times10^6$ cells/mouse) was transplanted subcutaneously in the abdomen of hTim-3 knock-in mice. The body weight and tumor volume were measured at a frequency of twice a week from the day of cell transplantation. The tumor volume was calculated using the following formula:

```
Tumor volume = long diameter x short diameter x short diameter/2
```

On day 6 of the study, the subject animals were grouped into 4 groups (6 animals per group) based on tumor measurements. From the day of grouping, administration of antibodies had begun according to the following table (Table 38). Antibodies were administrated by intraperitoneal administration.

The anti-PD-L1 IgG1LALA antibody used in Table 38 is an in-house prepared version of the variable region of Atezolizumab (PubChem SID 312642102), which are cross-reactive with human and mouse, in the form of the IgG1LALA formatted IgG1 antibody.

Table 38

| group | Antibody 1 (Antibody name) | Antibody 1 (Dosage) | Antibody 2 (Dosing frequency) | Antibody 2 (Antibody name) | Antibody 2 (Dosage) | Antibody 2 (Dosing frequency) |
|---|---|---|---|---|---|---|
| group 1 | IC_sc | 20 mg/kg | BIW (7 times) | IC_LA | 1 mg/kg | BIW (7 times) |
| group 2 | Hu149003ct | 20 mg/kg | BIW (7 times) | IC_LA | 1 mg/kg | BIW (7 times) |
| group 3 | IC_sc | 20 mg/kg | BIW (7 times) | Anti-PD-L1 IgG1 LALA | 1 mg/kg | BIW (7 times) |
| Group 4 | Hu149003ct | 20 mg/kg | BIW (7 times) | Anti-PD-L1 IgG1 LALA | 1 mg/kg | BIW (7 times) |

The body weight and tumor volume of mice were measured until day 31 of the study, and then the mice were sacrificed by euthanasia on day 32 of the study. Tumor tissues were excised from all of mice and analyzed for tumor infiltrating lymphocytes as described below.

The excised tumors were dissected into approximately 3 mm-sized cubic pieces using a clean scalpel, then immersed in an enzyme solution cocktail (Miltenyi Biotec: MACS Tumor Dissociation kit), and the tumor tissues were subsequently processed using the Gentle MACS device (Miltenyi Biotec) to obtain single-cell suspensions. The tumor single cells prepared were stained using fluorescently labeled antibodies for mouse cell surface antigens (BioLegend: anti-mouse CD3, 17A2; anti-mouse CD4, GK1.5, MBL; anti-mouse CD8, KT14) and OVA-tetramer (MBL: T-Select H-2Kb OVA Tetramer-SIINFEKL-APC) and tumor-infiltrating lymphocytes were detected using flow cytometry.

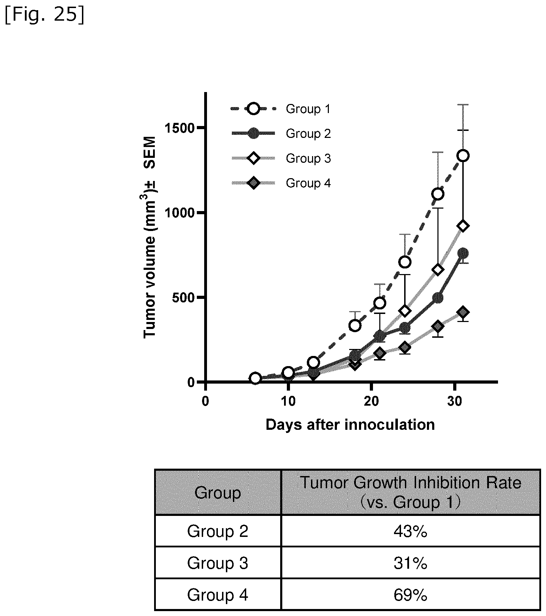

The changes in the average tumor volume for each treatment group, as well as the changes in the individual mouse tumor volume of each treatment group are shown in Figure 25. In the group administrated only with the humanized anti-human Tim-3 biparatopic antibody of the present invention, Hu149003ct, (Group 2), tumor growth was suppressed by an average of 31% at the endpoint (day 31), compared to the group with the isotype control antibody (Group 1). Further, the group administered only with the anti-mouse PD-L1 antibody (Group 3) exhibited a 43% suppression of tumor growth, while the group that received both the anti-mouse PD-L1 antibody and Hu149003ct (Group 4) showed a 69% suppression of tumor growth, indicating that Hu149003ct exhibited an additive effect on the action of the anti-mouse PD-L1 antibody. This demonstrates that the anti-human Tim-3 biparatopic antibody of the present invention exhibits an antitumor effect to subcutaneously transplanted tumors in mice, both when administered as a single agent or in combination with the anti-mouse PD-L1 antibody.

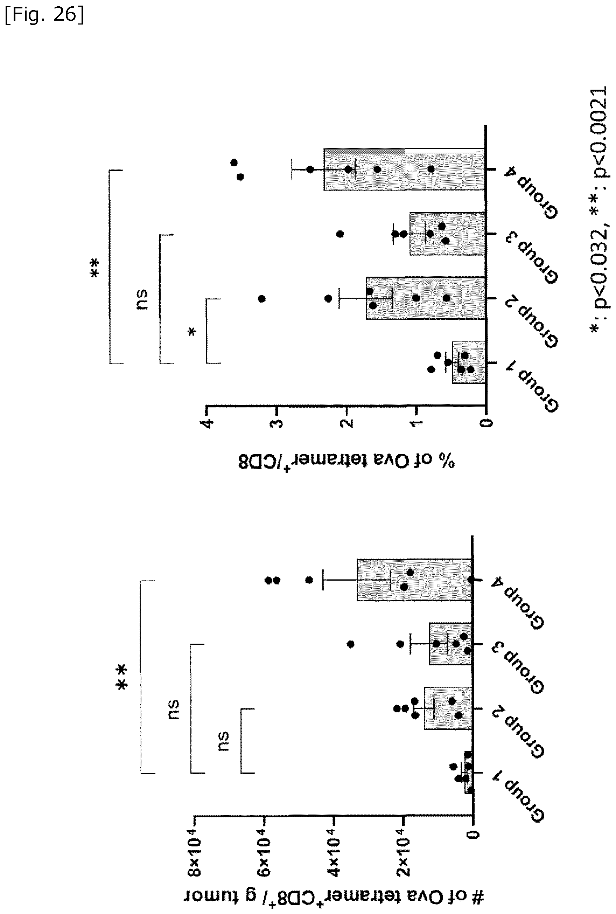

The analysis results of lymphocytes infiltrating tumor tissue collected from the four groups of mice are shown in Figure 26. The analysis results are shown as the number of OVA tetramer-positive CD8-positive T cells per gram of tumor (left) and the percentage of OVA tetramer-positive cells among CD8-positive tumor-infiltrating T cells (right). The average of the number of OVA tetramer-positive CD8-positive T cells per unit tumor weight was higher in group 2 than group 1, and higher in group 4 than group 3. The percentage of OVA tetramer-positive cells among CD8-positive tumor-infiltrating T cells was also significantly higher in groups 2 and 4 than group 1. These results suggest that the anti-human Tim-3 biparatopic antibody of the present invention promotes the infiltration of CD8-positive T cells specific for the antigen presented by the tumor or their proliferation in the tumor, which guessed as the mechanisms of its antitumor effect.

Example 23: Epitope Mapping

In this example, the epitopes of the anti-human Tim-3 antibodies Hu003_13_F20_scFv and Hu149_83_scFv were determined by X-ray crystal structural analysis.

(22-1) Antigen preparation

A DNA fragment encoding human Tim-3 IgV domain (aa. 22-130) with a polyhistidine tag at the N-terminus (SEQ ID No: 87) or human Tim-3 IgV domain (aa. 22-130) with a C-tag at the C-terminus (SEQ ID No: 88) was inserted into the pET47b vector, Rosetta-2(DE3)pLysS was transformed with the construct and cultured at 20°C in 2xYT medium to induce human Tim-3 IgV domain expression. The inclusion bodies were washed with Tris-HCl, pH 8.0, and Tris-HCl, pH 8.0 with 0.05% Triton X-100. The inclusion bodies were then denatured in a solution of 6 M Gdm-HCl, 50 mM Tris, pH 8.0, and 10 mM DTT and refolded in a solution of 100 mM Tris-HCl, pH 8.0 with 3.73 mM cystamine, 6.73 mM cysteamine, and 2 mM EDTA.

After refolding process, the human Tim-3 IgV domain (aa. 22-130) protein with a polyhistidine tag at the N-terminus was equilibrated in TBS, pH 7.4, and subjected to gel filtration using Superdex-75. After refolding process, the human Tim-3 IgV domain (aa. 22-130) protein with a C-tag at the C-terminus was passed through an anti-C-tag antibody column (Thermo, 494307201), washed with TBS (pH 7.4), eluted with 2 M $MgCl_2$, and then subjected to gel filtration using Superdex-75 after equilibration with TBS, pH 7.4.

(22-2) Preparation of Hu003_13_F20 scFv

To enable the large-scale expression and purification of Hu003_13_F20 as an scFv, a DNA fragment encoding Hu003_13_F20 scFv which had a pelB added at the N-terminus and a FLAG tag and polyhistidine tag added at the C-terminus was inserted into a pTZ19R vector with the a lac promoter, which was used to transform E. coli strain, DH12S, that was plated on LB agar medium containing 100 μg (microgram)/mL ampicillin and 1% glucose, then incubated overnight at 37°C. The resulting colony was transferred to Terrific Broth (TB) medium containing 100 μg (microgram)/mL ampicillin and 1 mM IPTG and cultured with shaking at 30°C for 24 hours to allow secretory expression of the scFv protein into the culture supernatant. The culture supernatant after centrifugation was subjected to 0.22 um (micrometer) filtration.

The supernatant after filtration was loaded on an anti-FLAG tag antibody column, washed with PBS (pH 7.4), and eluted with PBS (pH 7.4) containing 0.1 mg/mL FLAG tag peptide from the column. Then, the protein was subjected to gel filtration using Superdex-75 after equilibration with TBS (pH 7.4).

(22-3) Preparation of Hu149_83 scFv

To enable the large-scale expression and purification of Hu149_83 as an scFv, a DNA fragment encoding Hu149_83 scFv which had a pelB added at the N-terminus and a C-tag added at the C-terminus was inserted into a pTZ19R vector with a lac promoter, which was used to transform E. coli strain DH12S, that was plated on LB agar medium containing 100 μg (microgram)/mL ampicillin and 1% glucose, then incubated overnight at 37°C. The resulting colony was transferred to Terrific Broth (TB) medium containing 100 μg (microgram)/mL ampicillin and 1 mM IPTG and cultured with shaking at 30°C for 24 hours to allow secretory expression of the scFv protein into the culture supernatant. The culture supernatant after centrifugation was subjected to 0.22 um (micrometer) filtration.

The supernatant after filtration was loaded on an anti-C-tag antibody column (Thermo, 494307201), washed with TBS (pH 7.4), and eluted with 2 M $MgCl_2$ from the column. Then, the protein was subjected to gel filtration using Superdex-75 after equilibration with TBS (pH 7.4).

(22-4) Epitope Determination by X-ray Structural Analysis

The epitope determination by X-ray structural analysis was outsourced to Proteros biostructures GmbH. The details are as follows:

The prepared antigen and scFv were mixed and the complex of the antigen and scFv were purified by gel filtration. Crystallization conditions were screened and optimized using the purified complex, and diffraction experiments were conducted using the obtained crystals. For Hu003_13_F20, diffraction data with a resolution of 2.05 Å (angstrom) were obtained, and for Hu149_83, diffraction data with a resolution of 2.11 Å (angstrom) were obtained, respectively, and the structure of the complex was determined using molecular replacement method. The interaction sites were identified from the obtained structure, and the epitope was determined.

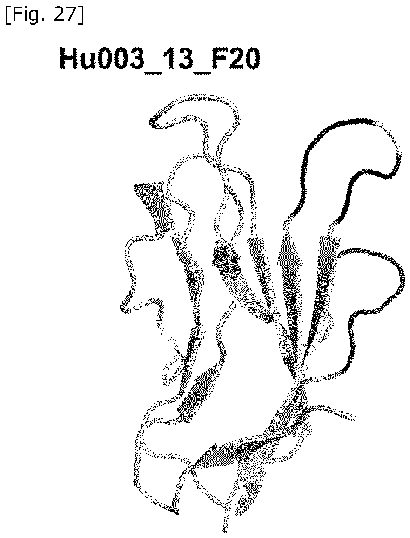

The structure of the human Tim-3 IgV domain obtained by X-ray crystal structural analysis and the epitope site of the Hu003_13_F20_scFv antibody are shown in Figure 27, and the epitope of the Hu003_13_F20_scFv antibody are shown in Table 39. The binding site of Phosphatidylserine to human Tim-3 is at 111R and 118M, and the human Tim-3 binds to the site between the F-G loop and C-C' loop of human Tim-3. The epitope of Hu003_13_F20_scFv antibody spans the F-G loop and C-C' loop of human Tim-3, and it can be seen to inhibit the binding of human Tim-3 to Phosphatidylserine. These results are consistent with the results of Example 13.

The binding site of human Galectin-9 to human Tim-3 is an N-linked sugar chain at 99N, and the Hu003_13_F20_scFv antibody does not directly bind to this binding site. However, the results of Example 13 depict that the Hu003_13_F20_scFv antibody was able to inhibit the binding of human Galectin-9 to human Tim-3 by approximately 60%, suggesting that steric hindrance caused by binding of the Hu003_13_F20_scFv antibody to human Tim-3 inhibits the binding of human Galectin-9 to human Tim-3.

[Table 39]

| Hu003_13_F20 Epitope | |
|---|---|
| 55K | 69R |
| 57A | 77Y |
| 58C | 113Q |
| 59P | 114I |
| 60V | 115P |
| 61F | 116G |
| 62E | 118M |
| 63C | 119N |
| 65N | 120D |
| 66V | |

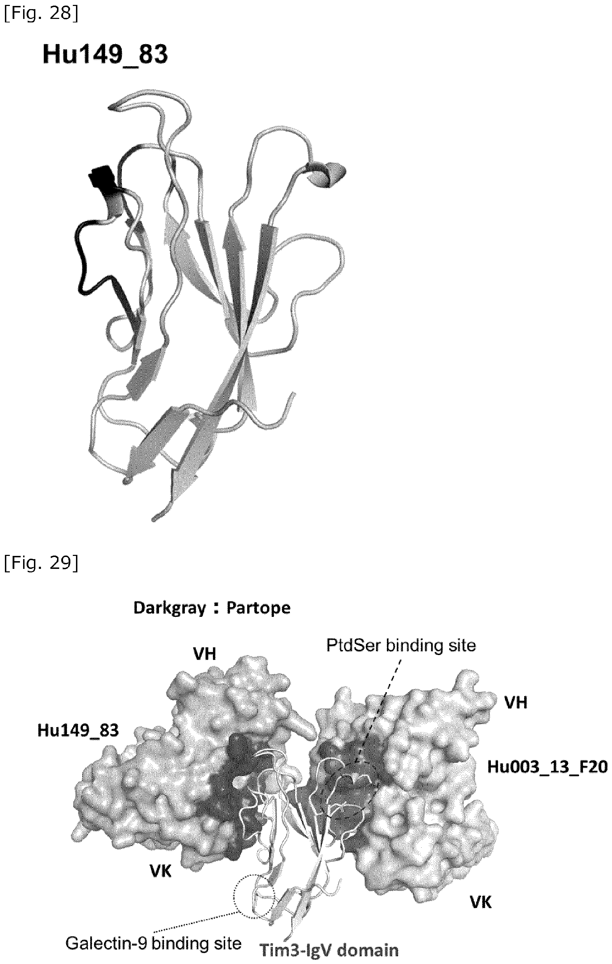

The structure of the human Tim-3 IgV domain obtained by X-ray crystal structural analysis and the epitope site of the Hu149_83_scFv antibody are shown in Figure 28, and the epitope of the Hu149_83_scFv antibody are shown in Table 40. The binding site of Phosphatidylserine to human Tim-3 is at 111R and 118M, and the human Tim-3 binds to the site between the F-G loop and C-C' loop of human Tim-3. The epitope of Hu149_83_scFv antibody does not participate in the F-G loop and C-C' loop of human Tim-3, and these results are consistent with the results of Example 13 that the Hu149_83_scFv antibody does not inhibit the binding of Phosphatidylserine to human Tim-3.

The binding site of human Galectin-9 to human Tim-3 is an N- linked sugar chain at 99N, and the Hu149_83_scFv antibody does not directly bind to this binding site. However, the results of Example 13 depict that the Hu149_83_scFv antibody was able to inhibit the binding of human Galectin-9 to human Tim-3 by approximately 60%, suggesting that steric hindrance caused by binding of the Hu149_83_scFv antibody to human Tim-3 inhibits the binding of human Galectin-9 to human Tim-3.

[Table 40]

| Hu149_83 Epitope | |
| --- | --- |
| 45P | 82Y |
| 46G | 83W |
| 71D | 84L |
| 73R | 85N |
| 74D | 86G |
| 75V | 87D |
| 78W | 89R |
| 80S | 90K |

(22-5) Binding Model of Biparatopic Antibody with Human Tim-3

A binding model of biparatopic antibody Hu149003 based on the results of the binding structural analysis of the complex of Hu003_13_F20_scFv antibody and human Tim-3 (22-3), as well as the results of the binding structural analysis of the complex of Hu149_83_scFv and human Tim-3 (22-4) is shown in Figure 29. This model shows that the humanized biparatopic antibody binds to human Tim-3 in such a way that the two scFv arms sandwich human Tim-3 without competing with each other, with completely inhibiting the binding of human Tim-3 to Phosphatidylserine by Hu003_13_F20_scFv, and effectively inhibits the binding of human Tim-3 and Galectin-9 by binding between the two arms, either through a conformational change of Tim-3 or steric hindrance.

Industrial applicability

The antibody etc. obtained by the present invention inhibits the function of Tim-3 expressed on cancer cells or immune cells, and activate immune cells, by relieving the exhaustion of immune cells (especially T cells), thereby demonstrating an effect of suppression of tumor growth and an effect of cancer treatment.

The present invention can be broadly applied for a therapeutic and diagnostic agent for cancer. The antibodies produced by this invention are expected to be particularly effective in combination therapies with other immunotherapies, since it exhibits the effect of releasing the exhaustion of immune cells.

**Claims**

1. A heterodimeric antibody or antibody derivative that is a combination of two different half-molecules each of which binds to the Tim-3 antigen and enhances immune activity against cancer cells.

2. The antibody or antibody derivative according to claim 1, wherein the one half-molecule of the antibody or antibody derivative binds to the first region of the Tim-3 antigen, and the other half-molecule of the antibody or antibody derivative binds to the second region of the Tim-3 antigen.

3. The antibody or antibody derivative according to claim 1 or 2, wherein the half-molecule of the antibody or antibody derivative is a combination of two different types of half-molecule of the antibody or antibody derivative comprising either set of the complementarity-determining regions of the heavy and light chains selected from the group consisting of:

(1) Complementarity-determining regions of the heavy chain, CDR1 (SYYMS, SEQ ID No: 1), CDR2 (TISNSGGSIYYLDSVKD, SEQ ID No: 2), and CDR3 (DPYYSNYVPMDY, SEQ ID No: 3), and complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);
(2) Complementarity-determining regions of the heavy chain, CDR1 (DYYMD, SEQ ID No: 9), CDR2 (YIYPNNGGTSYNQKFKG, SEQ ID No: 10), and CDR3 (SGYGNYYTMDY, SEQ ID No: 11), and complementarity-determining regions of the light chain: CDR1 (KASQSVGNNVA, SEQ ID No: 12), CDR2 (YASNRYT, SEQ ID No: 13), and CDR3 (QQHYSSPST, SEQ ID No: 14);

(3) Complementarity-determining regions of the heavy chain, CDR1 (DYYMD, SEQ ID No: 17), CDR2 (YIYPNNGGTSYNQKFKG, SEQ ID No: 18), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(4) Complementarity-determining regions of the heavy chain, CDR1 (DYYMD, SEQ ID No: 25), CDR2 (YIYPNNGGTSYNQKFKG, SEQ ID No: 26), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(5) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);

(6) Complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASENVGTYVS, SEQ ID No: 52), CDR2 (GASNRYT, SEQ ID No: 53), and CDR3 (GQSYSYPLT, SEQ ID No: 54);

(7) Complementarity-determining regions of the heavy chain, CDR1 (DYYMD, SEQ ID No: 9), CDR2 (YIYPNNAGTSYNQKFKG, SEQ ID No: 74), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(8) Complementarity-determining regions of the heavy chain, CDR1 (DYYMD, SEQ ID No: 9), CDR2 (YIYPNKGGTSYNQKFKG, SEQ ID No: 76), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(9) Complementarity-determining regions of the heavy chain, CDR1 (DYYMD, SEQ ID No: 17), CDR2 (YIYPNNAGTSYNQKFKG, SEQ ID No: 78), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(10) Complementarity-determining regions of the heavy chain, CDR1 (DYYMD, SEQ ID No: 17), CDR2 (YIYPNKGGTSYNQKFKG, SEQ ID No: 80), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(11) Complementarity-determining regions of the heavy chain, CDR1 (DYYMD, SEQ ID No: 25), CDR2 (YIYPNNAGTSYNQKFKG, SEQ ID No: 82), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(12) Complementarity-determining regions of the heavy chain, CDR1 (DYYMD, SEQ ID No: 25), CDR2 (YIYPNKGGTSYNQKFKG, SEQ ID No: 84), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30).

4. The antibody or antibody derivative according to any one of claims 1-3, wherein the one half-molecule of the antibody or antibody derivative is a half-molecule of (1), (5) or (6) and the other half-molecule of the antibody or antibody derivative is a half-molecule of (2) to (4) or (7) to (12).

5. The antibody or antibody derivative according to any one of claims 1-4, wherein the antibody derivative is selected from a modified antibody selected from a humanized antibody, a chimeric antibody, a single chain Fv antibody (a scFv antibody), Fab, Fab', F(ab')2, Fc effector function modified antibody, IgG1LALA, IgG1_N297A, IgG4_S228P or functional fragments thereof.

6. The antibody or antibody derivative according to any one of claims 1 to 5, wherein the amino acid sequence of the heavy chain variable region VH domain of the half-molecule of the antibody or antibody derivative is selected from the group consisting of:

(VH-1a) the amino acid sequence of SEQ ID No: 7, or an amino acid sequence comprising one or more amino acid substitutions (e.g., conservative substitutions), insertions, or deletions in regions of the amino acid sequence of SEQ ID No: 7 other than CDR1 (SEQ ID No: 1), CDR2 (SEQ ID No: 2), and CDR3 (SEQ ID No: 3);

(VH-1b) the amino acid sequence of SEQ ID No: 49, or an amino acid sequence comprising one or more amino acid substitutions (e.g., conservative substitutions), insertions, or deletions in regions of the amino acid sequence of SEQ ID No: 49 other than CDR1 (SEQ ID No: 1), CDR2 (SEQ ID No: 2), and CDR3 (SEQ ID No: 3);

(VH-1c) the amino acid sequence of SEQ ID No: 57, or an amino acid sequence comprising one or more amino acid substitutions (e.g., conservative substitutions), insertions, or deletions in regions of the amino acid sequence of SEQ ID No: 57 other than CDR1 (SEQ ID No: 1), CDR2 (SEQ ID No: 2), and CDR3 (SEQ ID No: 3);

(VH-2a) the amino acid sequence of SEQ ID No: 15, or an amino acid sequence comprising one or more amino acid substitutions (e.g., conservative substitutions), insertions, or deletions in regions of the amino acid sequence of SEQ ID No: 15 other than CDR1 (SEQ ID No: 9), CDR2 (SEQ ID No: 10), and CDR3 (SEQ ID No: 11);

(VH-2b) the amino acid sequence of SEQ ID No: 62, or an amino acid sequence comprising one or more amino acid substitutions (e.g., conservative substitutions), insertions, or deletions in regions of the amino acid sequence of SEQ ID No: 62 other than CDR1 (SEQ ID No: 9), CDR2 (SEQ ID No: 10), and CDR3 (SEQ ID No: 11);

(VH-3a) the amino acid sequence of SEQ ID No: 23, or an amino acid sequence comprising one or more amino acid substitutions (e.g., conservative substitutions), insertions, or deletions in regions of the amino acid sequence of SEQ ID No: 23 other than CDR1 (SEQ ID No: 17), CDR2 (SEQ ID No: 18), and CDR3 (SEQ ID No: 19);

(VH-3b) the amino acid sequence of SEQ ID No: 66, or an amino acid sequence comprising one or more amino acid substitutions (e.g., conservative substitutions), insertions, or deletions in regions of the amino acid sequence of SEQ ID No: 66 other than CDR1 (SEQ ID No: 17), CDR2 (SEQ ID No: 18), and CDR3 (SEQ ID No: 19);

(VH-4a) the amino acid sequence of SEQ ID No: 31, or an amino acid sequence comprising one or more amino acid substitutions (e.g., conservative substitutions), insertions, or deletions in regions of the amino acid sequence of SEQ ID No: 31 other than CDR1 (SEQ ID No: 25), CDR2 (SEQ ID No: 26), and CDR3 (SEQ ID No: 27);

(VH-4b) the amino acid sequence of SEQ ID No: 70, or an amino acid sequence comprising one or more amino acid substitutions (e.g., conservative substitutions), insertions, or deletions in regions of the amino acid sequence of SEQ ID No: 70 other than CDR1 (SEQ ID No: 25), CDR2 (SEQ ID No: 26), and CDR3 (SEQ ID No: 27);

(VH-5a) the amino acid sequence of SEQ ID No: 36, or an amino acid sequence comprising one or more amino acid substitutions (e.g., conservative substitutions), insertions, or deletions in regions of the amino acid sequence of SEQ ID No: 36 other than CDR1 (SEQ ID No: 33), CDR2 (SEQ ID No: 34), and CDR3 (SEQ ID No: 35);

(VH-5b) the amino acid sequence of SEQ ID No: 51, or an amino acid sequence comprising one or more amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35);

(VH-5c) the amino acid sequence of SEQ ID No: 59, or an amino acid sequence comprising one or more amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35);

(VH-6a) the amino acid sequence of SEQ ID No: 55, or an amino acid sequence comprising one or more amino acid substitutions (e.g., conservative substitutions), insertions, or deletions in regions of the amino acid sequence of SEQ ID No: 55 other than CDR1 (SEQ ID No: 33), CDR2 (SEQ ID No: 34), and CDR3 (SEQ ID No: 35);

(VH-6b) the amino acid sequence of SEQ ID No: 51, or an amino acid sequence comprising one or more amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35);

(VH-6c) the amino acid sequence of SEQ ID No: 59, or an amino acid sequence comprising one or more amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35);

(VH-7) the amino acid sequence of SEQ ID No: 75, or an amino acid sequence comprising one or more amino acid substitutions (e.g., conservative substitutions), insertions, or deletions in regions of the amino acid sequence of SEQ ID No: 75 other than CDR1 (SEQ ID No: 9), CDR2 (SEQ ID No: 74), and CDR3 (SEQ ID No: 11);

(VH-8) the amino acid sequence of SEQ ID No: 77, or an amino acid sequence comprising one or more amino acid substitutions (e.g., conservative substitutions), insertions, or deletions in regions of the amino acid sequence of SEQ ID No: 77 other than CDR1 (SEQ ID No: 9), CDR2 (SEQ ID No: 76), and CDR3 (SEQ ID No: 11);

(VH-9) the amino acid sequence of SEQ ID No: 79, or an amino acid sequence comprising one or more amino acid substitutions (e.g., conservative substitutions), insertions, or deletions in regions of the amino acid sequence of SEQ ID No: 79 other than CDR1 (SEQ ID No: 17), CDR2 (SEQ ID No: 78), and CDR3 (SEQ ID No: 19);

(VH-10) the amino acid sequence of SEQ ID No: 81, or an amino acid sequence comprising one or more amino acid substitutions (e.g., conservative substitutions), insertions, or deletions in regions of the amino acid sequence of SEQ ID No: 81 other than CDR1 (SEQ ID No: 17), CDR2 (SEQ ID No: 80), and CDR3 (SEQ ID No: 19);

(VH-11) the amino acid sequence of SEQ ID No: 83, or an amino acid sequence comprising one or more amino acid substitutions (e.g., conservative substitutions), insertions, or deletions in regions of the amino acid sequence of SEQ ID No: 83 other than CDR1 (SEQ ID No: 25), CDR2 (SEQ ID No: 82), and CDR3 (SEQ ID No: 27);

(VH-12) the amino acid sequence of SEQ ID No: 85, or an amino acid sequence comprising one or more amino acid substitutions (e.g., conservative substitutions), insertions, or deletions in regions of the amino acid sequence of SEQ ID No: 85 other than CDR1 (SEQ ID No: 25), CDR2 (SEQ ID No: 84), and CDR3 (SEQ ID No: 27).

7. The antibody or antibody derivative according to any one of claims 1-6, wherein the amino acid sequence of the light chain variable region VL domain of the half-molecule of the antibody or antibody derivative is selected from the group consisting of;

(VL-1a) the amino acid sequence of SEQ ID No: 8, or an amino acid sequence comprising one or more amino acid substitutions (e.g., conservative substitutions), insertions, or deletions in regions the amino acid sequence of SEQ ID No: 8 other than CDR1 (SEQ ID No: 4), CDR2 (SEQ ID No: 5), and CDR3 (SEQ ID No: 6);

(VL-1b) the amino acid sequence of SEQ ID No: 58, or an amino acid sequence comprising one or more amino acid substitutions (e.g., conservative substitutions), insertions, or deletions in regions the amino acid sequence of SEQ ID No: 58 other than CDR1 (SEQ ID No: 4), CDR2 (SEQ ID No: 5), and CDR3 (SEQ ID No: 6);

(VL-1c) the amino acid sequence of SEQ ID No: 50, or an amino acid sequence comprising one or more amino acid substitutions (e.g., conservative substitutions), insertions, or deletions in regions the amino acid sequence of SEQ ID No: 50 other than CDR1 (SEQ ID No: 4), CDR2 (SEQ ID No: 5), and CDR3 (SEQ ID No: 6);

(VL-2a) the amino acid sequence of SEQ ID No: 16, or an amino acid sequence comprising one or more amino acid substitutions (e.g., conservative substitutions), insertions, or deletions in regions the amino acid sequence of SEQ ID No: 16 other than CDR1 (SEQ ID No: 12), CDR2 (SEQ ID No: 13), and CDR3 (SEQ ID No: 14);

(VL-2b) the amino acid sequence of SEQ ID No: 63, or an amino acid sequence comprising one or more amino acid substitutions (e.g., conservative substitutions), insertions, or deletions in regions the amino acid sequence of SEQ ID No: 63 other than CDR1 (SEQ ID No: 12), CDR2 (SEQ ID No: 13), and CDR3 (SEQ ID No: 14);

(VL-2c) the amino acid sequence of SEQ ID No: 64, or an amino acid sequence comprising one or more amino acid substitutions (e.g., conservative substitutions), insertions, or deletions in regions the amino acid sequence of SEQ ID No: 64 other than CDR1 (SEQ ID No: 12), CDR2 (SEQ ID No: 13), and CDR3 (SEQ ID No: 14);

(VL-2d) the amino acid sequence of SEQ ID No: 65, or an amino acid sequence comprising one or more amino acid substitutions (e.g., conservative substitutions), insertions, or deletions in regions the amino acid sequence of SEQ ID No: 65 other than CDR1 (SEQ ID No: 12), CDR2 (SEQ ID No: 13), and CDR3 (SEQ ID No: 14);

(VL-3a) the amino acid sequence of SEQ ID No: 24, or an amino acid sequence comprising one or more amino acid substitutions (e.g., conservative substitutions), insertions, or deletions in regions the amino acid sequence of SEQ ID No: 24 other than CDR1 (SEQ ID No: 20), CDR2 (SEQ ID No: 21), and CDR3 (SEQ ID No: 22);

(VL-3b) the amino acid sequence of SEQ ID No: 67, or an amino acid sequence comprising one or more amino acid substitutions (e.g., conservative substitutions), insertions, or deletions in regions the amino acid sequence of SEQ ID No: 67 other than CDR1 (SEQ ID No: 20), CDR2 (SEQ ID No: 21), and CDR3 (SEQ ID No: 22);

(VL-3c) the amino acid sequence of SEQ ID No: 68, or an amino acid sequence comprising one or more amino acid substitutions (e.g., conservative substitutions), insertions, or deletions in regions the amino acid sequence of SEQ ID No: 68 other than CDR1 (SEQ ID No: 20), CDR2 (SEQ ID No: 21), and CDR3 (SEQ ID No: 22);

(VL-3d) the amino acid sequence of SEQ ID No: 69, or an amino acid sequence comprising one or more amino acid substitutions (e.g., conservative substitutions), insertions, or deletions in regions the amino acid sequence of SEQ ID No: 69 other than CDR1 (SEQ ID No: 20), CDR2 (SEQ ID No: 21), and CDR3 (SEQ ID No: 22);

(VL-4a) the amino acid sequence of SEQ ID No: 32, or an amino acid sequence comprising one or more amino acid substitutions (e.g., conservative substitutions), insertions, or deletions in regions the amino acid sequence of SEQ ID No: 32 other than CDR1 (SEQ ID No: 28), CDR2 (SEQ ID No: 29), and CDR3 (SEQ ID No: 30);

(VL-4b) the amino acid sequence of SEQ ID No: 71, or an amino acid sequence comprising one or more amino acid substitutions (e.g., conservative substitutions), insertions, or deletions in regions the amino acid sequence of SEQ ID No: 71 other than CDR1 (SEQ ID No: 28), CDR2 (SEQ ID No: 29), and CDR3 (SEQ ID No: 30);

(VL-4c) the amino acid sequence of SEQ ID No: 72, or an amino acid sequence comprising one or more amino acid substitutions (e.g., conservative substitutions), insertions, or deletions in regions the amino acid sequence of SEQ ID No: 72 other than CDR1 (SEQ ID No: 28), CDR2 (SEQ ID No: 29), and CDR3 (SEQ ID No: 30);

(VL-4d) the amino acid sequence of SEQ ID No: 73, or an amino acid sequence comprising one or more amino acid substitutions (e.g., conservative substitutions), insertions, or deletions in regions the amino acid sequence of SEQ ID No: 73 other than CDR1 (SEQ ID No: 28), CDR2 (SEQ ID No: 29), and CDR3 (SEQ ID No: 30);

(VL-5a) the amino acid sequence of SEQ ID No: 8, or an amino acid sequence comprising one or more amino acid substitutions (e.g., conservative substitutions), insertions, or deletions in regions the amino acid sequence of SEQ ID No: 8 other than CDR1 (SEQ ID No: 4), CDR2 (SEQ ID No: 5), and CDR3 (SEQ ID No: 6);

(VL-5b) the amino acid sequence of SEQ ID No: 58, or an amino acid sequence comprising one or more amino acid substitutions (e.g., conservative substitutions), insertions, or deletions in regions the amino acid sequence of SEQ ID No: 58 other than CDR1 (SEQ ID No: 4), CDR2 (SEQ ID No: 5), and CDR3 (SEQ ID No: 6);

(VL-5c) the amino acid sequence of SEQ ID No: 50, or an amino acid sequence comprising one or more amino acid substitutions (e.g., conservative substitutions), insertions, or deletions in regions the amino acid sequence of SEQ ID No: 50 other than CDR1 (SEQ ID No: 4), CDR2 (SEQ ID No: 5), and CDR3 (SEQ ID No: 6);

(VL-6a) the amino acid sequence of SEQ ID No: 56, or an amino acid sequence comprising one or more amino acid substitutions (e.g., conservative substitutions), insertions, or deletions in regions the amino acid sequence of SEQ ID No: 56 other than CDR1 (SEQ ID No: 52), CDR2 (SEQ ID No: 53), and CDR3 (SEQ ID No: 54);

(VL-6b) the amino acid sequence of SEQ ID No: 60, or an amino acid sequence comprising one or more amino acid substitutions (e.g., conservative substitutions), insertions, or deletions in regions the amino acid sequence of SEQ ID No: 60 other than CDR1 (SEQ ID No: 52), CDR2 (SEQ ID No: 53), and CDR3 (SEQ ID No: 54);

(VL-6c) the amino acid sequence of SEQ ID No: 61, or an amino acid sequence comprising one or more amino acid substitutions (e.g., conservative substitutions), insertions, or deletions in regions the amino acid sequence of SEQ ID No: 61 other than CDR1 (SEQ ID No: 52), CDR2 (SEQ ID No: 53), and CDR3 (SEQ ID No: 54).

8. The antibody or antibody derivative according to any one of claims 1-4, wherein the antibody derivative is selected from the following variable regions of the half-molecules composed of the single chain Fv antibodies (scFv):

(1) an amino acid sequence in which the heavy chain variable region of (VH-1a), (VH-1b), or (VH-1c) and the light chain variable region of (VL-1a), (VL-1b), or (VL-1c) are connected via a linker;

(2) an amino acid sequence in which the heavy chain variable region of (VH-2a) or (VH-2b) and the light chain variable region of (VL-2a), (VL-2b), (VL-2c), or (VL-2d) are connected via a linker;

(3) an amino acid sequence in which the heavy chain variable region of (VH-3a) or (VH-3b) and the light chain variable region of (VL-3a), (VL-3b), (VL-3c), or (VL-3d) are connected via a linker;

(4) an amino acid sequence in which the heavy chain variable region of (VH-4a) or (VH-4b) and the light chain variable region of (VL-4a), (VL-4b), (VL-4c), or (VL-4d) are connected via a linker;

(5) an amino acid sequence in which the heavy chain variable region of (VH-5a), (VH-5b), or (VH-5c) and the light chain variable region of (VL-5a), (VL-5b), or (VL-5c) are connected via a linker;

(6) an amino acid sequence in which the heavy chain variable region of (VH-6a), (VH-6b), or (VH-6c) and the light chain variable region of (VL-6a), (VL-6b), or (VL-6c) are connected via a linker;

(7) an amino acid sequence in which the heavy chain variable region of (VH-7) and the light chain variable region of (VL-2a), (VL-2b), (VL-2c), or (VL-2d) are connected via a linker;

(8) an amino acid sequence in which the heavy chain variable region of (VH-8) and the light chain variable region of (VL-2a), (VL-2b), (VL-2c), or (VL-2d) are connected via a linker;

(9) an amino acid sequence in which the heavy chain variable region of (VH-9) and the light chain variable region of (VL-3a), (VL-3b), (VL-3c), or (VL-3d) are connected via a linker;

(10) an amino acid sequence in which the heavy chain variable region of (VH-10) and the light chain variable region of (VL-3a), (VL-3b), (VL-3c), or (VL-3d) are connected via a linker;

(11) an amino acid sequence in which the heavy chain variable region of (VH-11) and the light chain variable region of (VL-4a), (VL-4b), (VL-4c), or (VL-4d) are connected via a linker;

(12) an amino acid sequence in which the heavy chain variable region of (VH-12) and the light chain variable region of (VL-4a), (VL-4b), (VL-4c), or (VL-4d) are connected via a linker;

of the half molecule comprising the single chain Fv antibody (scFv).

9. The antibody or antibody derivative according to any one of claims 1-8, wherein the enhancement of immune activity is activation of T cells selected from the group consisting of removal of immunosuppressive signals, relieving T cell exhaustion, proliferation of T cells, increase in cytotoxicity of T cells against cancer cells, and promotion of T cell cytokine secretion.

10. The antibody or antibody derivative according to any one of claims 1-9, wherein the antibody or antibody derivative induces cytotoxicity against cancer cells but not against normal cells.

11. The antibody or antibody derivative according to any one of claims 1-10, wherein the cancer cell is selected from the group consisting of melanoma, breast cancer, lung cancer, colon cancer, gastric cancer, pancreatic cancer, liver cancer, and blood cancer.

12. A homodimeric antibody or antibody derivative which binds to the Tim-3 antigen and enhances immune activity against cancer cells, comprising a set of the complementarity-determining regions of the heavy and light chains selected from the group consisting of:

(1) complementarity-determining regions of the heavy chain, CDR1 (SYYMS, SEQ ID No: 1), CDR2 (TISNSGGSIYYLDSVKD, SEQ ID No: 2), and CDR3 (DPYYSNYVPMDY, SEQ ID No: 3), and complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);

(2) complementarity-determining regions of the heavy chain, CDR1 (DYYMD, SEQ ID No: 9), CDR2 (YIYPNNGGTSYNQKFKG, SEQ ID No: 10), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(3) complementarity-determining regions of the heavy chain, CDR1 (DYYMD, SEQ ID No: 17), CDR2 (YIYPNNGGTSYNQKFKG, SEQ ID No: 18), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(4) complementarity-determining regions of the heavy chain, CDR1 (DYYMD, SEQ ID No: 25), CDR2 (YIYPNNGGTSYNQKFKG, SEQ ID No: 26), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(5) complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASQYVDTYVA, SEQ ID No: 4), CDR2 (SASTRHT, SEQ ID No: 5), and CDR3 (AQYSSSPLT, SEQ ID No: 6);

(6) complementarity-determining regions of the heavy chain, CDR1 (SYYMS, SEQ ID No: 33), CDR2 (TISNSGGSTYYPDSVKD, SEQ ID No: 34), and CDR3 (DPYYTNYVPMDY, SEQ ID No: 35), and complementarity-determining regions of the light chain, CDR1 (KASENVGTYVS, SEQ ID No: 52), CDR2 (GASNRYT, SEQ ID No: 53), and CDR3 (GQSYSYPLT, SEQ ID No: 54);

(7) complementarity-determining regions of the heavy chain, CDR1 (DYYMD, SEQ ID No: 9), CDR2 (YIYPNNAGTS YNQKFKG, SEQ ID No: 74), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(8) complementarity-determining regions of the heavy chain, CDR1 (DYYMD, SEQ ID No: 9), CDR2 (YIYPNKGGTS YNQKFKG, SEQ ID No: 76), and CDR3 (SGYGNYYTMDY, SEQ ID No: 11), and complementarity-determining regions of the light chain, CDR1 (KASQSVGNNVA, SEQ ID No: 12), CDR2 (YASNRYT, SEQ ID No: 13), and CDR3 (QQHYSSPST, SEQ ID No: 14);

(9) complementarity-determining regions of the heavy chain, CDR1 (DYYMD, SEQ ID No: 17), CDR2 (YIYPNNAGTS YNQKFKG, SEQ ID No: 78), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(10) complementarity-determining regions of the heavy chain, CDR1 (DYYMD, SEQ ID No: 17), CDR2 (YIYPNKGGTS YNQKFKG, SEQ ID No: 80), and CDR3 (GGYYSYYSYDY, SEQ ID No: 19), and complementarity-determining regions of the light chain, CDR1 (KASQSVGNNVA, SEQ ID No: 20), CDR2 (YASNRYT, SEQ ID No: 21), and CDR3 (QQHYSSPYT, SEQ ID No: 22);

(11) complementarity-determining regions of the heavy chain, CDR1 (DYYMD, SEQ ID No: 25), CDR2 (YIYPNNAGTS YNQKFKG, SEQ ID No: 82), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

(12) complementarity-determining regions of the heavy chain, CDR1 (DYYMD, SEQ ID No: 25), CDR2 (YIYPNKGGTS YNQKFKG, SEQ ID No: 84), and CDR3 (SGYKAYYAMDY, SEQ ID No: 27), and complementarity-determining regions of the light chain, CDR1 (KASQSVGNNVA, SEQ ID No: 28), CDR2 (YASNRYT, SEQ ID No: 29), and CDR3 (QQHYSSPYT, SEQ ID No: 30);

**13.** The antibody or antibody derivative according to claim 12, wherein the antibody derivative is selected from a modified antibody selected from a humanized antibody, a chimeric antibody, a single chain Fv antibody (a scFv antibody), Fab, Fab', F(ab')2, Fc effector function modified antibody, IgG1LALA, IgGl_N297A, IgG4_S228P, or a functional fragment thereof.

**14.** The antibody or antibody derivative according to claim 12 or 13,
wherein the amino acid sequence of the heavy chain variable region VH domain of the half-molecule of the antibody or antibody derivative is selected from the group consisting of:

(VH-1a) the amino acid sequence of SEQ ID No: 7, or an amino acid sequence comprising one or several amino acid substitutions (e.g., conservative substitutions), insertions, or deletions in regions of the amino acid sequence of SEQ ID No: 7 other than CDR1 (SEQ ID No: 1), CDR2 (SEQ ID No: 2), and CDR3 (SEQ ID No: 3);

(VH-1b) the amino acid sequence of SEQ ID No: 49, or an amino acid sequence comprising one or several amino acid substitutions (e.g., conservative substitutions), insertions, or deletions in regions of the amino acid sequence of SEQ ID No: 49 other than CDR1 (SEQ ID No: 1), CDR2 (SEQ ID No: 2), and CDR3 (SEQ ID No: 3);

(VH-1c) the amino acid sequence of SEQ ID No: 57, or an amino acid sequence comprising one or several amino acid substitutions (e.g., conservative substitutions), insertions, or deletions in regions of the amino acid sequence of SEQ ID No: 57 other than CDR1 (SEQ ID No: 1), CDR2 (SEQ ID No: 2), and CDR3 (SEQ ID No: 3);

(VH-2a) the amino acid sequence of SEQ ID No: 15, or an amino acid sequence comprising one or several amino acid substitutions (e.g., conservative substitutions), insertions, or deletions in regions of the amino acid sequence of SEQ ID No: 15 other than CDR1 (SEQ ID No: 9), CDR2 (SEQ ID No: 10), and CDR3 (SEQ ID No: 11);

(VH-2b) the amino acid sequence of SEQ ID No: 62, or an amino acid sequence comprising one or several amino acid substitutions (e.g., conservative substitutions), insertions, or deletions in regions of the amino acid sequence of SEQ ID No: 62 other than CDR1 (SEQ ID No: 9), CDR2 (SEQ ID No: 10), and CDR3 (SEQ ID No: 11);

(VH-3a) the amino acid sequence of SEQ ID No: 23, or an amino acid sequence comprising one or several amino acid substitutions (e.g., conservative substitutions), insertions, or deletions in regions of the amino acid sequence of SEQ ID No: 23 other than CDR1 (SEQ ID No: 17), CDR2 (SEQ ID No: 18), and CDR3 (SEQ ID No: 19);

(VH-3b) the amino acid sequence of SEQ ID No: 66, or an amino acid sequence comprising one or several amino acid substitutions (e.g., conservative substitutions), insertions, or deletions in regions of the amino acid sequence of SEQ ID No: 66 other than CDR1 (SEQ ID No: 17), CDR2 (SEQ ID No: 18), and CDR3 (SEQ ID No: 19);

(VH-4a) the amino acid sequence of SEQ ID No: 31, or an amino acid sequence comprising one or several amino acid substitutions (e.g., conservative substitutions), insertions, or deletions in regions of the amino acid sequence of SEQ ID No: 31 other than CDR1 (SEQ ID No: 25), CDR2 (SEQ ID No: 26), and CDR3 (SEQ ID No: 27);

(VH-4b) the amino acid sequence of SEQ ID No: 70, or an amino acid sequence comprising one or several amino acid substitutions (e.g., conservative substitutions), insertions, or deletions in regions of the amino acid sequence of SEQ ID No: 70 other than CDR1 (SEQ ID No: 25), CDR2 (SEQ ID No: 26), and CDR3 (SEQ ID No: 27);

(VH-5a) the amino acid sequence of SEQ ID No: 36, or an amino acid sequence comprising one or several amino acid substitutions (e.g., conservative substitutions), insertions, or deletions in regions of the amino acid sequence of SEQ ID No: 36 other than CDR1 (SEQ ID No: 33), CDR2 (SEQ ID No: 34), and CDR3 (SEQ ID No: 35);

(VH-5b) the amino acid sequence of SEQ ID No: 51, or an amino acid sequence comprising one or several amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35);

(VH-5c) the amino acid sequence of SEQ ID No: 59, or an amino acid sequence comprising one or several amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35);

(VH-6a) the amino acid sequence of SEQ ID No: 55, or an amino acid sequence comprising one or several amino acid substitutions (e.g., conservative substitutions), insertions, or deletions in regions of the amino acid sequence of SEQ ID No: 55 other than CDR1 (SEQ ID No: 33), CDR2 (SEQ ID No: 34), and CDR3 (SEQ ID No: 35);

(VH-6b) the amino acid sequence of SEQ ID No: 51, or an amino acid sequence comprising one or several amino acid substitutions (e.g., conservative substitutions), insertions, or deletions in regions of the amino acid sequence of SEQ ID No: 51 other than CDR1 (SEQ ID No: 33), CDR2 (SEQ ID No: 34), and CDR3 (SEQ ID No: 35);

(VH-6c) the amino acid sequence of SEQ ID No: 59, or an amino acid sequence comprising one or several amino acid substitutions (e.g., conservative substitutions), insertions, or deletions in regions of the amino acid sequence of SEQ ID No: 59 other than CDR1 (SEQ ID No: 33), CDR2 (SEQ ID No: 34), and CDR3 (SEQ ID No: 35);

(VH-7) the amino acid sequence of SEQ ID No: 75, or an amino acid sequence comprising one or several amino acid substitutions (e.g., conservative substitutions), insertions, or deletions in regions of the amino acid sequence of SEQ ID No: 75 other than CDR1 (SEQ ID No: 9), CDR2 (SEQ ID No: 74), and CDR3 (SEQ ID No: 11);

(VH-8) the amino acid sequence of SEQ ID No: 77, or an amino acid sequence comprising one or several amino acid substitutions (e.g., conservative substitutions), insertions, or deletions in regions of the amino acid sequence of SEQ ID No: 77 other than CDR1 (SEQ ID No: 9), CDR2 (SEQ ID No: 76), and CDR3 (SEQ ID No: 11);

(VH-9) the amino acid sequence of SEQ ID No: 79, or an amino acid sequence comprising one or several amino acid substitutions (e.g., conservative substitutions), insertions, or deletions in regions of the amino acid sequence of SEQ ID No: 79 other than CDR1 (SEQ ID No: 17), CDR2 (SEQ ID No: 78), and CDR3 (SEQ ID No: 19);

(VH-10) the amino acid sequence of SEQ ID No: 81, or an amino acid sequence comprising one or several amino acid substitutions (e.g., conservative substitutions), insertions, or deletions in regions of the amino acid sequence of SEQ ID No: 81 other than CDR1 (SEQ ID No: 17), CDR2 (SEQ ID No: 80), and CDR3 (SEQ ID No: 19);

(VH-11) the amino acid sequence of SEQ ID No: 83, or an amino acid sequence comprising one or several amino acid substitutions (e.g., conservative substitutions), insertions, or deletions in regions of the amino acid sequence of SEQ ID No: 83 other than CDR1 (SEQ ID No: 25), CDR2 (SEQ ID No: 82), and CDR3 (SEQ ID No: 27);

(VH-12) the amino acid sequence of SEQ ID No: 85, or an amino acid sequence comprising one or several amino acid substitutions (e.g., conservative substitutions), insertions, or deletions in regions of the amino acid sequence of SEQ ID No: 85 other than CDR1 (SEQ ID No: 25), CDR2 (SEQ ID No: 84), and CDR3 (SEQ ID No: 27).

15. The antibody or antibody derivative according to any one of claims 12-14, wherein the amino acid sequence of the light chain variable region VL domain of the antibody or antibody derivative is selected from the group consisting of:

(VL-1a) the amino acid sequence of SEQ ID No: 8, or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6);

(VL-1b) the amino acid sequence of SEQ ID No: 58, or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6);

(VL-1c) the amino acid sequence of SEQ ID No: 50, or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6);

(VL-2a) the amino acid sequence of SEQ ID No: 16, or an amino acid sequence comprising one or several amino acid substitutions (e.g., conservative substitutions), insertions, or deletions in regions of the amino acid sequence of SEQ ID No: 16 other than CDR1 (SEQ ID No: 12), CDR2 (SEQ ID No: 13), and CDR3 (SEQ ID No: 14);

(VL-2b) the amino acid sequence of SEQ ID No: 63, or an amino acid sequence comprising one or several amino acid substitutions (e.g., conservative substitutions), insertions, or deletions in regions of the amino acid sequence of SEQ ID No: 63 other than CDR1 (SEQ ID No: 12), CDR2 (SEQ ID No: 13), and CDR3 (SEQ ID No: 14);

(VL-2c) the amino acid sequence of SEQ ID No: 64, or an amino acid sequence comprising one or several amino acid substitutions (e.g., conservative substitutions), insertions, or deletions in regions of the amino acid sequence of SEQ ID No: 64 other than CDR1 (SEQ ID No: 12), CDR2 (SEQ ID No: 13), and CDR3 (SEQ ID No: 14);

(VL-2d) the amino acid sequence of SEQ ID No: 65, or an amino acid sequence comprising one or several amino acid substitutions (e.g., conservative substitutions), insertions, or deletions in regions of the amino acid sequence of SEQ ID No: 65 other than CDR1 (SEQ ID No: 12), CDR2 (SEQ ID No: 13), and CDR3 (SEQ ID No: 14);

(VL-3a) the amino acid sequence of SEQ ID No: 24, or an amino acid sequence comprising one or several amino acid substitutions (e.g., conservative substitutions), insertions, or deletions in regions of the amino acid sequence of SEQ ID No: 24 other than CDR1 (SEQ ID No: 20), CDR2 (SEQ ID No: 21), and CDR3 (SEQ ID No: 22);

(VL-3b) the amino acid sequence of SEQ ID No: 67, or an amino acid sequence comprising one or several amino acid substitutions (e.g., conservative substitutions), insertions, or deletions in regions of the amino acid sequence of SEQ ID No: 67 other than CDR1 (SEQ ID No: 20), CDR2 (SEQ ID No: 21), and CDR3 (SEQ ID No: 22);

(VL-3c) the amino acid sequence of SEQ ID No: 68, or an amino acid sequence comprising one or several amino acid substitutions (e.g., conservative substitutions), insertions, or deletions in regions of the amino acid sequence of SEQ ID No: 68 other than CDR1 (SEQ ID No: 20), CDR2 (SEQ ID No: 21), and CDR3 (SEQ ID No: 22);

(VL-3d) the amino acid sequence of SEQ ID No: 69, or an amino acid sequence comprising one or several amino acid substitutions (e.g., conservative substitutions), insertions, or deletions in regions of the amino acid sequence of SEQ ID No: 69 other than CDR1 (SEQ ID No: 20), CDR2 (SEQ ID No: 21), and CDR3 (SEQ ID No: 22);

(VL-4a) the amino acid sequence of SEQ ID No: 32, or an amino acid sequence comprising one or several amino acid substitutions (e.g., conservative substitutions), insertions, or deletions in regions of the amino acid sequence of SEQ ID No: 32 other than CDR1 (SEQ ID No: 28), CDR2 (SEQ ID No: 29), and CDR3 (SEQ ID No: 30);

(VL-4b) the amino acid sequence of SEQ ID No: 71, or an amino acid sequence comprising one or several amino acid substitutions (e.g., conservative substitutions), insertions, or deletions in regions of the amino acid sequence of SEQ ID No: 71 other than CDR1 (SEQ ID No: 28), CDR2 (SEQ ID No: 29), and CDR3 (SEQ ID No: 30);

(VL-4c) the amino acid sequence of SEQ ID No: 72, or an amino acid sequence comprising one or several amino acid substitutions (e.g., conservative substitutions), insertions, or deletions in regions of the amino acid sequence of SEQ ID No: 72 other than CDR1 (SEQ ID No: 28), CDR2 (SEQ ID No: 29), and CDR3 (SEQ ID No: 30);

(VL-4d) the amino acid sequence of SEQ ID No: 73, or an amino acid sequence comprising one or several amino acid substitutions (e.g., conservative substitutions), insertions, or deletions in regions of the amino acid sequence of SEQ ID No: 73 other than CDR1 (SEQ ID No: 28), CDR2 (SEQ ID No: 29), and CDR3 (SEQ ID No: 30);

(VL-5a) the amino acid sequence of SEQ ID No: 8, or an amino acid sequence comprising one or several amino acid substitutions (e.g., conservative substitutions), insertions, or deletions in regions of the amino acid sequence of SEQ ID No: 8 other than CDR1 (SEQ ID No: 4), CDR2 (SEQ ID No: 5), and CDR3 (SEQ ID No: 6);

(VL-5b) the amino acid sequence of SEQ ID No: 58, or an amino acid sequence comprising one or several amino acid substitutions (e.g., conservative substitutions), insertions, or deletions in regions of the amino acid sequence of SEQ ID No: 58 other than CDR1 (SEQ ID No: 4), CDR2 (SEQ ID No: 5), and CDR3 (SEQ ID No: 6);

(VL-5c) the amino acid sequence of SEQ ID No: 50, or an amino acid sequence comprising one or several amino acid substitutions (e.g., conservative substitutions), insertions, or deletions in regions of the amino acid sequence of SEQ ID No: 50 other than CDR1 (SEQ ID No: 4), CDR2 (SEQ ID No: 5), and CDR3 (SEQ ID No: 6);

(VL-6a) the amino acid sequence of SEQ ID No: 56, or an amino acid sequence comprising one or several amino acid substitutions (e.g., conservative substitutions), insertions, or deletions in regions of the amino acid sequence of SEQ ID No: 56 other than CDR1 (SEQ ID No: 52), CDR2 (SEQ ID No: 53), and CDR3 (SEQ ID No: 54);

(VL-6b) the amino acid sequence of SEQ ID No: 60, or an amino acid sequence comprising one or several amino acid substitutions (e.g., conservative substitutions), insertions, or deletions in regions of the amino acid sequence of SEQ ID No: 60 other than CDR1 (SEQ ID No: 52), CDR2 (SEQ ID No: 53), and CDR3 (SEQ ID No: 54);

(VL-6c) the amino acid sequence of SEQ ID No: 61, or an amino acid sequence comprising one or several amino acid substitutions (e.g., conservative substitutions), insertions, or deletions in regions of the amino acid sequence of SEQ ID No: 61 other than CDR1 (SEQ ID No: 52), CDR2 (SEQ ID No: 53), and CDR3 (SEQ ID No: 54).

16. The antibody or antibody derivative according to claim 12 or 13,
wherein the variable region of the half-molecule composed of the single chain Fv antibody (scFv) is selected from the group consisting of:

(1) an amino acid sequence in which the heavy chain variable region of (VH-1a), (VH-1b) or (VH-1c) and the light chain variable region of (VL-1a), (VL-1b) or (VL-1c) are connected via a linker,
(2) an amino acid sequence in which the heavy chain variable region of (VH-2a) or (VH-2b) and the light chain variable region of (VL-2a), (VL-2b), (VL-2c) or (VL-2d) are connected via a linker,
(3) an amino acid sequence in which the heavy chain variable region of (VH-3a) or (VH-3b) and the light chain variable region of (VL-3a), (VL-3b), (VL-3c) or (VL-3d) are connected via a linker,
(4) an amino acid sequence in which the heavy chain variable region of (VH-4a) or (VH-4b) and the light chain variable region of (VL-4a), (VL-4b), (VL-4c) or (VL-4d) connected via a linker, and
(5) an amino acid sequence in which the heavy chain variable region of (VH-5a), (VH-5b) or (VH-5c) and the light chain variable region of (VL-5a), (VL-5b) or (VL-5c) are connected via a linker,
(6) an amino acid sequence in which the heavy chain variable region of (VH-6a), (VH-6b) or (VH-6c) and the light chain variable region of (VL-6a), (VL-6b) or (VL-6c) are connected via a linker,

(7) an amino acid sequence in which the heavy chain variable region of (VH-7) and the light chain variable region of (VL-2a), (VL-2b), (VL-2c) or (VL-2d) are connected via a linker,

(8) an amino acid sequence in which the heavy chain variable region of (VH-8) and the light chain variable region of (VL-2a), (VL-2b), (VL-2c) or (VL-2d) are connected via a linker,

(9) an amino acid sequence in which the heavy chain variable region of (VH-9) and the light chain variable region of (VL-3a), (VL-3b), (VL-3c) or (VL-3d) are connected via a linker,

(10) an amino acid sequence in which the heavy chain variable region of (VH-10) and the light chain variable region of (VL-3a), (VL-3b), (VL-3c) or (VL-3d) are connected via a linker,

(11) an amino acid sequence in which the heavy chain variable region of (VH-11) and the light chain variable region of (VL-4a), (VL-4b), (VL-4c) or (VL-4d) are connected via a linker,

(12) an amino acid sequence in which the heavy chain variable region of (VH-12) and the light chain variable region of (VL-4a), (VL-4b), (VL-4c) or (VL-4d) are connected via a linker.

17. The antibody or antibody derivative according to any one of claims 12-16, wherein the enhancement of immune activity is activation of T cells selected from the group consisting of removal of immunosuppressive signals, relieving T cell exhaustion, proliferation of T cells, increase in cytotoxicity of T cells against cancer cells, and promotion of T cell cytokine secretion.

18. The antibody or antibody derivative according to any one of claims 12-17, wherein the antibody or antibody derivative induces cytotoxicity against cancer cells but not against normal cells.

19. The antibody or antibody derivative according to any one of claims 12-18, wherein the cancer cell is selected from the group consisting of melanoma, breast cancer, lung cancer, colon cancer, gastric cancer, pancreatic cancer, liver cancer, and blood cancer.

20. A pharmaceutical composition for the treatment of cancer, comprising the antibody or antibody derivative according to any one of claims 1-19.

21. The pharmaceutical composition according to claim 20, wherein the cancer is selected from the group consisting of melanoma, breast cancer, lung cancer, colon cancer, gastric cancer, pancreatic cancer, liver cancer, and blood cancer.

22. A method for predicting increased cytotoxicity against cancer cells, comprising

a step of bringing cancer cells collected from a subject into contact in vitro with the antibody or antibody derivative according to any one of claims 1-19, and
a step of measuring whether the cell viability of the cancer cells is decreased or a step of measuring whether the secretion of immune-activating substances is enhanced under culture conditions.

23. The method according to claim 22, wherein whether cell viability of cancer cells is decreased or whether immune cells derived from peripheral blood lymphocytes are activated, is measured in the presence of peripheral blood lymphocytes from the same subject.

24. The method according to claim 22 or 23, wherein the enhancement of cytotoxicity against the cancer cells in vitro when the antibody or antibody derivative according to any one of claims 1-19 is administered to that subject is predicted based on the in vitro cytotoxicity against cancer cells collected from the subject.

25. The method according to claim 22 or 23, wherein the enhancement of cytotoxicity against cancer cells when the antibody or antibody derivative according to any one of claims 1 to 19 is administered to the subject is predicted based on the enhancement of secretion of an immune activating substance in vitro.

26. A measurement kit comprising the antibody or antibody derivative according to any one of claims 1-19, for measuring in vitro the cytotoxicity against cancer cells from a subject, secretion of immune activating substances against the cancer cells, or activation of immune cells against cancer cells of the antibody or antibody derivative.

[Fig. 1]

Each arrow indicates a noncompeting relationship.

[Fig. 2]

[Fig. 3]

[Fig. 4]

[Fig. 5 ]

[Fig. 6 ]

[Fig. 7]

[Fig. 8]

[Fig. 9 ]

[Fig. 10-1]

Change in Number of Cells
Donor 1

Donor 2

[Fig. 10-2]

## Tim-3 expression level （%）
## Donor 1

## Donor 2

[Fig. 11-1]

Donor 1

[Fig. 11-2]

## Donor 2

[Fig. 12]

[Fig. 13]

[Fig. 14]

[Fig. 15]

[Fig. 16-1]

Change in Number of Cells

[Fig. 16-2]

Tim-3 expression level（%）

[Fig. 17]

[Fig. 18]

[Fig. 19]

[Fig. 20]

[Fig. 21-1]

CD4+IFN-g+ / CD8+IFN-g+

[Fig. 21-2]

CD4+IFN-g+ / CD8+IFN-g+

[Fig. 22]

## Humanized antibody

## Chimeric antibody

[Fig. 23]

[Fig. 24]

[Fig. 25]

| Group | Tumor Growth Inhibition Rate (vs. Group 1) |
|---|---|
| Group 2 | 43% |
| Group 3 | 31% |
| Group 4 | 69% |

[Fig. 26]

[Fig. 27]

## Hu003_13_F20

[Fig. 28]

## Hu149_83

[Fig. 29]

**Darkgray : Partope**

# EP 4 372 006 A1

## INTERNATIONAL SEARCH REPORT

International application No.

**PCT/JP2022/027809**

### A. CLASSIFICATION OF SUBJECT MATTER

*C07K 16/28*(2006.01)i; *A61K 39/395*(2006.01)i; *A61K 47/55*(2017.01)i; *A61K 47/68*(2017.01)i; *A61P 35/00*(2006.01)i; *A61P 37/04*(2006.01)i; *C07K 16/46*(2006.01)i; *C12M 1/34*(2006.01)i; *C12N 15/13*(2006.01)i; *C12Q 1/02*(2006.01)i; *G01N 33/50*(2006.01)i

FI: C07K16/28 ZNA; C07K16/46; C12M1/34 A; C12M1/34 F; C12Q1/02; A61P35/00; A61P37/04; A61K47/68; A61K47/55; A61K39/395 G; A61K39/395 U; A61K39/395 T; A61K39/395 E; G01N33/50 Z; C12N15/13

According to International Patent Classification (IPC) or to both national classification and IPC

### B. FIELDS SEARCHED

Minimum documentation searched (classification system followed by classification symbols)

C07K16/28; A61K39/395; A61K47/55; A61K47/68; A61P35/00; A61P37/04; C07K16/46; C12M1/34; C12N15/13; C12Q1/02; G01N33/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq

### C. DOCUMENTS CONSIDERED TO BE RELEVANT

| Category* | Citation of document, with indication, where appropriate, of the relevant passages | Relevant to claim No. |
|---|---|---|
| X | JP 2018-537950 A (F. HOFFMANN-LA ROCHE AG) 27 December 2018 (2018-12-27) claims, abstract, in particular, examples 16, 18 | 1, 6-11, 20-26 |
| X | WO 2017/055393 A1 (F. HOFFMANN-LA ROCHE AG) 06 April 2017 (2017-04-06) claims, in particular, example 3 | 1, 6-11, 20-26 |
| X | GRABMEIER-PFISTERSHAMMER, K. et al. Antibodies targeting BTLA or TIM-3 enhance HIV-1 specific T cell responses in combination with PD-1 blockade. Clinical Immunology. 2017, vol. 183, pp. 167-173 p. 167, summary, p. 169, right column, line 1 to p. 171, left column, line 28 | 12-26 |
| Y | p. 167, summary, p. 169, right column, line 1 to p. 171, left column, line 28 | 2-11, 20-26 |

☑ Further documents are listed in the continuation of Box C.          ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 September 2022** | **11 October 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/027809**

**C.    DOCUMENTS CONSIDERED TO BE RELEVANT**

| Category* | Citation of document, with indication, where appropriate, of the relevant passages | Relevant to claim No. |
|---|---|---|
| X | HAYASHI, E. et al. Anti-TIM-3 antibody prevents lymphocyte apoptosis and enhances dendritic cell cancer therapy. Showa Univ. J. Med. Sci. 2015, vol. 27, no. 1, pp. 1-9<br>p. 1, summary, fig. 1 | 12-26 |
| Y | p. 1, summary, fig. 1 | 2-11, 20-26 |
| X | JP 2018-503396 A (F. HOFFMANN-LA ROCHE AG) 08 February 2018 (2018-02-08)<br>claims, in particular, examples 4, 5, 10-12 | 12-26 |
| Y | claims, in particular, examples 4, 5, 10-12 | 2-11, 20-26 |
| Y | AKIBA, H. et al. Development and activities, including immunocomplex formation, of biparatopic antibodies and alternative scaffold proteins. Translational and Regulatory Science. 2020, vol. 2, no. 1, pp. 1-6<br>p. 1, summary, in particular, table 1 | 2-11, 20-26 |
| Y | JP 2019-506841 A (BOEHRINGER INGELHEIM INTERNATIONAL GMBH) 14 March 2019 (2019-03-14)<br>claims, examples | 2-11, 20-26 |
| P, X | MISHIMA, Y. et al. Novel biparatopic TIM3 antibody effectively blocks multiple inherent ligands and activates anti-tumor immunity. SITC 36th Anniversary Annual Meeting (SITC2021) Abstracts, No.235. Journal of ImmunoTherapy of Cancer. November 2021, vol. 9, suppl. 2<br>in particular, column "Results" | 1, 2, 9-11, 20-26 |
| P, Y | in particular, column "Results" | 3-26 |

Form PCT/ISA/210 (second sheet) (January 2015)

**INTERNATIONAL SEARCH REPORT**

International application No.

**PCT/JP2022/027809**

**Box No. I**      **Nucleotide and/or amino acid sequence(s) (Continuation of item 1.c of the first sheet)**

1.   With regard to any nucleotide and/or amino acid sequence disclosed in the international application, the international search was carried out on the basis of a sequence listing:

     a.   ☑   forming part of the international application as filed:

         ☑   in the form of an Annex C/ST.25 text file.

         ☐   on paper or in the form of an image file.

     b.   ☐   furnished together with the international application under PCT Rule 13*ter*.1(a) for the purposes of international search only in the form of an Annex C/ST.25 text file.

     c.   ☐   furnished subsequent to the international filing date for the purposes of international search only:

         ☐   in the form of an Annex C/ST.25 text file (Rule 13*ter*.1(a)).

         ☐   on paper or in the form of an image file (Rule 13*ter*.1(b) and Administrative Instructions, Section 713).

2.   ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.   Additional comments:

     The "form of an Annex C/ST.25 text file" is replaced with the "form of ST.26."

Form PCT/ISA/210 (continuation of first sheet) (January 2015)

**INTERNATIONAL SEARCH REPORT**
Information on patent family members

International application No.

**PCT/JP2022/027809**

| Patent document cited in search report | | | Publication date (day/month/year) | Patent family member(s) | | | Publication date (day/month/year) |
|---|---|---|---|---|---|---|---|
| JP | 2018-537950 | A | 27 December 2018 | JP | 2020-58353 | A | |
| | | | | JP | 2018-533725 | A | |
| | | | | WO | 2017/055404 | A1 | |
| | | | | claims, abstract, in particular, examples 16, 18 | | | |
| | | | | US | 2017/0114135 | A1 | |
| | | | | US | 2019/0322748 | A1 | |
| | | | | US | 2018/0328920 | A1 | |
| | | | | WO | 2017/055399 | A1 | |
| | | | | EP | 3954709 | A1 | |
| WO | 2017/055393 | A1 | 06 April 2017 | (Family: none) | | | |
| JP | 2018-503396 | A | 08 February 2018 | US | 2016/0257749 | A1 | |
| | | | | US | 2018/0072804 | A1 | |
| | | | | US | 2019/0382480 | A1 | |
| | | | | WO | 2016/071448 | A1 | |
| | | | | claims, in particular, examples 4, 5, 10-12 | | | |
| | | | | EP | 3632935 | A1 | |
| JP | 2019-506841 | A | 14 March 2019 | JP | 2020-178701 | A | |
| | | | | US | 2017/0174762 | A1 | |
| | | | | US | 2020/0199222 | A1 | |
| | | | | WO | 2017/093478 | A1 | |
| | | | | claims, examples | | | |
| | | | | EP | 3797790 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4816567 A
- US 5223409 A
- US 5403484 A
- US 5571698 A, Ladner
- US 5427908 A
- US 5580717 A, Dower
- US 5969108 A
- US 6172197 B, McCafferty
- US 5885793 A
- US 6521404 B
- US 6544731 B
- US 6555313 B
- US 6582915 B
- US 6593081 B, Griffiths
- US 7183076 B2
- US 9605070 B2

**Non-patent literature cited in the description**

- **ISABEL SADA-OVALLE et al.** *J. Immunol.,* 2012, vol. 189 (12), 5896-902
- **CHEN ZHU et al.** *Nat. Immunol.,* 2005, vol. 6 (12), 1245-52
- **YOSHIKANE KIKUSHIGE et al.** *Cell Stem Cell,* 2015, vol. 17, 341-352
- **KOHLER ; MILSTEIN et al.** *Nature,* 1975, vol. 256, 495-97
- **HONGO et al.** *Hybridoma,* 1995, vol. 14 (3), 253-260
- **HARLOW et al.** Antibodies: A Laboratory Manual. Cold Spring Harbor Laboratory Press, 1988, vol. 2
- **HAMMERLING et al.** Monoclonal Antibodies and T-Cell Hybridomas. Elsevier, 1981, 563-681
- *Clin. Microbiol. Rev.,* 1994, vol. 7, 277-289